# EUROPEAN PATENT SPECIFICATION

(11) **EP 1 458 394 B1**
(45) Date of publication and mention of the grant of the patent: **22.10.2008**
(21) Application number: 02793675.6
(22) Date of filing: 18.12.2002
(51) Int. Cl.: A61K 31/517, C07D 403/04, C07D 403/14, A61P 25/28, A61P 25/14, A61P 25/18, A61P 25/24, A61P 15/16, A61P 9/10

(54) **USE OF OXINDOLE DERIVATIVES IN THE TREATMENT OF DEMENTIA RELATED DISEASES, ALZHEIMER S DISEASE AND CONDITIONS ASSOCIATED WITH GLYCOGEN SYNTHASE KINASE-3**
VERWENDUNG VON OXINDOL-DERIVATEN BEI DER BEHANDLUNG VON DEMENZBEDINGTEN KRANKHEITEN, ALZHEIMER-KRANKHEIT UND ERKRANKUNGEN IM ZUSAMMENHANG MIT GLYKOGEN-SYNTHASE-KINASE-3
UTILISATION DE DERIVES D'OXINDOLE DANS LE TRAITEMENT DES MALADIES ASSOCIEES A LA DEMENCE, LA MALADIE D'ALZHEIMER ET LES ETATS PATHOLOGIQUES ASSOCIES A LA GLYCOGENE SYNTHASE KINASE-3

(30) Priority: 21.12.2001 US 344887 P
(43) Date of publication of application: 22.09.2004
(73) Proprietor: AstraZeneca AB, 151 85 Södertälje (SE)
(72) Inventor: Berg, Stefan, 151 85 Södertälje (SE); Bhat, Ratan, 151 85 Södertälje (SE); Edwards, Philip, Wilmington, DE 19850-5437 (US); Hellberg, Sven, 151 85 Södertälje (SE)
(86) International application number: PCT/SE2002/002370
(87) International publication number: WO 2003/055492

(56) References cited:
- EP-A1- 1 136 493
- WO-A1-00/10975
- WO-A1-95/33750
- WO-A1-97/42187
- WO-A1-99/10349
- PIYASENA HEWAWASAM ET AL.: 'Synthesis and structure-activity relationships of 3-aryloxindoles: A new class of calcium-dependent, large conductance potassium (Maxi-K) channel openers with neuroprotective properties' J. MED. CHEM. vol. 45, 2002, pages 1487 - 1499, XP002959071
- IMAHORI: 'Physiology and pathology...' J. BIOCHEM. vol. 121, no. 2, 1997, pages 179 - 188
- HOSCHI: 'REGULATION OF MITHOCHONDRIAL...' PROC. NAT. ACAD. SCIENCE vol. 93, 1996, pages 2719 - 2723
- BHAT: 'Regulation and localization of tyrosine...' PNAS vol. 97, no. 20, 2000,
- STAMBOLIC: 'Lithium inhibits...' CURRENT BIOLOGY vol. 6, no. 12, 1996, pages 1664 - 1668
- KLEIN: 'Molecular mechanism...' PROC. NAT. ACAD. SCI. vol. 93, pages 8455 - 8459
- KOZLOSKY: 'Low GSK...' AM. J. PSYCHIATRY vol. 157, 2000, pages 831 - 833
- COTTER: 'Abnormalities of Wnt signalling...' NEUROREPORT vol. 9, no. 7, 1998, pages 1379 - 1383
- GAT: 'De novo hair follocle' CELL vol. 95, 1998, pages 605 - 614

## Description

### FIELD OF THE INVENTION

The present invention relates to a new use of oxindole derivatives, as a free base or salts thereof, in the manufacture of a medicament for the prevention and/or treatment of Alzheimer's Disease and dementia related diseases and, wherein the dementia related diseases are selected from the group consisting of Frontotemporal dementia Parkinson's Type, Parkinson dementia complex of Guam, HIV dementia, diseases with associated neurofibrillar tangle pathologies, predemented states, vascular dementia, dementia with Lewy bodies, Frontotemporal dementia and dementia pugilistica; amyotrophic lateral sclerosis, corticobasal degeneration, Down syndrome, Huntington's Disease, Parkinson's Disease, postencephelatic parkinsonism, progressive supranuclear palsy, Pick's Disease, Niemann-Pick's Disease, stroke, head trauma and other chronic neurodegenerative diseases, Bipolar Disease, affective disorders, depression, schizophrenia, cognitive disorders, hair loss and contraceptive medication, Mild Cognitive Impairment, Age-Associated Memory Impairment, Age-Related Cognitive Decline, Cognitive Impairment No Dementia, mild cognitive decline, mild neurocognitive decline, Late-Life Forgetfulness, memory impairment and cognitive impairment and androgenetic alopecia as well as new compounds.

### BACKGROUND OF THE INVENTION

Glycogen synthase kinase 3 (GSK3) is a serine / threonine protein kinase composed of two isoforms (α and β), which are encoded by distinct genes but are highly homologous within the catalytic domain. GSK3 is highly expressed in the central and peripheral nervous system. GSK3 phosphorylates several substrates including tau, β-catenin, glycogen synthase, pyruvate dehydrogenase and elongation initiation factor 2b (eIF2b). Insulin and growth factors activate protein kinase B, which phosphorylates GSK3 on the serine 9 residue and inactivates it.

### Alzheimer's Disease (AD) dementias, and taupathies.

AD is characterized by cognitive decline, cholinergic dysfunction and neuronal death, neurofibrillary tangles and senile plaques consisting of amyloid-β deposits. The sequence of these events in AD is unclear, but believed to be related. Glycogen synthase kinase 3β (GSK3β) or Tau (τ) phosphorylating kinase selectively phosphorylates the microtubule associated protein τ in neurons at sites that are hyperphosphorylated in AD brains. Hyperphosphorylated protein τ has lower affinity for microtubules and accumulates as paired helical filaments, which are the main components that constitute neurofibrillary tangles and neuropil threads in AD brains. This results in depolymerization of microtubules, which leads to dying back of axons and neuritic dystrophy. Neurofibrillary tangles are consistently found in diseases such as AD, amyotrophic lateral sclerosis, tangles are consistently found in diseases such as AD, amyotrophic lateral sclerosis, parkinsonism-dementia complex of Gaum, corticobasal degeneration, dementia pugilistica and head trauma, Down's syndrome, postencephalatic parkinsonism, progressive supranuclear palsy, Niemann-Pick's Disease and Pick's Disease. Addition of amyloid-β to primary hippocampal cultures results in hyperphosphorylation of τ and a paired helical filaments-like state via induction of GSK3β activity, followed by disruption of axonal transport and neuronal death (Imahori and Uchida., J. Biochem 121:179-188, 1997).

GSK3β preferentially labels neurofibrillary tangles and has been shown to be active in pretangle neurons in AD brains. GSK3 protein levels are also increased by 50% in brain tissue from AD patients. Furthermore, GSK3β phosphorylates pyruvate dehydrogenase, a key enzyme in the glycolytic pathway and prevents the conversion of pyruvate to acetyl-Co-A (Hoshi et al., PNAS 93:2719-2723, 1996). Acetyl-Co-A is critical for the synthesis of acetylcholine, a neurotransmitter with cognitive functions. Thus, GSK3β inhibition may have beneficial effects in progression as well as the cognitive deficits associated with Alzheimer's disease and other above-referred to diseases.

### Chronic and Acute Neurodegenerative Diseases.

Growth factor mediated activation of the PI3K /Akt pathway has been shown to play a key role in neuronal survival. The activation of this pathway results in GSK3β inhibition. Recent studies (Bhat et. al., PNAS 97:11074-11079 (2000)) indicate that GSK3β activity is increased in cellular and animal models of neurodegeneration such as cerebral ischemia or after growth factor deprivation. For example, the active site phosphorylation was increased in neurons vulnerable to apoptosis, a type of cell death commonly thought to occur in chronic and acute degenerative diseases such as Alzheimer's Disease, Parkinson's Disease, amyotrophic lateral sclerosis, Huntington's Disease and HIV dementia, ischemic stroke and head trauma. Lithium was neuroprotective in inhibiting apoptosis in cells and in the brain at doses that resulted in the inhibition of GSK3β. Thus, GSK3β inhibitors could be useful in attenuating the course of neurodegenerative diseases.

### Bipolar Disorders (BD)

Bipolar Disorders are characterised by manic episodes and depressive episodes. Lithium has been used to treat BD based on its mood stabilising effects. The disadvantage of lithium is the narrow therapeutic window and the danger of overdosing, that can lead to lithium intoxication. The recent discovery that lithium inhibits GSK3 at therapeutic concentrations has raised the possibility that this enzyme represents a key target of lithium's action in the brain (Stambolic et al., Curr. Biol. 6:1664-1668, 1996; Klein and Melton; PNAS 93:8455-8459, 1996). Inhibition of GSK3β may therefore be of therapeutic relevance in the treatment of BD as well as in AD patients that have affective disorders.

### Schizophrenia

GSK3 is involved in signal transduction cascades of multiple cellular processes, particularly during neural development. Kozlovsky et al (Am J Psychiatry 2000 May;157(5):831-3) found that GSK3β levels were 41% lower in the schizophrenic patients than in comparison subjects. This study indicates that schizophrenia involves neurodevelopmental pathology and that abnormal GSK3 regulation could play a role in schizophrenia. Furthermore, reduced β-catenin levels have been reported in patients exhibiting schizophrenia (Cotter et al., Neuroreport 9:1379-1383 (1998)).

### Diabetes

Insulin stimulates glycogen synthesis in skeletal muscles via the dephosphorylation and thus activation of glycogen synthase. Under resting conditions, GSK3 phosphorylates and inactivates glycogen synthase via dephosphorylation. GSK3 is also over-expressed in muscles from Type II diabetic patients (Nikoulina et al., Diabetes 2000 Feb;49(2):263-71). Inhibition of GSK3 increases the activity of glycogen synthase thereby decreasing glucose levels by its conversion to glycogen. GSK3 inhibition may therefore be of therapeutic relevance in the treatment of Type I and Type II diabetes and diabetic neuropathy.

### Hair Loss

GSK3 phosphorylates and degrades β-catenin. β-catenin is an effector of the pathway for keratonin synthesis. β-catenin stabilisation may be lead to increase hair development. Mice expressing a stabilised β-catenin by mutation of sites phosphorylated by GSK3 undergo a process resembling de novo hair morphogenesis (Gat et al., Cell 1998 Nov 25;95 (5):605-14)). The new follicles formed sebaceous glands and dermal papilla, normally established only in embryogenesis. Thus GSK3 inhibition may offer treatment for baldness.

### DETAILED DESCRIPTION OF THE INVENTION

Compounds of general formula I below are disclosed in WO 97/42187. According to WO 97/42187 the effect of the compounds on reducing antiangiogenic and/or vascular permeability in mammals has been investigated.

It has now surprisingly been found that the group of oxindole derivatives as decribed in WO 97/42187 are well suited for inhibiting glycogen synthase kinase-3. Said glycogen synthase kinase-3 inhibitors are suitable in the prevention and/or treatment of conditions associated with glycogen synthase kinase-3 in the central and peripheral nervous system.

In particular, the compounds of the invention are expected to be suitable for prevention and/or treatment of especially dementia related diseases and Alzheimer's Disease.

The dementia related diseases are selected from the group consisting of Frontotemporal dementia Parkinson's Type, Parkinson dementia complex of Guam, HIV dementia, predemented states, vascular dementia, dementia with Lewy bodies, Frontotemporal dementia and dementia pugilistica. The compounds of the invention are also expected to be suitable for prevention and/or treatment of amyotrophic lateral sclerosis, corticobasal degeneration, Down syndrome, Huntington's Disease, Parkinson's Disease, postencephelatic parkinsonism, progressive supranuclear palsy, Pick's Disease, Niemann-Pick's Disease, stroke, head trauma and other chronic neurodegenerative diseases, Bipolar Disease, affective disorders, depression, schizophrenia, cognitive disorders and hair loss.

The compounds of the invention are further expected to be suitable for prevention and/or treatment of Mild Cognitive Impairment, Age-Associated Memory Impairment, Age-Related Cognitive Decline, Cognitive Impairement No Dementia, mild cognitive decline, mild neurocognitive decline, Late-Life Forgetfulness, memory impairment and cognitive impairment and androgenetic alopecia.

In the present invention a compound of the general formula **I,** as a free base or salts thereof, may be used, in the manufacturing of a medicament for the prevention and/or treatment of Alzheimer's Disease and dementia related diseases and, wherein the dementia related diseases are selected from the group consisting of Frontotemporal dementia Parkinson's Type, Parkinson dementia complex of Guam, HIV dementia, diseases with associated neurofibrillar tangle pathologies, predemented states, vascular dementia, dementia with Lewy bodies, Frontotemporal dementia and dementia pugilistica; amyotrophic lateral sclerosis, corticobasal degeneration, Down syndrome, Huntington's Disease, Parkinson's Disease, postencephelatic parkinsonism, progressive supranuclear palsy, Pick's Disease, Niemann-Pick's Disease, stroke, head trauma and other chronic neurodegenerative diseases, Bipolar Disease, affective disorders, depression, schizophrenia, cognitive disorders, hair loss and contraceptive medication, Mild Cognitive Impairment, Age-Associated Memory Impairment, Age-Related Cognitive Decline, Cognitive Impairment No Dementia, mild cognitive decline, mild neurocognitive decline, Late-Life Forgetfulness, memory impairment and cognitive impairment and androgenetic alopecia: 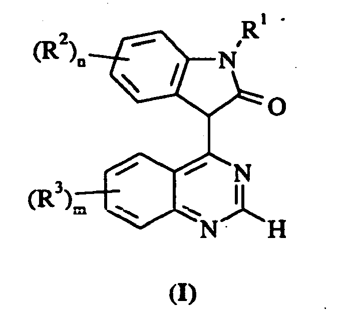 wherein:
- R¹ is: hydrogen or C₁₋₃alkyl;
- R² is: hydroxy, halogeno, trifluoromethyl, cyano, amino, nitro, carboxy, C₁₋₃alkyl, C₁₋₃alkoxy, C₁₋₃alkanoyloxy, C₂₋₄alkanoyl, C₁₋₄alkanoylamino, C₁₋₄alkoxycarbonyl, C₁₋₄alkylthio, C₁₋₄alkylsulphinyl, C₁₋₄alkylsulphonyl, carbamoyl, *N*-C₁₋₄alkylcarbamoyl, *N,N*-di(C₁₋₄alkyl)carbamoyl, aminosulphonyl, *N*-C₁₋₄alkylaminosulphonyl, *N,N*-di(C₁₋₄alkyl)aminosulphonyl, C₁₋₄alkylsulphonylamino, or a group R⁴X¹,
wherein X¹ is a direct bond, C₂₋₄alkanoyl, CONR⁵R⁶, SO₂NR⁷R⁸ or SO₂R⁹ (wherein R⁵ and R⁷ each independently are hydrogen or C₁₋₂alkyl, and R⁶, R⁸ and R⁹ each independently are C₁₋₄alkyl, and wherein R⁴ is linked to R⁶, R⁸ or R⁹); and
R⁴ is phenyl or a 5 or 6 membered heterocyclic group with one or two heteroatoms, selected independently from O, S and N, which heterocyclic group may be saturated or unsaturated and which phenyl or heterocyclic group may be substituted with one or two substituents selected independently from hydroxy, halogeno, C₁₋₃alkyl, C₁₋₃alkoxy, C₁₋₃alkanoyloxy, trifluoromethyl, cyano, amino, nitro and C₁₋₄alkoxycarbonyl;
- R³ is: hydroxy, halogeno, nitro, trifluoromethyl, C₁₋₃alkyl, cyano, amino or R¹⁰X², wherein X² is O, CH₂, S, SO, SO₂, NR¹¹CO, CONR¹², SO₂NR¹³, NR¹⁴SO₂ or NR¹⁵ (wherein R¹¹, R¹², R¹³, R¹⁴ and R¹⁵ each independently are hydrogen, C₁₋₃alkyl or C₁₋₃alkoxyC₂₋₃alkyl), or X² is a direct bond; and
R¹⁰ is selected from one of the following groups:
1) hydrogen or C₁₋₅alkyl which may be substituted with one or more groups selected independently from hydroxy, fluoro and amino;
2) C₁₋₅alkylX³COR¹⁶ (wherein X³ is O or NR¹⁷ (wherein R¹⁷ is hydrogen, C₁₋₃alkyl or C₁₋₃alkoxyC₂₋₃alkyl) and R¹⁶ is C₁₋₃alkyl, NR¹⁸R¹⁹ or OR²⁰ (wherein R¹⁸, R¹⁹ and R²⁰ each independently are hydrogen, C₁₋₃alkyl or C₁₋₃alkoxyC₂₋₃alkyl));
3) C₁₋₅alkylX⁴R²¹ (wherein X⁴ is O, S, SO, SO₂, OCO, NR²²CO, CONR²³, SO₂NR²⁴, NR²⁵SO₂ or NR²⁶ (wherein R²², R²³, R²⁴, R²⁵ and R²⁶ each independently are hydrogen, C₁₋₃alkyl or C₁₋₃alkoxyC₂₋₃alkyl) and R²¹ is hydrogen, C₁₋₃alkyl, cyclopentyl, cyclohexyl or a 5 or 6 membered saturated heterocyclic group with one or two heteroatoms selected independently from O, S and N, which C₁₋₃alkyl group may be substituted with one or two substituents selected independently from oxo, hydroxy, halogeno and C₁₋₄alkoxy and which heterocyclic group may be substituted with one or two substituents selected independently from oxo, hydroxy, halogeno, C₁₋₄alkyl, C₁₋₄ydroxyalkyl and C₁₋₄alkoxy);
4) C₁₋₅alkylX⁵C₁₋₅alkylX⁶R²⁷ (wherein X⁵ and X⁶ each independently are O, S, SO, SO₂, NR²⁸CO, CONR²⁹, SO₂NR³⁰, NR³¹SO₂ or NR³² (wherein R²⁸, R²⁹, R³⁰, R³¹ and R³² each independently are hydrogen, C₁₋₃alkyl or C₁₋₃alkoxyC₂₋₃alkyl) and R²⁷ is hydrogen or C₁₋₃alkyl);
5) C₁₋₅alkylR³³ (wherein R³³ is a 5 or 6 membered saturated heterocyclic group with one or two heteroatoms selected independently from O, S and N, which heterocyclic group may be substituted with one or two substituents selected independently from oxo, hydroxy, halogeno, C₁₋₄alkyl, C₁₋₆carbonyl, C₁₋₄hydroxyalkyl and C₁₋₄alkoxy);
6) C₂₋₅alkenylR³³ (wherein R³³ is as defined hereinbefore);
7) C₂₋₅alkynylR³³ (wherein R³³ is as defined hereinbefore);
8) R³⁴ (wherein R³⁴ is a pyridone group, a phenyl group or a 5 or 6 membered aromatic heterocyclic group with 1 to 3 heteroatoms selected independently from O, N and S, which pyridone, phenyl or heterocyclic group may carry up to 5 substituents selected independently from hydroxy, halogeno, amino, C₁₋₄alkyl, C₁₋₄alkoxy, C₁₋₄hydroxyalkyl, C₁₋₄aminoalkyl, C₁₋₄alkylamino, C₁₋₄hydroxyalkoxy, carboxy, cyano, CONR³⁵R³⁶ and NR³⁷COR³⁸ (wherein R³⁵, R³⁶, R³⁷ and R³⁸ each independently are hydrogen, C₁₋₄alkyl or C₁₋₃alkoxyC₂₋₃alkyl));
9) C₁₋₅alkylR³⁴ (wherein R³⁴ is as defined hereinbefore);
10) C₂₋₅alkenylR³⁴ (wherein R³⁴ is as defined hereinbefore);
11) C₂₋₃alkynylR³⁴ (wherein R³⁴ is as defined hereinbefore);
12) C₁₋₅alkylX⁷R³⁴ (wherein X⁷ is O, S, SO, SO₂, NR³⁹CO, CONR⁴⁰, SO₂NR⁴¹, NR⁴²SO₂ or NR⁴³ (wherein R³⁹, R⁴⁰, R⁴¹, R⁴² and R⁴³ each independently are hydrogen, C₁₋₃alkyl or C₁₋₃alkoxyC₂₋₃alkyl) and R³⁴ is as defined hereinbefore);
13) C₂₋₅alkenylX⁸R³⁴ (wherein X⁸ is O, S, SO, SO₂, NR⁴⁴CO, CONR⁴⁵, SO₂NR⁴⁶, NR⁴⁷SO₂ or NR⁴⁸ (wherein R⁴⁴, R⁴⁵, R⁴⁶, R⁴⁷ and R⁴⁸ each independently are hydrogen, C₁₋₃alkyl or C₁₋₃alkoxyC₂₋₃alkyl) and R³⁴ is as defined hereinbefore);
14) C₂₋₅alkynylX⁹R³⁴ (wherein X⁹ is O, S, SO, SO₂, NR⁴⁹CO, CONR⁵⁰, SO₂NR⁵¹, NR⁵²SO₂ or NR⁵³ (wherein R⁴⁹, R⁵⁰, R⁵¹, R⁵² and R⁵³ each independently are hydrogen, C₁₋₃alkyl or C₁₋₃alkoxyC₂₋₃alkyl) and R³⁴ is as defined hereinbefore);
15) C₁₋₃alkylX¹⁰C₁₋₃alkylR³⁴ (wherein X¹⁰ is O, S, SO, SO₂, NR⁵⁴CO, ONR⁵⁵, SO₂NR⁵⁶, NR⁵⁷SO₂ or NR⁵⁸ (wherein R⁵⁴, R⁵⁵, R⁵⁶, R⁵⁷ and R⁵⁸ each independently are hydrogen, C₁₋₃alkyl or C₁₋₃alkoxyC₂₋₃alkyl) and R³⁴ is as defined hereinbefore);
16) R³³ (wherein R³³ is as defined hereinbefore); and
17) C₁₋₃alkylX¹⁰C₁₋₃alkylR³³ (wherein X¹⁰ and R³³ are as defined hereinbefore);
18) C₁₋₅alkylCOR³³ (wherein R³³ is as defined hereinbefore);
- n is: 0, 1, 2, 3 or 4;
- m is: 0, 1, 2, 3 or 4;
as a free base or a pharmaceutically acceptable salt thereof.

One aspect of the invention relates to the use of compounds of formula I, wherein R³ is R¹⁰X²,
wherein X² is O, CH₂, S, SO, SO₂, NR¹¹CO, CONR¹², SO₂NR¹³, NR¹⁴SO₂ or NR¹⁵ (wherein R¹¹, R¹², R¹³, R¹⁴ and R¹⁵ each independently are hydrogen, C₁₋₃alkyl or C₁₋₃alkoxyC₂₋₃alkyl), or X² is a direct bond; and
R¹⁰ is selected from one of the following groups:
1) hydrogen or C₁₋₅alkyl which may be substituted with one or more groups selected independently from hydroxy, fluoro and amino;
2) C₁₋₅alkylX³COR¹⁶ (wherein X³ is O or NR¹⁷ (wherein R¹⁷ is hydrogen, C₁₋₃alkyl or C₁₋₃alkoxyC₂₋₃alkyl) and R¹⁶ is C₁₋₃alkyl, NR¹⁸R¹⁹ or OR²⁰ (wherein R¹⁸, R¹⁹ and R²⁰ each independently are hydrogen, C₁₋₃alkyl or C₁₋₃alkoxyC₂₋₃alkyl));
3) C₁₋₅alkylX⁴R²¹ (wherein X⁴ is O, S, SO, SO₂, OCO, NR²²CO, CONR²³, SO₂NR²⁴, NR²⁵SO₂ or NR²⁶ (wherein R²², R²³, R²⁴, R²⁵ and R²⁶ each independently are hydrogen, C₁₋₃alkyl or C₁₋₃alkoxyC₂₋₃alkyl) and R²¹ is hydrogen, C₁₋₃alkyl, cyclopentyl, cyclohexyl or a 5 or 6 membered saturated heterocyclic group with one or two heteroatoms selected independently from O, S and N, which C₁₋₃alkyl group may be substituted with one or two substituents selected independently from oxo, hydroxy, halogeno and C₁₋₄alkoxy and which heterocyclic group may be substituted with one or two substituents selected independently from oxo, hydroxy, halogeno, C₁₋₄alkyl, C₁₋₄hydroxyalkyl and C₁₋₄alkoxy);
4) C₁₋₅alkylX⁵C₁₋₅alkylX⁶R²⁷ (wherein X⁵ and X⁶ each independently are O, S, SO, SO₂, NR²⁸CO, CONR²⁹, SO₂NR³⁰, NR³¹SO₂ or NR³² (wherein R²⁸, R²⁹, R³⁰, R³¹ and R³² each independently are hydrogen, C₁₋₃alkyl or C₁₋₃alkoxyC₂₋₃alkyl) and R²⁷ is hydrogen or C₁₋₃alkyl);
5) C₁₋₅alkylR³³ (wherein R³³ is a 5 or 6 membered saturated heterocyclic group with one or two heteroatoms selected independently from O, S and N, which heterocyclic group may be substituted with one or two substituents selected independently from oxo, hydroxy, halogeno, C₁₋₄alkyl, C₁₋₄hydroxyalkyl and C₁₋₄alkoxy);
6) C₂₋₅alkenylR³³ (wherein R³³ is as defined hereinbefore);
7) C₂₋₅alkynylR³³ (wherein R³³ is as defined hereinbefore);
8) R³⁴ (wherein R³⁴ is a pyridone group, a phenyl group or a 5 or 6 membered aromatic heterocyclic group with 1 to 3 heteroatoms selected independently from O, N and S, which pyridone, phenyl or heterocyclic group may carry up to 5 substituents selected independently from hydroxy, halogeno, amino, C₁₋₄alkyl, C₁₋₄alkoxy, C₁₋₄hydroxyalkyl, C₁₋₄aminoalkyl, C₁₋₄alkylamino, C₁₋₄hydroxyalkoxy, carboxy, cyano, CONR³⁵R³⁶ and NR³⁷COR³⁸ (wherein R³⁵, R³⁶, R³⁷ and R³⁸ each independently are hydrogen, C₁₋₄alkyl or C₁₋₃alkoxyC₂₋₃alkyl));
9) C₁₋₅alkylR³⁴ (wherein R³⁴ is as defined hereinbefore);
10) C₂₋₅alkenylR³⁴ (wherein R³⁴ is as defined hereinbefore);
11) C₂₋₅alkynylR³⁴ (wherein R³⁴ is as defined hereinbefore);
12) C₁₋₅alkylX⁷R³⁴ (wherein X⁷ is O, S, SO, SO₂, NR³⁹CO, CONR⁴⁰, SO₂NR⁴¹, NR⁴²SO₂ or NR⁴³ (wherein R³⁹, R⁴⁰, R⁴¹, R⁴² and R⁴³ each independently are hydrogen, C₁₋₃alkyl or C₁₋₃alkoxyC₂₋₃alkyl) and R³⁴ is as defined hereinbefore);
13) C₂₋₅alkenylX⁸R³⁴ (wherein X⁸ is O, S, SO, SO₂, NR⁴⁴CO, CONR⁴⁵, SO₂NR⁴⁶, NR⁴⁷SO₂ or NR⁴⁸ (wherein R⁴⁴, R⁴⁵, R⁴⁶, R⁴⁷ and R⁴⁸ each independently are hydrogen, C₁₋₃alkyl or C₁₋₃alkoxyC₂₋₃alkyl) and R³⁴ is as defined hereinbefore);
14) C₂₋₅alkynylX⁹R³⁴ (wherein X⁹ is O, S, SO, SO₂, NR⁴⁹CO, CONR⁵⁰, SO₂NR⁵¹, NR⁵²SO₂ or NR⁵³ (wherein R⁴⁹, R⁵⁰, R⁵¹, R⁵² and R⁵³ each independently are hydrogen, C₁₋₃alkyl or C₁₋₃alkoxyC₂₋₃alkyl) and R³⁴ is as defined hereinbefore);
15) C₁₋₃alkylX¹⁰C₁₋₃alkylR³⁴ (wherein X¹⁰ is O, S, SO, SO₂, NR⁵⁴CO, ONR⁵⁵, SO₂NR⁵⁶, NR⁵⁷SO₂ or NR⁵⁸ (wherein R⁵⁴, R⁵⁵, R⁵⁶, R⁵⁷ and R⁵⁸ each independently are hydrogen, C₁₋₃alkyl or C₁₋₃alkoxyC₂₋₃alkyl) and R³⁴ is as defined hereinbefore);
16) R³³ (wherein R³³ is as defined hereinbefore); and
17) C₁₋₃alkylX¹⁰C₁₋₃alkylR³³ (wherein X¹⁰ and R³³ are as defined hereinbefore).

Another aspect of the invention relates to the use of compounds of formula I, wherein R¹ is hydrogen.

In a further aspect of the invention compounds of formula I may be used, wherein R² is halogeno, cyano, nitro, carboxy, C₁₋₄alkoxycarbonyl, trifluoromethyl, C₁₋₃alkyl, C₁₋₃alkoxy, *N*-C₁₋₄alkylcarbamoyl, *N,N*-di(C₁₋₄alkyl)carbamoyl, aminosulphonyl, or a group R⁴X¹,
wherein X¹ is CONR⁵R⁶, (wherein R⁵ is hydrogen or C₁₋₂alkyl, and R⁶ is C₁₋₄alkyl, and
wherein R⁴ is linked to R⁶); and
R⁴ is phenyl or a 5 or 6 membered heterocyclic group with one or two heteroatoms, selected independently from O and N, which heterocyclic group may be saturated or unsaturated and which phenyl or heterocyclic group may be substituted with one or two substituents selected independently from hydroxy, halogeno, C₁₋₃alkyl, C₁₋₃alkoxy, C₁₋₃alkanoyloxy, trifluoromethyl, cyano, amino, nitro and C₁₋₄alkoxycarbonyl;
n is 0, 1 or 2.

In one aspect of the invention compounds of formula I may be used,
wherein R³ is R¹⁰X²,
wherein X² is O; and
R¹⁰ is selected from one of the following groups:
1) hydrogen or C₁₋₅alkyl;
3) C₁₋₅alkylX⁴R²¹ (wherein X⁴ is O or NR²⁶ (wherein R²¹ and R²⁶ each independently are hydrogen, C₁₋₃alkyl, cyclopentyl or cyclohexyl));
4) C₁₋₅alkylX⁵C₁₋₅alkylX⁶R²⁷ (wherein X⁵ and X⁶ are O and R²⁷ is hydrogen or C₁₋₃alkyl);
5) C₁₋₅alkylR³³ (wherein R³³ is a 5 or 6 membered saturated heterocyclic group with one or two heteroatoms, selected independently from O, S and N, which heterocyclic group may be substituted with one or two substituents selected independently from oxo, hydroxy, halogeno, C₁₋₄alkyl, C₁₋₆carbonyl, C₁₋₄ydroxyalkyl and C₁₋₄alkoxy);
9) C₁₋₅alkylR³⁴ (wherein R³⁴ is a 5 membered aromatic heterocyclic group with 1 to 3 heteroatoms selected independently from O and N, which heterocyclic group may carry up to 5 substituents selected independently from halogeno, amino, C₁₋₄alkyl, C₁₋₄alkoxy, C₁₋₄hydroxyalkyl, C₁₋₄aminoalkyl, C₁₋₄alkylamino, C₁₋₄hydroxyalkoxy, carboxy, hydoxy, cyano, CONR³⁵R³⁶ and NR³⁷COR³⁸ (wherein R³⁵, R³⁶, R³⁷ and R³⁸ each independently are hydrogen, C₁₋₄alkyl or C₁₋₃alkoxyC₂₋₃alkyl)); and
17) C₁₋₃alkylX¹⁰C₁₋₃alkylR³³ (wherein X¹⁰ is O and R³³ are as defined hereinbefore);
18) C₁₋₅alkylCOR³³ (wherein R³³ is as defined hereinbefore);
m is 0, 1 or 2.

In another aspect of the invention compounds of formula I may be used,
wherein R³ is R¹⁰X²,
wherein X² is O; and R¹⁰ is
4) C₁₋₅alkylX⁵C₁₋₅alkylX⁶R²⁷ (wherein X⁵ and X⁶ are O and R²⁷ is hydrogen or C₁₋₃alkyl).

In yet another aspect of the invention compounds of formula I may be used, wherein the R² is substituted on position 5 and/or 6 and R³ is substituted on position 6, 7 and/or 8.

The present invention further relates to novel compounds, which are
3-[7-(3-Dimethylaminopropoxy)quinazolin-4-yl]-2-hydroxy-1*H*-indol-5-carbonitrile hydrochloride,
3-[7-(2-Dimethylaminoethoxy)quinazolin-4-yl]-2-hydroxy-1*H*-indol-5-carbonitrile fumarate,
3-{7-[2-(Isopropylmethylamino)ethoxy]quinazolin-4-yl]-2-oxo-2,3-dihydro-1*H*-indol-5-carbonitrile fumarate and
3-[7-(2-Diisopropylamino)ethoxy)quinazolin-4-yl]-2-hydoxy-1*H*-indol-5-carbonitrile fumarate,
as a free base or salts thereof.

In another aspect of the invention the compounds listed hereinbefore may be used in the manufacturing of a medicament for the prevention and/or treatment of Alzheimer's Disease and dementia related diseases and, wherein the dementia related diseases are selected from the group consisting of Frontotemporal dementia Parkinson's Type, Parkinson dementia complex of Guam, HIV dementia, diseases with associated neurofibrillar tangle pathologies, predemented states, vascular dementia, dementia with Lewy bodies, Frontotemporal dementia and dementia pugilistica; amyotrophic lateral sclerosis, corticobasal degeneration, Down syndrome, Huntington's Disease, Parkinson's Disease, postencephelatic parkinsonism, progressive supranuclear palsy, Pick's Disease, Niemann-Pick's Disease, stroke, head trauma and other chronic neurodegenerative diseases, Bipolar Disease, affective disorders, depression, schizophrenia, cognitive disorders, hair loss and contraceptive medication, Mild Cognitive Impairment, Age-Associated Memory Impairment, Age-Related Cognitive Decline, Cognitive Impairment No Dementia, mild cognitive decline, mild neurocognitive decline, Late-Life Forgetfulness, memory impairment and cognitive impairment and androgenetic alopecia.

For the avoidance of doubt it is to be understood that where in this specification a group is qualified by 'hereinbefore defined' or 'defined hereinbefore' the said group encompasses the first occurring and broadest definition as well as each and all of the preferred definitions of that group.
For the avoidance of doubt it is to be understood that in this specification 'C₁₋₅' means a carbon group having 1, 2, 3, 4 or 5 carbon atoms.
In this specification, unless stated otherwise, the term "alkyl" includes both straight and branched chain alkyl groups. C₁₋₅alkyl may be methyl, ethyl, n-propyl, i-propyl, n-butyl, i-butyl, s-butyl, t-butyl, n-pentyl, i-pentyl, t-pentyl, neo-pentyl.
The term "alkoxy" as used herein, unless stated otherwise includes "alkyl"O groups in which "alkyl" is as hereinbefore defined. C₁₋₅alkoxy may be methoxy, ethoxy, n-propoxy, i-propoxy, n-butoxy, i-butoxy, s-butoxy, t-butoxy, n-pentyloxy, i-pentyloxy, t-pentyloxy, neo-pentyloxy.
The term "alkanoyl" as used herein, unless otherwise stated includes formyl and alkylC=O groups in which "alkyl" is as defined hereinbefore, for example C₂alkanoyl is ethanoyl and refers to CH₃C=O, C₁alkanoyl is formyl and refers to CHO.
In this specification, unless stated otherwise, the term "alkenyl" includes both straight and branched chain alkenyl groups but references to individual alkenyl groups such as 2-butenyl are specific for the straight chain version only. Unless otherwise stated, the term "alkenyl" advantageously refers to chains with 2 to 5 carbon atoms, preferably 3 to 4 carbon atoms.
In this specification, unless stated otherwise, the term "alkynyl" includes both straight and branched chain alkynyl groups but references to individual alkynyl groups such as 2-butynyl are specific for the straight chain version only. Unless otherwise stated, the term "alkynyl" advantageously refers to chains with 2 to 5 carbon atoms, preferably 3 to 4 carbon atoms.
In this specification, unless stated otherwise, the term "5 or 6 membered heterocyclic group with one or two heteroatoms, selected independently from O, S and N, which heterocyclic group may be saturated or unsaturated" includes both heteroaromatic rings and heterocyclic rings that are saturated. Examples of such heterocyclic groups includes, but are not limited to, furyl, imidazolyl, isoxazolyl, isothiazolyl, oxazolyl, pyrazinyl, pyrazolyl, pyridazinyl, pyridyl, pyrimidyl, pyrrolyl, thiazolyl, thienyl, imidazolidinyl, imidazolinyl, morpholinyl, piperazinyl, piperidyl, piperidonyl, pyrazolidinyl, pyrazolinyl, pyrrolidinyl, pyrrolinyl, tetrahydropyranyl or thiomorpholinyl.
In this specification, unless stated otherwise, the term "5 or 6 membered saturated heterocyclic group with one or two heteroatoms, selected independently from O, S and N" may be, but are not limited to, pyrrolidinyl, imidazolidinyl, pyrazolidinyl, morpholinyl, piperidinyl, oxathianyl, thiomorpholinyl, piperazinyl. In this specification, unless stated otherwise, the term "5 or 6 membered aromatic heterocyclic group with 1 to 3 heteroatoms, selected independently from O, N and S" may be, but are not limited to, furyl, imidazolyl, isoxazolyl, isothiazolyl, oxazolyl, pyrazinyl, triazinyl, pyrazolyl, pyridazinyl, pyridyl, pyrimidyl, pyrrolyl, thiazolyl or thienyl. In this specification, unless stated otherwise, the term halogeno may be fluor, chlorine, bromine or iodine.

For the avoidance of any doubt, it is to be understood that when X² is, for example, a group of formula NR¹¹CO, it is the nitrogen atom be substituted withing the R¹¹ group which is attached to the quinazoline ring and the carbonyl (CO) group is attached to R¹⁰, whereas when X² is, for example, a group of formula CONR¹², it is the carbonyl group which is attached to the quinazoline ring and the nitrogen atom be substituted withing the R¹² group is attached to R¹⁰. A similar convention applies to the other two atoms X² linking groups such as NR¹⁴SO₂ and SO₂NR¹³. When X² is NR¹⁵ it is the nitrogen atom be substituted withing the R¹⁵ group which is linked to the quinazoline ring and to R¹⁰. An analogous convention applies to other groups. It is further to be understood that when X² is NR¹⁵ and R¹⁵ is C₁₋₃alkoxyC₂₋₃alkyl it is the C₂₋₃alkyl moiety, which is linked to the nitrogen atom of X² and an analogous convention applies to other groups.
For the avoidance of any doubt, it is to be understood that in a compound of the formula I when R¹⁰ is, for example, a group of formula C₁₋₅alkylX¹⁰C₁₋₅alkylR³⁴, it is the terminal C₁₋₅alkyl moiety, which is linked to X², similarly when R¹⁰ is, for example, a group of formula C₂₋₅alkenylR³⁴ it is the C₂₋₅alkenyl moiety which is linked to X² and an analogous convention applies to other groups.
For the avoidance of any doubt, it is to be understood that when R³⁴ carries a C₁₋₄aminoalkyl substituent it is the C₁₋₄alkyl moiety which is attached to R³⁴ whereas when R³⁴ carries a C₁₋₄alkylamino substituent it is the amino moiety which is attached to R³⁴ and an analogous convention applies to other groups.
For the avoidance of any doubt when X¹ is C₂₋₄alkanoy it is the carbonyl moiety which is linked to the benzen ring of the oxindole group and it is the alkyl moiety which is linked to R⁴ and an analogous convention applies to other groups.

Some compounds of formula **I** may have chiral centres and/or geometric isomeric centres (E- and Z- isomers), and it is to be understood that the invention encompasses all such optical, diastereoisomers and geometric isomers that possess GSK3 inhibitory activity.

It is to be understood that the present invention also relates to any and all tautomeric forms of the compounds of formula **I.**

The present invention relates to the use of compounds of formula **I** and to new compounds as hereinbefore defined as well as to the salts thereof. Salts for use in pharmaceutical compositions will be pharmaceutically acceptable salts, but other salts may be useful in the production of the compounds of formula **I** and their pharmaceutically acceptable salts. Both organic and inorganic acids can be employed to form non-toxic pharmaceutically acceptable acid addition salts of the compounds of this invention. In addition, a suitable pharmaceutically acceptable salt of the compounds of the invention is an alkali metal salt, an alkaline earth metal salt or a salt with an organic base.

### Methods of Preparation

Compounds of formula I, or salt thereof, may be prepared by any process known to be applicable to the preparation of chemically-related compounds. Such processes include, for example, those illustrated in European Patent Applications Publication Nos. 0520722, 0566226, 0602851, 0635498 and 0636608 and PCT application WO 97/42187.

Throughout the following description of such processes it is understood that, where appropriate, suitable protecting groups will be added to, and subsequently removed from, the various reactants and intermediates in a manner that will be readily understood by one skilled in the art of organic synthesis. Conventional procedures for using such protecting groups as well as examples of suitable protecting groups are described, for example, in "Protective Groups in Organic Synthesis" T.W. Greene, P.G.M. Wuts, Wiley-Interscience, New York, 1999.
The terms "room" and "ambient" temperature refer to a temperature between 16°C and 25°C.

### Methods of Preparation of Intermediates

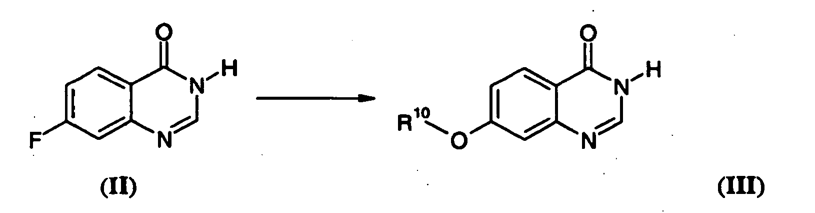

(i) Conversion of a compound of formula **II** to a compound of formula **III,** wherein R¹⁰ is as defined in formula I, may be carried with a suitable reagent R¹⁰-OH in a suitable solvent such as dimethylsulphoxide, dioxane or *N,N*-dimethylformamide in the presence of a suitable base such as an alkali metal or alkaline earth metal carbonate or hydroxide such as sodium carbonate, potassium carbonate, calcium carbonate, sodium hydroxide or potassium hydroxide or an alkali metal hydride such as sodium hydride, or an alkali metal or alkaline earth metal amide such as sodium amide, sodium bis(trimethylsilyl)amide, potassium amide or potassium bis(trimethylsilyl)amide and the reaction may occur at a temperature between 0 °C and +150 °C. 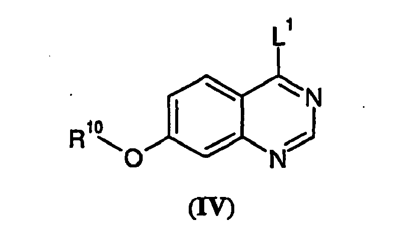
(ii) Conversion of a compound of formula **III** to a compound of formula **IV,** wherein L¹ is a suitable leaving group such as a halogeno e.g. chlorine or bromine, may be carried with a suitable halogenation reagent such as thionyl chloride, oxalyl chloride, phosphorus oxychloride, phosphoric trichloride or aluminum tribromide in a suitable solvent such as methylene chloride, chloroform, toluene or using the halogenation reagent neat and the reaction may occur at a temperature between +20 °C and +130 °C. 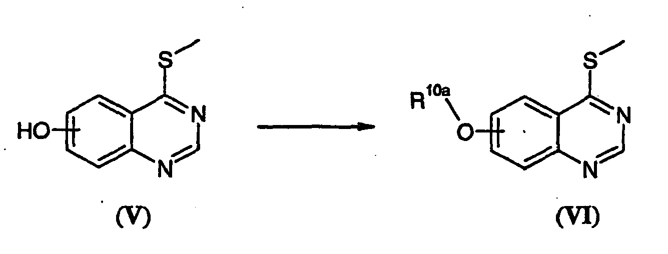
(iii) Conversion of a compound of formula **V** to a compound of formula **VI,** wherein R^{10a} is C₂-C₅alkyl-L², wherein L² is a suitable leaving group such as a halogeno e.g. chlorine or bromine, or R^{10a} is R¹⁰ wherein R¹⁰ is as defined in formula **I,** may be carried out with a suitable reagent such as
   a) an alkylating reagent of the formula **IX** or a reagent L²-C₂-C₅alkyl-L³, wherein L² and L³ may be the same or different, e.g. halogeno e.g. chlorine or bromine, in a suitable solvent such as *N,N*-dimethylformamide, methylene chloride or acetonitrile in the presence of a suitable base such as an alkali metal or alkaline earth metal carbonate or hydroxide such as sodium carbonate, potassium carbonate, calcium carbonate, sodium hydroxide or potassium hydroxide or an alkyl amine base such as triethyl amine and the reaction may occur between +20 °C and +150 °C, or
   b) R¹⁰-OH in a suitable solvent such as methylene chloride, chloroform, diethyl ether or tetrahydrofuran in the presence of a suitable coupling reagent such as diisopropyl azocarbodiimide or diethyl azocarbodiimide and triphenylphosphine and the reaction may occur at a temperature between +10 and +150 °C. 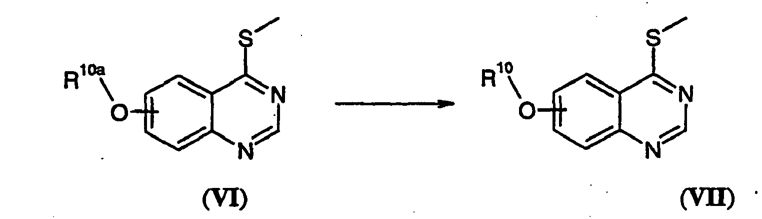
(iv) Conversion of a compound of formula **VI,** wherein R^{10a} is C₂-C₅alkyl-L², wherein L² is a suitable leaving group such as a halogeno e.g. chlorine or bromine, to a compound of formula **VII,** wherein R¹⁰ is as defined in formula **I**, may be carried out by 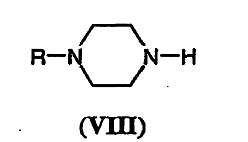 alkylation of a compound of formula **VIII,** wherein R is an alkanoyl group, in a suitable solvent such as *N,N*-dimethylformamide, methylene chloride or acetonitrile or using a compound of formula **VIII** neat and the reaction may occur at a temperature between +20 and +150 °C. 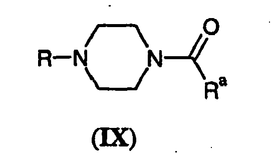
(v) Conversion of a compound of formula **VIII,** wherein R is an alkanoyl group, to a compound of formula **IX**, wherein R^{a} is C₁-C₅alkylL³ and L³ is as defined above may be carried out by reaction with a reagent L²(CO)C₁-C₅alkylL³, wherein L² and L³ may be the same or different and are defined as above, in a suitable solvent such as methylene chloride, chloroform or acetonitrile in the presence of a suitable base such as an alkali metal or alkaline earth metal carbonate or hydroxide such as sodium carbonate, potassium carbonate, calcium carbonate, sodium hydroxide or potassium hydroxide or an alkyl amine base such as triethyl amine and the reaction may occur at a temperature between -70 °C and +80 °C. 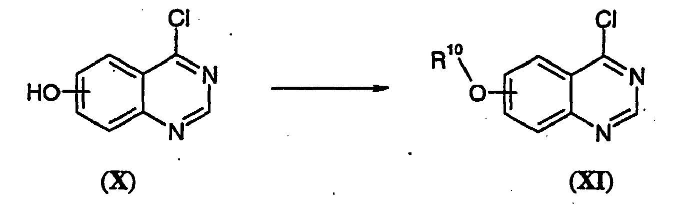
(vi) Conversion of a compound of formula **X** to a compound of formula **XI,** wherein R¹⁰ is as defined in formula I, may be carried out with a suitable reagent such as R¹⁰-OH in as suitable solvent such as methylene chloride, chloroform, diethyl ether or tetrahydrofuran in the presence of a suitable coupling reagent such as diisopropyl azocarbodiimide or diethyl azocarbodiimide and triphenylphosphine and the reaction may occur at a temperature between +10 and +150 °C. 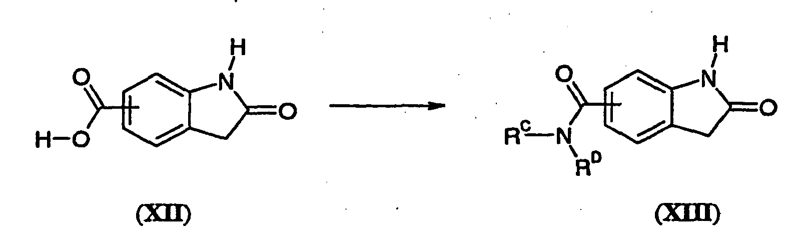
(vii) Conversion of a compound of formula **XII** to a compound of formula **XIII,** wherein R^{C} and R^{D} is hydrogen or C₁₋₄alkyl, may be carried out by activation of the acid function in a compound of formula **XII**
   a) with a halogenation reagent such as thionyl chloride or oxalyl chlorid in a suitable solvent such as methylene chloride or toluene or using the reagent neat folllowed by a reaction with the appropriate substituted amine R^{C}R^{D}NH in a suitable solvent such as methylene chloride, chloroform or acetonitrile in the presence of a suitable base such as an alkali metal or alkaline earth metal carbonate or hydroxide such as sodium carbonate, potassium carbonate, calcium carbonate, sodium hydroxide or potassium hydroxide or an alkyl amine base such as triethyl amine and the reaction may occur at a temperature between -70 °C and +80 °C, or
   b) with a suitable coupling reagent such as 1,1'-carbonyldiimidazole or 1-ethyl-3-(3-dimethylaminopropyl)carbodiimide in a suitable solvent such as *N,N*-dimethylformamide or tetrahydrofuran followed by addition and reaction with the appropriate substituted amine R^{C}R^{D}NH and at a reaction temperature between +20 °C and +130 °C. 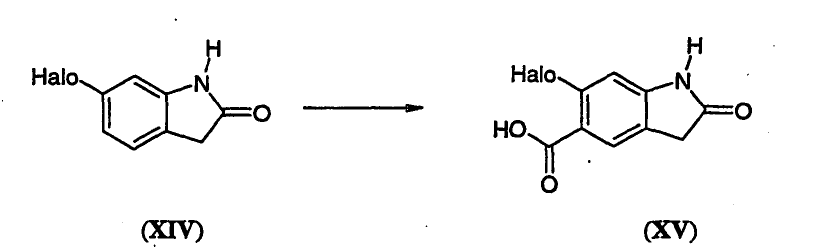
(viii) Conversion of a compound of formula **XIV** to a compound of formula **XV,** wherein halo is halogeno and is as defined in formula **I,** may be carried out by
   a) a Friedel-Craft acylation using acylating reagent such as chloroacetyl chloride and aluminum trichloride in a suitable solvent such as methylene chloride, chloroform or nitrobenzene and at a reaction temperature between ±0 °C and 60 °C, followed by,
   b) reaction of the formed chloroketone with pyridine followed by hydrolysis in a suitable solvent such as water or a mixture of water and an alcohol such as ethanol or methanol in the presence of a suitable base such as sodium hydroxide or potassium hydroxide and at a reaction temperature between +20 °C and reflux resulting in a compound of formula **XV.** 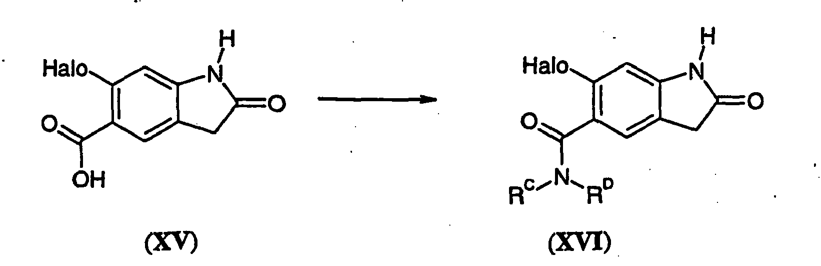
(ix) Conversion of a compound of formula **XV** to a compound of formula **XVI,** wherein R^{C} and R^{D} are as defined hereinbefore,
   may be carried out by activation of the acid function in a compound of formula **XV,**
   a) with a halogenation reagent such as thionyl chloride or oxalyl chlorid in a suitable solvent such as methylene chloride or toluene or using the reagent neat followed by reaction with the appropriate substituted amine R^{C}R^{D}NH in a suitable solvent such as methylene chloride, chloroform or acetonitrile in the presence of a suitable base such as an alkali metal or alkaline earth metal carbonate or hydroxide such as sodium carbonate, potassium carbonate, calcium carbonate, sodium hydroxide or potassium hydroxide or an alkyl amine base such as triethyl amine and the reaction may occur at a temperature between -70 °C and +80 °C, or
   b) with a suitable coupling reagent such as 1,1'-carbonyldiimidazole or 1-methyl-3-(3-dimethylaminopropyl)carbodiimide in a suitable solvent such as *N,N*-dimethylformamide or tetrahydrofuran followed by addition and reaction with the appropriate substituted amine R^{C}R^{D}NH and at a reaction temperature between +20 °C and +130 °C. 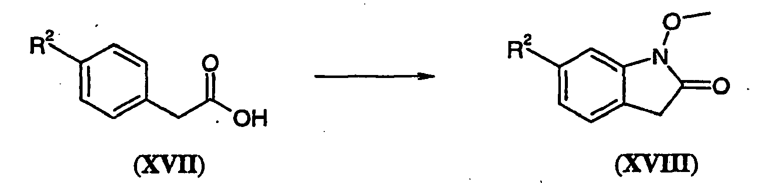
(x) Conversion of a compound of formula **XVII** to a compound of formula **XVIII,** wherein R² is C₁₋₃alkyl, may be carried out by
   a) activation of the acid function in a compound of formula **XVII** with a halogenation reagent such as thionyl chloride or oxalyl chlorid in a suitable solvent such as methylene chloride or toluene or using the reagent neat and at a reaction temperature between +20 °C and reflux, followed by,
   b) conversion of the acid chloride to the corresponding *N*-methoxycarboxamide by a reaction with methoxyamine hydrochloride in an appropriate solvent such as methylene chloride, chloroform or toluene or solvent mixtures with water such as toluene and water in the presence of a suitable base such as an alkali metal or alkaline earth metal carbonate or hydroxide such as sodium carbonate, potassium carbonate, calcium carbonate, sodium hydroxide or potassium hydroxide or an alkyl amine base such as triethyl amine and the reaction may occur between +20 °C and +120 °C, followed by,
   c) cyclization, to form the compound of formula **XVIII,** wherein R² is C₁₋₃alkyl, in a suitable solvent such as methylene chloride or chloroform using a suitable reagent such as *tert*-butyl hypochlorite and at a reaction temperature between ±0 °C and reflux. 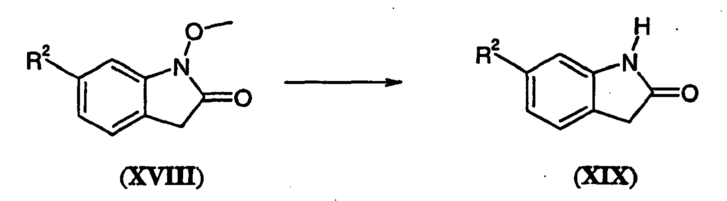
(xi) Conversion of a compound of formula **XVIII** to a compound of formula **XIX,** wherein R² is C₁₋₃alkyl, may be carried out by hydrogenation using a catalyst containing palladium; platinum, rhodium or nickel in a suitable solvent such as acetic acid or an alcohol e.g. ethanol or methanol at atmospheric or elevated pressure and at a reaction temperature between +20 °C and +120 °C. 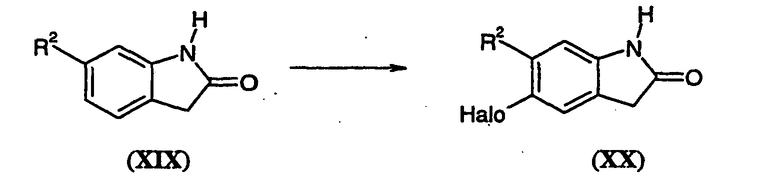
(xii) Halogenation of the compound of formula **XIX,** wherein R₂ is C₁₋₃alkyl and halo is halogeno as defined hereinbefore, to obtain a compound of formula **XX** may be performed by aromatic electrophilic substitution using a suitable halogenation agent such as Br₂, Cl₂, I₂, ICl, or SO₂Cl₂ or another suitable halogenation agent such as *N*-bromosuccinimid in an appropriate solvent e.g. acetonitrile, acetic acid, HCl/ethanol or water with or without a suitable base e.g. alkali metal acetate such as sodium acetate and at a reaction temperature between -20 °C and room temperature. 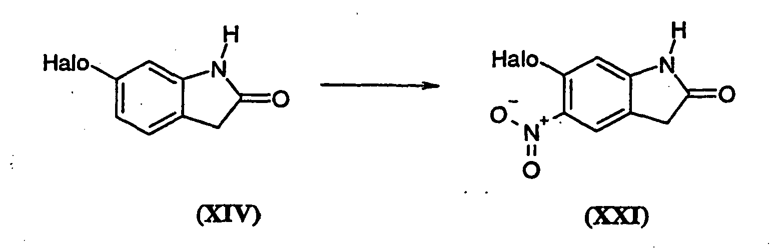
(xiii) Nitration of a compound of formula **XIV** wherein halo is halogeno, as defined hereinbefore, to obtain a compound of formula **XXI**, may be carried out by aromatic electrophilic substitution using a suitable nitration reagent such as potassium nitrate, nitric acid and sulphuric acid in a suitable solvent such as acetic acid, acetic anhydride, sulphuric acid or water at a reaction temperature between -20 °C and room temperature.

### Methods of Preparation of End products

A compound of general formula I, as a free base or a pharmaceutically acceptable salt thereof, can be prepared by
**i)**
   Reacting a compound of formula **B (IV, VI, VII, XI),** wherein L⁴ is a leaving group L¹ or SCH₃, with a compound of formula **C**, to obtain a compound of formula I, and R¹, R², R³, m and n are as defined in general formula **I** 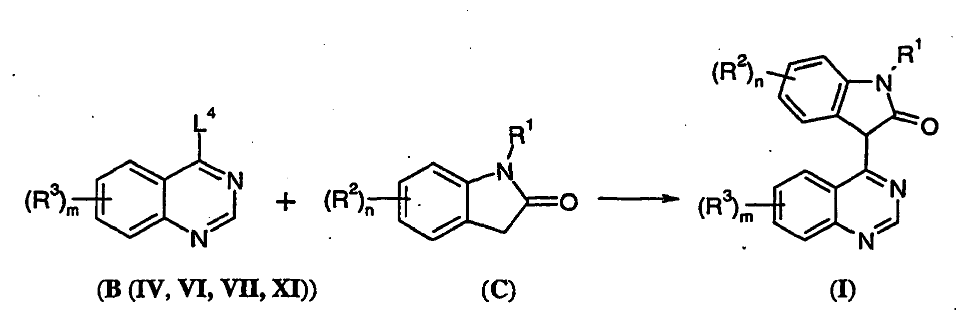 Thus, the reaction of the process may be carried out in an appropriate solvent such as an ether e.g. tetrahydrofuran or 1,4-dioxan, an aromatic hydrocarbon solvent such as toluene, or a dipolar aprotic solvent such as *N,N*-dimethylformamide, *N,N*-dimethylacetamide, *N*-methylpyrrolidin-2-one or dimethyl sulphoxide and the reaction is conveniently effected at a temperature in the range of +10 to +150 °C, preferably in the range of +20 to +90 °C. The reaction is advantageously effected in the presence of a base. Such a base may be an organic amine base such as pyridine, 2,6-lutidine, collidine, 4-dimethylaminopyridine, triethylamine, morpholine, *N*-methylmorpholine or diazabicyclo[5.4.0]undec-7-ene, tetramethylguanidine, an alkali metal or alkaline earth metal carbonate or hydroxide such as sodium carbonate, potassium carbonate, calcium carbonate, sodium hydroxide or potassium hydroxide. Alternatively, such a base is an alkali metal hydride such as sodium hydride, or an alkali metal or alkaline earth metal amide such as sodium amide, sodium bis(trimethylsilyl)amide, potassium amide or potassium bis(trimethylsilyl)amide. When it is desired to obtain the acid salt, the free base may be treated with an acid, using a conventional procedure.
**ii)**
   Hydrolysis of a compound of formula **Ia,** wherein R² is C₁₋₄alkoxycarbonyl to obtain a compound of formula **Ib,** wherein R² is carboxy and R¹, R³, m and n are as defined in general formula **I**. 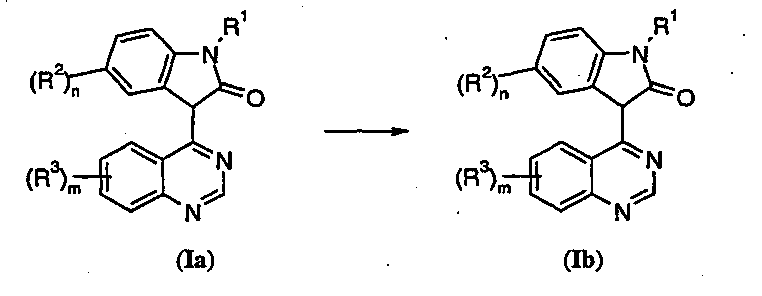 This reaction may be carried out under acidic conditions using acids such as sulfuric acid, hydrochloride or hydrobromide in a suitable solvent e.g. water, ethanol, methanol or mixtures thereof and the reaction may occur between +20 °C and +100 °C or under basic conditions using bases such as sodium hydroxide or potassium hydroxide in a suitable solvent e.g. water, ethanol, methanol or mixtures thereof and the reaction may occur between +20 °C and +100 °C.
**iii)**
   Amidation of a compound of formula **Ib,** wherein R² is carboxy to obtain a compound of formula **Ic,** wherein R² is R⁴X¹ and X¹ is CONR⁵R⁶ and R¹, R³, R⁴, R⁵, R⁶, m and n are as defined in general formula **I**

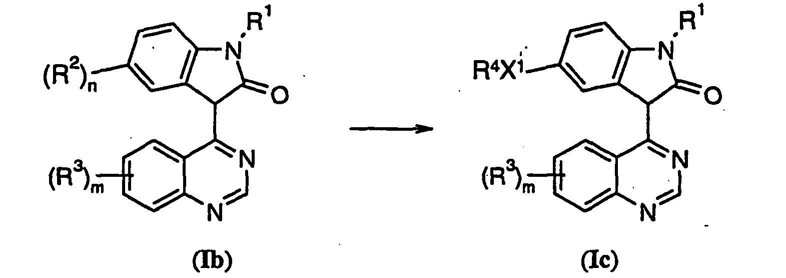

This reaction may be performed by activation of a compound of formula **Ib,** wherein R² is carboxy, by treating the compound with coupling reagents e.g.
1-[3-(dimethylamino)propyl]-3-ethylcarbodiimide hydrochloride and
1-hydroxybenzotriazole hydrate or hydroxybenzimidazole, 1,3-dicyclohexylcarbodiimide and 1-hydroxybenzotriazole hydrate, 1,1'-carbonyldiimidazole or *O*-(7-azabenzotriazol-1-yl)-*N,N,N',N*'-tetramethyluronium hexafluorophosphate, or using an acyl halide reagent e.g. cyanuric chloride, oxalyl chloride, thionyl chloride or : bromotrispyrrolidinophosphonium hexafluorophosphate, followed by treatment with the appropriate amine with or without the presence of *N,N*-dimethylaminopyridine, in a suitable solvent such as *N,N*-dimethylformamide, tetrahydrofuran, *N*-methylpyrrolidone, methylene chloride or chloroform at a reaction temperature between 0 °C and +80 °C.

### Examples

The invention will now be illustrated by the following Examples of compounds which might be used according to the claims.

### Example 1

### 7-(2-Morpholin-4-yl)ethoxy)-3H-quinazolin-4-one

A mixture of sodium hydride (12.7 g, 0.317 mol, 60% oil dispersion) and dimethyl sulfoxide (60 mL, 0.84 mol) was heated at 75 °C. After 30 min the hydrogen gas evolution had ceased and the reaction was cooled to room temperature.
4-(2-Hydroxyethyl)morpholine (48 mL, 0.40 mol) was added portionwise to the reaction mixture. After stirring for 30 min, 7-fluoro-3*H*-quinazolin-4-one (13.0 g, 79.2 mmol; described in Rewcastle G. et al J. Med. Chem, 1996, 39, 4, 918-928) was added and the reaction solution was heated for 3 h at 150 °C. The reaction mixture was cooled to room temperature and the resulting syrup was dissolved in ethyl acetate (500 mL) and triturated with diethyl ether (2 L). The solid was filtered off under a nitrogen atmosphere and washed several times with diethyl ether to obtain the crude product as a powder. The crude product was purified by flash chromatography (500 g silica gel column topped with a layer of celite) using methanol/methylene chloride systems (methanol/methylene chloride: 7:93 (4 L); 10:90 (2 L); 15:85 (2 L); 25:75 (4 L)) as stepwise gradient eluents. The fraction containing the product was concentrated to dryness, triturated with acetone and filtered to give 21.8 g (65% yield) of the title compound as an off-white solid: MS (AP+) *m*/*z* 276.0 (M⁺+1).

### Example 2

### 4-Chloro-7-[(2-morpholin-4-yl)ethoxy]quinazoline

Oxalyl chloride (4.55 mL, 52 mmol) was added dropwise to a suspension of 7-(2-morpholin-4-yl)ethoxy)-3*H*-quinazolin-4-one (11.9 g, 43.3 mmol) in methylene chloride (175 mL) followed by dropwise addition of *N,N*-dimethylformamide (1.5 mL).
The reaction mixture was heated for 2 h at reflux. The solvent was removed *in vacuo* and the resulting solid was triturated with diethyl ether. The pale yellow solid was filtered off under nitrogen atmosphere to give 17.2 g (99% yield) of the title compound as a pale yellow powder: MS (AP+) *m*/*z* 294.0 (M⁺+1).

### Example 3

### 2-Hydroxy-3-[7-(2-morpholin-4-ylethoxy)quinazolin-4-yl]-1H-indole-5-carbonitril dihydrochloride

Sodium hydride (490 mg, 12.2 mmol, 60% oil dispersion) was washed with petroleum ether (2x10 mL) and dried under vacuum and the obtained material was suspended in anhydrous *N,N*-dimethylformamide (5 mL) and 5-cyanooxindole (323 mg, 2.04 mmol) in *N,N*-dimethylformamide (3 mL) was added. The resulting suspension was stirred for 30 min at room temperature and 4-chloro-7-[(2-morpholin-4-yl)ethoxy]quinazoline (200 mg, 0.68 mmol) in *N,N*-dimethylformamide (5 mL) was added. The reaction mixture was stirred for 1 h at room temperature. The reaction was quenched with aqueous hydrochloric acid (5 mL, 1 M) and *N,N*-dimethylformamide was removed *in vacuo*. To the resulting syrup was added water (50 mL) and the mixture were stirred vigorously. The solid formed was filtered off and dried at 70 °C under vacuum over night. The crude product was refluxed in methanol for 15 min and the insoluble material was filtered off and dried at 85 °C under vacuum over night to give 225 mg (68% yield) of the title compound as an orange powder: MS (AP-) *m*/*z* 413.9 (M-1); Anal. (C₂₃H₂₁N₅O₃x2HClx0.1H₂O) C, H, N.

### Example 4

### Methyl 2-Hydroxy-3-[7-(2-methoxyethoxy)quinazolin-4-yl]-1H-indole-5-carboxylate

Sodium hydride (58 mg, 1.45 mmol, 60% in oil) was washed with petroleum ether (3x5 mL) and dried *in vacuo*. The solid was suspended in tetrahydrofuran (3 mL) and methyl 2-oxo-5-indolinecarboxylate (140 mg, 0.73 mmol) in tetrahydrofuran (2 mL) and *N*-methylpyrrolidinone (2 mL) was added. The reaction mixture was stirred for 30 min at room temperature. A solution of 4-chloro-7-(2-methoxyethoxy)quinazoline (183 mg, 0.77 mmol, described in WO 97/42187) in tetrahydrofuran (2 mL) and *N*-methylpyrrolidinone (1 mL) was added and the reaction mixture was stirred for 1.5 h at room temperature. The solvent was removed *in vacuo* and 1 M hydrochloric acid was added. The precipitate formed was filtered off and dried at 40 °C *in vacuo* over night to give 150 mg (99% yield) of the title compound as an orange solid: MS (AP+) *m*/*z* 394.2 (M⁺+1).

### Example 5

### 2-Hydroxy-3-[7-(2-methoxyethoxy)quinazolin-4-yl]-1H-indole-5-carboxylic Acid

To a mixture of methyl 2-hydroxy-3-[7-(2-methoxyethoxy)quinazolin4-yl]-1*H*-indole-5-carboxylate (5.15 g, 13.1 mmol), methanol (100 mL) and water (50 mL) was added aqueous sodium hydroxide (92 mL, 1 M) and the reaction mixture was stirred at 40°C over night. Methanol was removed *in vacuo* and the basic aqueous layer was acidified with 1 M hydrochloric acid and stirred for 30 min. The precipitate formed was filtered off, washed with hydrochloric acid (50 mL, 1 M) and water (2x50 mL) and dried *in vacuo* at 50 °C over night. The crude product was stirred in methanol at room temperature over night. The solid was filtered off to give 4.23 g (85% yield) of the title compound as an orange solid: MS (AP+) *m*/*z* 380.3 (M⁺+1).

### Examples 6-11

### General Method A

Stock solution A was prepared by dissolving 2-hydroxy-3-[7-(2-methoxyethoxy)-quinazolin-4-yl]-1*H*-indole-5-carboxylic acid (2.0 g), (3-dimethylaminopropyl)-3-ethylcarbodiimide hydrochloride (2.2 g) and hydroxybenzimidazole (1.54 g) in *N*-methylpyrrolidinone (160 mL). Stock solution B was prepared by dissolving *N,N*-dimethylaminopyridine (2.8 g) in *N*-methylpyrrolidinone (40 mL). The amidation reaction was performed by adding solution A (8 mL, corresponding to 2-hydroxy-3-[7-(2-methoxyethoxy)-quinazolin-4-yl]-1*H*-indole-5-carboxylic acid: 100 mg, 0.26 mmol, 1eq; (3-dimethylaminopropyl)-3-ethylcarbodiimide hydrochloride: 110 mg, 0.51 mmol, 2.2 eq; hydroxybenzimidazole: 77 mg, 0.57 mmol, 2.2 eq) to a reaction vessel containing the desired amine (0.4 mmol, 1.5 eq). Solution B (2 mL, corresponing to *N,N*-dimethylaminopyridine: 140 mg, 1.14 mmol, 4.4 eq) was added and the resulting solution was stirred at room temperature over night. The solvent was removed *in vacuo* to give the crude product.

### Example 6

### 2-Hydroxy-3-[7-(2-methoxymethoxy)quinazolin-4-yl]-1H-indole-5-carboxylic Acid [2-(1-Methylpyrrolidin-2-yl)ethyl]amide

The reaction was performed as described in method A using 2-(2-aminoethyl)-1-methylpyrrolidine (0.06 mL, 0.40 mmol). The crude product was triturated with acetonitrile. The solid was decanted and washed with methanol/diethyl ether to give 36 mg (28% yield) of the title compound: MS (AP+) *m*/*z* 490.4 (M⁺+1).

### Example 7

### 2-Hydroxy-3-[7-(2-methoxyethoxy)quinazolin-4-yl]-1H-indole-5-carboxylic Acid (Tetrahydrofuran-2-ylmethyl)amide

The reaction was performed as described in method A using tetrahydro-2-furanylmethylamine (0.06 mL, 0.395 mmol). The crude product was triturated with acetone and the solid was washed with hot methanol to give 19 mg (16 % yield) of the title compound: MS (AP+) *m*/*z* 537.3 (M⁺+1).

### Example 8

### 2-Hydroxy-3-[7-(2-methoxyethoxy)quinazolin-4-yl]-1H-indole-5-carboxylic Acid (3-Morpholin-4-yl-propyl)amide

The reaction was performed as described in method A using 3-(morpholin-4-yl)propanamine (0.06 mL, 0.395 mmol). The crude product was triturated with acetonitrile and the solid was washed with hot methanol to give 15 mg (12% yield) of the title compound: MS (AP+) *m*/*z* 506.3 (M⁺+1).

### Example 9

### 2-Hydroxy-3-[7-(2-methoxyethoxy)quinazolin-4-yl]-1H-indole-5-carboxylic Acid (4-Phenylbutyl)amide

The reaction was performed as described in method A using 4-phenylbutylamine (0.06 mL, 0.395 mmol). The crude product was triturated with acetone and the solid was washed with hot methanol to give 38 mg (29% yield) of the title compound: MS (AP+) *m*/*z* 511.3 (M ⁺+1).

### Example 10

### 2-Hydroxy-3-[7-(2-methoxyethoxy)quinazolin-4-yl]-1H-indole-5-carboxy lic Acid [2-(1H-Imidazol-4-yl)ethyl]amide

The reaction was performed as described in method A using 2-(1*H*-imidazol-4-yl)ethylamine dihydrochloride (73 mg, 0.395 mmol). The crude product was triturated with acetonitril. The solid decanted and triturated with acetonitrile/methanol/diethyl ether and washed with hot methanol to give 26 mg (21 % yield) of the title compound: MS (AP+) *m*/*z* 473.3 (M⁺+1).

### Example 11

### 7-[2-(2-Methoxyethoxy)ethoxy]-3H-quinazolin-4-one

Dimethyl sulfoxide (6.9 mL, 97 mmol) was added to sodium hydride (1.46 g, 36.6 mmol) and the formed foamy suspension was heated at 75 °C for 30 min until the gas evolution had ceased. The formed green cloudy solution was cooled to room temperature and 2-(2-methoxyethoxy)ethanol (5.44 mL, 45.7 mmol) was added slowly and the mixture was stirred for 30 min. 7-Fluoro-3*H*-quinazolin-4-one (1.5 g, 9.14 mmol; described in Rewcastle G. et al J. Med. Chem, 1996, 39, 4, 918-928) was added and heated at 150 °C for 4 h. The reaction mixture was cooled and diluted with ethyl acetate (60 mL) followed by the addition of diethyl ether (200 mL). The formed precipitate was filtered under nitrogen atmosphere and washed with diethyl ether. The hygroscopic crude product was purified on a silica gel column using chloroform/methanol (20: 1), as the eluent to give 1.2 gram (50% yield) of the title compound as a slightly coloured solid: MS (TSP) *m*/*z* 265 (M⁺+1).

### Example 12

### 4-Chloro-7-[2-(2-methoxyethoxy)ethoxy]quinazoline

To a solution of 7-[2-(2-methoxyethoxy)ethoxy]-3H-quinazolin-4-one (0.86 g, 3.25 mmol) in methylene chloride (15 mL) was added oxalyl chloride (0.34 mL, 3.9 mmol) and dimethyl sulfoxide (0.1 mL) and the reaction was heated at reflux for 2 h. The solvent was evaporated *in vacuo* and the residue was washed with diethyl ether and dried *in vacuo* to give 0.9 gram of the title compound as a semi-solid: MS (TSP) *m*/*s* 283 (M⁺+1).

### Example 13

### 2-Hydroxy-3-{7-[2-(2-methoxyethoxy)ethoxy]quinazolin-4-yl}-1H-indole-5-carbonitrile hydrochloride

To a suspension of sodium hydride (0.52 g, 12.8 mmol, pre-washed with hexane) in tetrahydrofuran (6 mL) was added a solution of 5-cyanooxindole (1 g, 6.4 mmol) in tetrahydrofuran (11 mL) and 1-methyl-2-pyrrolidone (11 mL). The mixture was stirred for 30 min under nitrogen atmosphere. 4-Chloro-7-[2-(2-methoxyethoxy)ethoxy]quinazoline (0.9 g, 3.2 mmol) dissolved in 1-methyl-2-pyrrolidone (10 mL) was added dropwise and the dark red solution was stirred at ambient temperature for 1.5 h. The solvent was evaporated *in vacuo* until approximately 10 mL of 1-methyl-2-pyrrolidone remained, whereupon aqueous hydrochloric acid (1 M, 25 mL) was added. The formed precipitation was filtered and washed with 1 M hydrochloric acid. Drying *in vacuo* afforded 1.1 gram (78% yield) of the title compound as an orange solid: MS (TSP) *m*/*s* 405 (M⁺+1)

### Example 14

### 3-[7-(2-Imidazol-yl-ethoxy)-6-methoxyquinazolin-4-yl]-2-oxo-2,3-dihydro-1H-indole-5-sulfonamide acetate

Sodium hydride (59 mg, 1.48 mmol, pre-washed with pentane) was added to a suspension of 2-oxo-2,3-dihydro-1*H*-indole-5-sulfonamide (313 mg, 1.48 mmol; described in WO 9742187) in *N,N*-dimethylformamide (2.5 mL). After stirring 30 min at ambient temperature, 4-chloro-7-(2-(imidazol-1-yl)ethoxy)-6-methoxyquinazoline (150 mg, 0.49 mmol; described in WO 97/42187) was added followed by dimethyl sulfoxide (0.5 mL). After stirring for 10 min at ambient temperature, the mixture was stirred at 60 °C for 2 h. The mixture was poured onto diethyl ether/water (1:1, 100 mL). The organic layer was separated and the aqueous layer was adjusted to pH 6.3 (5 M HCl_{(aq)}). The precipitate was filtered, washed with water, followed by diethyl ether, methylene chloride and dried *in vacuo*. The solid was dissolved in water adjusted to pH 1.5 (5 M HCl_{(aq)} and the solution was purified by preparative C 18 HPLC eluting with methanol/water (1:9, containing 1% acetic acid) followed by 15:85 followed by 4:6. The fractions containing the expected product were combined, concentrated and the product was freeze-dried and dried in *vacuo* at 60 °C for 48 h to give 133 mg (54% yield) of the title compound: EIMS *m*/*z* 480 (M⁺).

### Example 15

### 6-Chloro-3-[7-(3-morpholin-4-yl-propoxy)quinazolin-4-yl]-1,3-dihydro-indol-2-one hydrochloride

6-Chlorooxindole (383 mg, 2.28 mmol) was added to a suspension of sodium hydride (92 mg, 2.28 mmol) in *N,N*-dimethylformamide (3 mL). After stirring for 20 main at ambient temperature, 4-chloro-7-(3-(morpholin-4-yl)propoxy)quinazoline (233 mg, 0.76 mmol; described in WO 97/42187) in *N,N*-dimethylformamide (3 mL) was added. The mixture was stirred at 70 °C for 45 min. After cooling, the volatiles were removed *in vacuo* and the residue was partitioned between ethyl acetate and water. The pH of the aqueous layer was adjusted to 8.4 with 2 M HCl. The organic layer was separated and washed with brine, dried (MgSO₄) and evaporated. The residue was purified by column chromatography eluting with methanol/methylene chloride, 5:95, followed by 10:90. The fractions containing the expected product were combined and evaporated. The residue was dissolved in methylene chloride (10 mL) and methanol (3 mL) and HCl in diethyl ether (3.8 M, 0.5 mL) was added. The precipitate was filtered, washed with diethyl ether and dried in *vacuo* to give 141 mg (34% yield) of the title compound: ESI-MS 439 (M⁺+1).

### Example 16

### 6-Bromo-3-[6-methoxy-7-(3-morpholin-4-ylpropoxy)quinazolin-4-yl]-1,3-dihydroindol-2-one dihydrochloride

Sodium hydride, (71 mg, 1.78 mmol, 60% in oil) was added to a solution of 6-bromooxindole (126 mg, 0.59 mmol) in *N,N*-dimethylformamide (3mL). After stirring for 15 min at ambient temperature, 4-chloro-6-methoxy-7-(3-(morpholin-4-yl)propoxy)quinazoline (200 mg, 0.59 mmol; described in WO 97/42187) in *N,N-*dimethylformamide (4 mL) and tetrahydrofuran (4 mL) was added. After stirring at 60 °C for 2.5 h, water was added and the volatiles were removed *in vacuo*. Water (50 mL) was added and the pH of the solution was adjusted to 8.5 with aqueous hydrochloric acid (2 M). Ethyl acetate was added. The organic layer was separated, washed with brine, dried (MgSO₄) and evaporated. The residue was purified by column chromatography eluting with methanol/ methylene chloride, 5:95, followed by 8:92. The fractions containing the expected product were combined and evaporated. The solid was dissolved in methylene chloride (10 mL) and methanol (3 mL) and 3.8 M HCl in diethyl ether (1 mL) was added. The solution was concentrated and the solid was filtered to give 70 mg (23% yield) of the title compound: ESI-MS: 513-515 (M⁺+1).

### Example 17

### 6-Bromo-3-quinazolin-4-yl-1,3-dihydroindol-2-one hydrochloride

To a suspension of sodium hydride (117 mg, 2.9 mmol, pre-washed with pentane) in *N,N*-dimethylformamide (1.5 mL was added a solution of 6-bromooxindole (618 mg, 2.9 mmol) in *N,N*-dimethylformamide (4 mL). After stirring 15 min at ambient temperature, 4-chloroquinazoline (618 mg, 2.9 mmol) in *N,N*-dimethylformamide (4 mL) was added and the mixture was stirred at 80 °C for 45 min. After cooling, the volatiles were removed under vacuum and the residue was suspended in water and the pH was adjusted to 7 with aqueous hydrochloric acid (2 M). The precipitate was filtered, washed with water and dried over P₂O₅ over night. The solid was dissolved in methanol/methylene chloride and adsorbed on silica and purified by column chromatography, eluting with methylene chloride/methanol, 99:1, followed by 98:2 and 95:5. The fractions containing the expected product were combined and evaporated. The product was purified a second time on silica eluting with methylene chloride/methanol, 97:3, followed by 95:5. The solid was the suspended in methylene chloride/methanol, 1:1, and HCl in diethyl ether (3.6 M, 1 mL) was added. The precipitate was filtered, washed with diethyl ether and dried *in vacuo* to give 270 mg (74% yield) of the title compound: ESI-MS 340-342 (M⁺+1)

### Example 18

### 6-Bromo-3-{6-methoxy-7-[2-(2-methoxyethoxy)ethoxy]quinazolin-4-yl}-1,3-dihydroindol-2-one hydrochloride

To a suspension of sodium hydride (48 mg, 1.2 mmol, pre-washed with pentane) in dimethyl sulfoxide (1 mL) was added a solution of 6-bromooxindole (255 mg, 1.2 mmol) in dimethyl sulfoxide (2 mL). After stirring for 15 min, 6-methoxy-7-[2-(2-methoxyethoxy)ethoxy]-4-(methylthio)quinazoline (130 mg, 0.4 mmol; described in WO 97/42187) was added. The mixture was stirred at 100 °C for 3 h. After cooling, the mixture was partitioned between ethyl acetate and water. The pH of the aqueous layer was adjusted to 7 with aqueous hydrochloric acid (2 M). The organic layer was separated, washed with brine, dried (MgSO₄) and evaporated. The residue was purified by column chromatography eluting with methylene chloride/methanol, 93:7. The fractions containing the expected product were combined and evaporated. The solid was dissolved in methylene chloride/methanol and HCl in diethyl ether (3.8 M, 2 mL) was added. The solution was concentrated under vacuum and the solid was filtered, washed with diethyl ether and dried *in vacuo* to give 109 mg (52% yield) of the title compound: ESI-MS 488-490 (M⁺+1).

### Example 19

### 3-{7-[2-(2-Morpholin-4-yl-ethoxy)ethoxy]quinazolin-4-yl}-2-oxo-2,3-dihydro-1H-indole-5-carbonitrile hydrochloride

A solution of 5-cyanooxindole (240 mg, 1.5 mmol) was added portion wise to a suspension of sodium hydride (60 mg, 1.5 mmol, pre-washed with pentane) in dimethyl sulfoxide (2 mL). After stirring for 30 min at ambient temperature, 4-(methylthio)-7-[2-(2-morpholin-4-ylethoxy)ethoxy]quinazoline (175 mg, 0.5 mmol) was added. The mixture was stirred at 100 °C for 2 h. After cooling, water was added and the pH was adjusted to 7 with aqueous hydrochloric acid (2.5 M). The precipitate was filtered, washed with water and dried over night *in vacuo* over P₂O₅. The solid was purified by column chromatography eluting with methanol/methylene chloride, 5:95, followed by 10:90. The fractions containing the expected product were combined and evaporated. The solid was dissolved in methanol/methylene chloride, 1:1, and HCl in diethyl ether (3.5 M, 0.5 mL) was added, followed by diethyl ether. The precipitate was filtered, washed with diethyl ether and dried *in vacuo* to give 155 mg (53% yield) of the title compound: ESI-MS 460 (M⁺+1).

### Example 20

### 4-(Methylthio)-7-[2-(2-morpholin-4-ylethoxy)ethoxy]quinazoline

Diethyl azodicarboxylate (2.46 mL, 15.6 mmol) was added dropwise to a suspension of 4-(methylthio)-7-hydroxyquinazoline (1.2 g, 6.25 mmol; described in WO 99/10349), triphenylphosphine (4.09 g, 15.6 mmol) and 2-(2-morpholin-4 ylethoxy)ethanol (1.42 g, 8.12 mmol; described in J. Med. Chem,. 1994, 37, 15, 2285-2291) in methylene chloride (30 mL). After stirring 1.5 h at ambient temperature, the volatiles were removed *in vacuo*. The residue was dissolved in ethyl acetate/methylene chloride, filtered and poured onto a column of silica. The product was eluted with methylene chloride/methanol, 97:3, followed by 95:5. The fractions containing the expected product were combined and evaporated. The residue was triturated with diethyl ether and the solid was filtered and dried *in vacuo* to give 1.1 g (49% yield) of the title compound: ESI-MS 350 (M⁺+1).

### Example 21

### 6-Chloro-3-{7-[2-(2-methoxyethoxy)ethoxy]quinazolin-4-yl}-1,3-dihydro-indol-2-one hydrochloride

6-Chlorooxindole (285 mg, 1.7 mmol) was added to a suspension of sodium hydride (102 mg, 2.55 mmol, pre-washed with pentane) in dimethyl sulfoxide (4 mL). After stirring 30 min at ambient temperature, a solution of 7-[2-(2-methoxyethoxy)ethoxy]-4-(methylthio)quinazoline (250 mg, 0.85 mmol; described in WO 99/10349) in dimethyl sulfoxide (2 mL) was added and the mixture was stirred at 90 °C for 1 h. The mixture was poured onto water (20 mL) and aqueous hydrochloric acid (2 M, 5 mL). The precipitate was filtered, washed with water and dried over P₂O₅. The solid was triturated with diethyl ether, filtered, washed with diethyl ether and dried *in vacuo* over night. The solid was dissolved in methylene chloride/methanol and HCl in diethyl ether (3.8 M) was added. The solution was concentrated and the solid was filtered and dried *in vacuo* to give 231 mg (60% yield) of the title compound: ESI-MS 414-416 (M⁺+1).

### Example 22

### 3-{7-[2-(2-Methoxyethoxy)ethoxy]quinazolin-4-yl}-1,3-dihydroindol-2-one hydrochloride

The compound was prepared as descirbed for Example 29 using 7-[2-(2-methoxyethoxy)ethoxy]-4-(methylthio)quinazoline (257 mg, 1.93 mmol; described in WO 99/10349) and oxindole (190 mg, 0.64 mmol) to give 191 mg (70% yield) of the title compound as the hydrochloride: ESI-MS 380 (M⁺+1).

### Example 23

### 3-{7-[2-(4-Acetylpiperazin-1-yl)ethoxy]quinazolin-4-yl}-2-oxo-2,3-dihydro-1H-indole-5-carbonitrile hydrochloride

A solution of 5-cyanooxindol (260 mg, 1.64 mmol) in dimethyl sulfoxide (2 mL) was added to a suspension of sodium hydride (66 mg, 1.64 mmol, pre-washed with pentane) in dimethyl sulfoxide (0.8 mL). The mixture was stirred for 20 min at ambient temperature and 7-[2-(4-acetylpiperazin-1-yl)ethoxy]-4-methylthioquinazoline (190 mg, 0.55 mmol) was added and stirring was continued for 3 h at 100 °C. The mixture was poured onto a saturated aqueous solution of ammonium chloride and ethyl acetate was added. The formed precipitate was filtered and the filtrate was extracted with ethyl acetate. The ethyl acetate layers were dried (Na₂SO₄) and evaporated to give a solid. The solid and the initial precipitate were dissolved in methylene chloride/methanol and adsorbed on silica. The product was purified by column chromatography eluting with methylene chloride/methanol, 9:1. The fractions containing the expected product were combined and evaporated. The oily residue was dissolved in methylene chloride/methanol, 1:1, and 3.8 M HCl in diethyl ether (2 mL) was added. The solution was concentrated and the precipitate was filtered, washed with diethyl ether and dried *in vacuo* to give 189 mg (63% yield) of the title compound: ESI-MS 457 (M⁺+1).

### Example 24

### 7-[2-(4-Acetylpiperazin-1-yl)ethoxy]-4-methylthioquinazoline

7-(2-Bromoethoxy)-4-methylthioquinazoline (250 mg, 0.83 mmol) and acetylpiperazine (225 mg, 1.75 mmol) was stirred at 120 °C for 10 min. The solid was dissolved in ethyl acetate and water and the pH of the aqueous layer was adjusted to 9 with 2 M NaOH_{(aq)}. The organic layer was separated and the aqueous layer was extracted with ethyl acetate. The organic layers were combined, washed with brine, dried (MgSO₄) and evaporated and the residue was purified by column chromatography eluting with methylene chloride/methanol, 92:8, to give 226 mg (78% yield) of the title compound: ESI-MS 347 (M⁺+1).

### Example 25

### 5-Chloro-3-{7-[2-(2-methoxyethoxy)ethoxy]quinazolin-4-yl}-1,3-dihydro-indol-2-one hydrochloride

5-Chlorooxindol (230 mg, 1.14 mmol) was added to a suspension of sodium hydride (92mg, 2.28 mmol; 60% in oil) in dimethyl sulfoxide (4 mL). After stirring 15 min at ambient temperature, 7-[2-(2-methoxyethoxy)ethoxy]-4-(methylthio)quinazoline (223 mg, 0.76 mmol, described in WO 99/10349) was added. The mixture was stirred at 110 °C for 1 h and then poured onto water (75 mL). The pH was adjusted to 8 with aqueous hydrochloric acid (2 M). The precipitate was filtered, washed with water and dried *in vacuo*. The solid was dissolved in methylene chloride/methanol and HCl in diethyl ether (3.8 M, 2 mL) was added. The volatiles were removed *in vacuo* to give 233 mg (67% yield) of the title compound: ESI-MS 414 (M⁺+1).

### Example 26

### 3-{7-[2-(4-Butyrylpiperazin-1-yl)ethoxy]quinazolin-4-yl}-2-oxo-2,3-dihydro-1H-indole-5-carbonitrile hydrochloride

5-Cyanooxindol (221 mg, 1.4 mmol) in dimethyl sulfoxide (1.7 mL) was added to a suspension of sodium hydride (56 mg, 1.4 mmol, pre-washed with pentane) in dimethyl sulfoxide (0.8 mL). After stirring 10 min at ambient temperature, 7-[2-(4-butyrylpiperazin-1-yl)ethoxy]-4-(methylthio)quinazoline (175 mg, 0.47 mmol) was added and the mixture was stirred at 100 °C for 3 h. After cooling, the mixture was partitioned between ethyl acetate and water and the pH was adjusted to 8.2 with aqueous hydrochloric acid (2 M). The precipitate was filtered, washed with water followed by ethyl acetate. The aqueous layer was extracted with ethyl acetate and the ethyl acetate layers were combined and evaporated to give a solid. The solid and the first precipitate were combined, dissolved in ethyl acetate and methanol and adsorbed on silica. The product was purified by column chromatography eluting with methylene chloride/acetonitrile/methanol, 60:42:8 followed by 70:20: 10. The fractions containing the expected product were combined and evaporated. The solid was dissolved in methylene chloride/methanol and 3.8 M HCl in diethyl ether was added. The volatiles were removed under vacuum and the solid was filtered, washed with diethyl ether and dried *in vacuo* to give 195 mg (74% yield) of the title compound: ¹HNMR (DMSO-*d6*, TFA-*d*) δ 0.92 (t, 3 H), 1.55 (m, 2 H), 2.38 (dd, 2 H), 2.9-3.7 (br m, 6 H), 3.72 (br s, 2 H), 4.05-4.2 (br s, 1 H), 4.4-4.6 (br s, 1 H), 4.65 (m, 2 H), 7.1 (d, 1 H), 7.25 (d, 1 H), 7.38 (dd, 1 H), 7.5 (d, 1 H), 8.1 (s, 1 H), 8.65 (d, 1 H), 8.7 (s, 1 H); ESI-MS 485 (M⁺+1).

### Example 27

### 7-(2-Bromoethoxy)-4-(methylthio)guinazoline

7-Hydroxy-4-(methylthio)quinazoline (1 g, 5.2 mmol; described in WO 99/10349), 1,2-dibromoethane (673 µL, 7.8 mmol) and potassium carbonate (2.1 g, 15 mmol) in *N,N*-dimethylformamide (15 mL) was stirred for 6 h at ambient temperature. Additional 1,2-dibromoethane (224 µL) was added and stirring was continued over night. The solid was filtered and washed with ethyl acetate. The filtrate was washed with water, brine, dried (MgSO₄) and evaporated. The residue was purified by column chromatography eluting with methylene chloride/methanol, 98:2. The fraction containing the expected product were combined and evaporated to give 179 mg (11% yield) of the title compound: ESI-MS 299-301 (M⁺+1).

### Example 28

### 7-[2-(4-Butyrylpiperazin-1-yl)ethoxy]-4-(methylthio)quinazoline

7-(2-Bromoethoxy)-4-(methylthio)quinazoline (0.55 g, 1.8 mmol) and 1-butyrylpiperazine (0.6 g, 3.86 mmol) was stirred at 120 °C. The mixture was then diluted with water and methylene chloride and the pH was adjusted to 9 with 2 N aqueous Na₂CO₃. The organic layer was separated and the aqueous layer was further extracted with methylene chloride. The organic layer was combined, washed with brine, dried (MgSO₄) and evaporated. The residue was purified by column chromatography eluting with methylene chloride/methanol, 94:6. The fractions containing the expected product were combined and evaporated to give 461 mg (68% yield) of the title compound: ESI-MS 375 (M⁺+1).

### Example 29

### 3-{7-[2-(4-Acetylpiperazin-1-yl)-2-oxoethoxy]quinazolin-4-yl}-2-oxo-2,3-dihydro-1H-indole-5-carbonitrile hydrochloride

A solution of 5-cyanooxindol (329 mg, 2.1 mmol) in dimethyl sulfoxide (3 mL) was added to a suspension of sodium hydride (83 mg, 2.1 mmol, 60% in oil) in dimethyl sulfoxide (1 mL). After stirring 10 min at ambient temperature, 7-[2-(4-acetylpiperazin-1-yl)-2-oxoethoxy]-4-(methylthio)quinazoline (250 mg, 0.69 mmol) was added. After stirring at 100 °C for 2 h, the mixture was poured onto saturated aqueous ammonium chloride. The precipitate was filtered, washed with water followed by ethyl acetate and dried *in vacuo*. The solid was dissolved in methylene chloride/methanol and dried over Na₂SO₄. After filtration, the filtrate was concentrated and the residue was adsorbed onto silica. The product was purified by column chromatography, eluting with methylene chloride/methanol, 93:7, followed by 90:10. The fractions containing the expected product were combined and evaporated. The solid was dissolved in methylene chloride/methanol and 3.8 M HCl in diethyl ether was added (2 mL). The volatiles were removed *in vacuo* and the solid was filtered, washed with diethyl ether and dried *in vacuo* to give 189 mg (52% yield) of the title compound: ESI-MS 471 (M⁺+1).

### Example 30

### 7-[2-(4-Acetylpiperazin-1-yl)-2-oxoethoxy]-4-(methylthio)quinazoline

7-Hydroxy-4-methylthioquinazoline (600 mg, 3.12 mmol; described in WO 99/10349) was added to a suspension of sodium hydride (131 mg, 3.28 mmol, pre-washed with pentane) in *N,N*-dimethylformamide (7mL). After stirring for 15 min, 1-acetyl-4-bromoacetylpiperazine (855 mg, 3.43 mmol; described in Chem. Europ. J., 2001, 7, 1, 288-296) was added and the mixture was stirred at 50 °C for 1 h. The mixture was poured onto water containing some ethyl acetate.The precipitate was filtered , washed with water followed by diethyl ether and dried over night *in vacuo* over P₂O₅ to give 975 mg (99% yield) of the title compound: ESI-MS 361 (M⁺+1).

### Example 31

### 3-{7-[4-(4-Acetylpiperazin-1-yl)-4-oxobutoxy]quinazolin-4-yl}-2-oxo-2,3-dihydro-1H-indole-5-carbonitrile hydrochloride

The compound was prepared as described for Example 29 using 5-cyanooxindole (221 mg, 1.4 mmol) and 7-[4-(4-acetylpiperazin-1-yl)-4-oxobutoxy]-4-(methylthio)quinazoline (181 mg, 0.466 mmol). After extraction and purification by column chromatography eluting with methylene chloride/ acetonitrile/methanol, 60:32:8, followed by 70:20:10 and trituation with HCl in diethyl ether to give 165 mg (62% yield) of the title compound: ESI-MS 499 (M⁺+1).

### Example 32

### 1-Acetyl-4-(4-chlorobutanoyl)piperazine

A solution of 4-chlorobutanoyl chloride (1.54 mL, 13.7 mmol) in methylene chloride (5 mL) was added dropwise over 30 min to a solution of acetylpiperazine (1.6 g, 12.5 mmol) in methylene chloride (25 mL) cooled at -70 °C. After stirring 1 h at -70 °C and 2.5 h at ambient temperature, the mixture was poured onto a saturated solution of ammonium chloride and the pH of the aqueous layer was adjusted to 8 with solid Na₂CO₃. The organic layer was separated and the aqueous layer was extracted with methylene chloride. The organic layers were combined, dried (MgSO₄) and evaporated. The residue was purified by column chromatography eluting with methylene chloride/methanol, 98:2, followed by 96:2 to give 2.51 g (86% yield) of the title compound: ESI-MS 233-235 (M⁺+1).

### Example 33

### 7-[4-(4-Acetylpiperazin-1-yl)-4-oxobutoxy]-4-(methylthio)quinazoline

7-Hydroxy-4-(methylthio)quinazoline (600 mg, 3.12 mmol; described in WO 99/10349) was added to a suspension of sodium hydride (131 mg, 3.28 mmol, pre-washed with pentane) in *N,N*-dimethylformamide (7 mL). After stirring for 15 min, a solution of 1-acetyl-4-(4-chlorobutanoyl)piperazine (1.1 g, 3.43 mmol) in *N,N*-dimethylformamide (2 mL) was added. The mixture was stirred for 4 h at 50 °C and 1 h at 70 °C. The mixture was poured onto a saturated aqueous solution of ammonium chloride and extracted with ethyl acetate. The organic layer was dried (MgSO₄), filtered and evaporated. The residue was purified by column chromatography eluting with methylene chloride/acetonitrile/methanol, 60:39:1, to give 400 mg (33% yield) of the title compound: ESI-MS 389 (M⁺+1).

### Example 34

### 6-Fluoro-3-[7-(3-morpholin-4-ylpropoxy)quinazolin-4-yl]-1,3-dihydro-indol-2-one dihydrochloride

To a suspension of sodium hydride (36 mg, 1.5 mmol, pre-washed with pentane) in dimethyl sulfoxide (2 mL) was added 6-fluorooxindole (226 mg, 1.5 mmol). The mixture was stirred 30 min at ambient temperature and 4-methylsulfanyl-7-(3-morpholin-4-ylpropoxy)quinazoline (182 mg, 0.5 mmol; described in WO 97/42187) was added. The mixture was stirred at 80 °C for 1.5 h and 6-fluorooxindole (76 mg, 0.5 mmol) was further added. The mixture was stirred at 80 °C for 30 min. After cooling the mixture was poured onto a column of silica and was eluted with methylene chloride/methanol (gradient from 100->0 to 94->6). The fractions containing the expected product were combined and concentrated. 3.8 M HCl in diethyl ether (2 mL) was added and the volatiles were removed *in vacuo*. The solid was filtered, washed with diethyl ether and dried in *vacuo* to give 52 mg (34% yield) of the title compound: ¹HNMR (DMSO-*d6*, TFA-*d*) δ 2.2-2.35 (m, 2 H), 3.1-3.2 (m, 2 H), 3.3-3.4 (m, 2 H), 3.51 (d, 2 H), 3.75 (dd, 2 H), 4.02 (d, 2 H), 4.3 (dd, 2 H), 6.72-6.85 (m, 2 H), 7.22 (s, 1 H), 7.3 (d, 1 H), 7.68 (m, 1 H), 8.42 (d, 1 H), 8.8 (s, 1 H).

### Example 35

### 7-Fluoro-3-[6-methoxy-7-(3-morpholin-4-ylpropoxy)quinazolin-4-yl]-1,3-dihydroindol-2-one dihydrochloride

A stirred solution of 7-fluorooxindole (570 mg, 3.77 mmol) in *N,N*-dimethylformamide (15 mL) was degassed by subjecting it to several cycles of vacuum and argon and stirred in an argon atmosphere whilst sodium hydride (175 mg, 4.38 mmol, 60% dispersion in oil) was added in one portion. After stirring for a further 30 min , 4-chloro-6-methoxy-7-(3-(4-morpholinyl)propoxy)quinazoline (420 mg, 1.24 mmol; described in WO 97/42187) was added, the reaction mixture stirred at 90 °C for 1 h and allowed to cool to room temperature. The solvent was evaporated *in vacuo* and the residue absorbed onto silica from a methylene chloride/methanol, 95:5, solvent mixture. The product was purified by column chromatography eluting initially with methylene chloride/methanol, 95:5, and then with methylene chloride/methanol, 90:10. After evaporation of the solvent, the formed solid was triturated with acetone, filtered and dried to give 220 mg (39% yield) of the title compound: mp 211-214 °C; ESI-MS 453 (M⁺+1).

### Example 36

### 6-Bromo-3-[7-(2-imidazol-1-ylethoxy)-6-methoxyquinazolin-4-yl]-1,3-dihydroindol-2-one dihydrochloride

7-(2-Imidazol-1-yl-ethoxy)-6-methoxy-3*H*-quinazolin-4-one (300 mg, 1.05 mmol, described in WO 97/42187) and thionyl chloride (10 mL) containing *N,N*-dimethylformamide (5 drops) was refluxed for 1 h. The volatiles were removed *in vacuo* and the residue was dissolved in ethyl acetate. The organic layer was washed with aqueous sodium bicarbonate and dried (MgSO₄). The 4-chloroquinazoline was used in the next step without further purification. Sodium hydride (126 mg, 3.15 mmol, 60% in oil) was added to a suspension of 6-bromooxindole (660 mg, 3.15 mmol) in *N,N*-dimethylformamide (25 mL) and the 4-chloroquinazoline previously prepared was added dropwise and the mixture was stirred at 90 °C for 2 h. After cooling, the mixture was worked-up as described for Example 44 and the residue was purified by column chromatography eluting with methylene chloride followed by methylene chloride/methanol/sat. NH_{3(aq)}, 100:10:1. The fractions containing the expected product were combined and evaporated. The solid was dissolved in methylene chloride/methanol, 1:1, and 1 M hydrogen chloride in diethyl ether (5 mL) was added. The solid was filtered, washed with diethyl ether and dried *in vacuo* to give 225 mg (46% yield) of the title compound: EIS-MS 480 (M⁺+1).

### Example 37

### 3-[7-(2-Imidazol-1-ylethoxy)-6-methoxyquinazolin-4-yl]-2-oxo-2,3-dihydro-1H-indole-6-carbonitrile dihydrochloride

The compound was prepared as described for Example 44 using 6-methoxy-7-(2-(imidazol-1-yl)ethoxy)-4-chloroquinazoline and 6-cyanooxindole to give 160 mg of the title compound: EIS-MS 427 (M⁺+1).

### Example 38

### 2-Oxo-2.3-dihydro-1H-indole-5-carboxylic acid dimethylamide

To a solution of 2-oxo-2,3-dihydro-1*H*-indole-5-carboxylic acid (1.36 g, 7.68 mmol; described in Sun, L. J. Med. Chem., 1999, 42, 5120) in *N,N*-dimethylformimide (25 mL) was added 1,1'-carbonyldiimidazole (1.24 g, 7.68 mmol), and the mixture was heated at 70 °C under nitrogen for 15 min. The solution was allowed to cool for 25 min, and dimethylamine (40% in water, 1.1 mL, 8.44 mmol) was added. The reaction mixture was stirred at room temperature over night. The solvent was removed *in vacuo*, and the residual oil was partitioned between chloroform and an aqueous hydrochloric acid solution (2 M). The aqueous layer was extracted several times with chloroform. The combined organic layers were dried (MgSO₄), and the solvent was removed *in vacuo*. The crude product was purified by column chromatography on silica using chloroform/ethanol, 95:5, as the eluent affording 0.69 g (44% yield) of the title compound as a pale pink solid: mp 147.2-147.5 °C; MS (ESP) *m*/*z* 205 (M⁺+1).

### Example 39

### 2-Oxo-2,3-dihydro-1H-indole-5-carboxylic acid methylamide

To a solution of 2-oxo-2,3-dihydro-1*H*-indole-5-carboxylic acid (293 mg, 1.65 mmol) in *N,N*-dimethylformamide (15 mL) were added methylamine (2 M solution in tetrahydrofuran, 0.91 mL, 1.82 mmol), 1-ethyl-3-(3-dimethylaminopropyl)carbodiimide hydrochloride (698 mg, 3.64 mmol), and 4-dimethylaminopyridine (882 mg, 7.28 mmol).

The reaction mixture was stirred at room temperature over night. The solvent was removed *in vacuo*, and the residue was dissolved in 5 mL of a chloroform/methanol mixture (85:15). HCl in diethyl ether (3 M) was added until acidic pH. The solution was placed on a short column of silica (glass sinter funnel d=70 mm, using chloroform/ethanol, 90:10, as the eluent affording 195 mg (62% yield) of the pure product as a pale pink solid: mp 295 °C (decomp.); MS (EI) *m*/*z* (relative intensity) 190 (66, M⁺), 160 (100), 132 (35), 104 (15), 77 (14).

### Example 40

### 7-[3-(4-Methylpiperazin-1-yl)propoxy]-3H-quinazolin-4-one

To a solution of 3-(4-methylpiperazin-1-yl)propan-1-ol (1.19 g, 7.52 mmol; described in Brundage, Steck. J. Am. Chem. Soc., 1959, 81, 6511) in *N,N*-dimethylformamide (5 mL) was added sodium hydride (300 mg, 7.52 mmol, 60% dispersion in oil), and the mixture was stirred at room temperature for 55 min. 7-fluoro-3*H*-quinazolin-4-one (411 mg, 2.51 mmol; described in Rewcastle G. et al J. Med. Chem., 1996, 39, 4, 918-928) was added in portions, and the mixture was heated at 140 °C for 1.5 h. The mixture was allowed to cool, and the solvent was removed *in vacuo*. The residue was partitioned between ethyl acetate and water at pH 7 (adjusted by adding an aqueous hydrochloric acid solution, 2 M). The aqueous layer was extracted with another portion of ethyl acetate. To the aqueous layer was added a saturated NaHCO₃ solution followed by extraction with tetrahydrofuran. The combined organic layers were dried (Na₂SO₄), and the solvent was removed *in vacuo* affording 0.55 g of a yellowish semi-solid which was used in the next step without further purification.

### Example 41

### 4-Chloro-7-[3-(4-methylpiperazin-1-yl)propoxy]quinazoline

A mixture of crude 7-[3-(4-methylpiperazin-1-yl)propoxy]-3*H*-quinazolin-4-one from Example 40 and phosphorous oxychloride (3 mL) was heated at reflux for 30 min. *N,N*-Dimethylaniline (0.3 mL) was added whereby the solubility increased. The mixture was heated for another 30 min, and the excess of phosphorous oxychloride was removed *in vacuo*. The residue was partitioned between ethyl acetate and a saturated NaHCO₃ solution. The organic layer was dried (MgSO₄), and the solvent was removed *in vacuo*. The crude product was purified on a silica column using chloroform/methanol, 85:15, as the eluent affording 69 mg (9% yield) of the title compound as a white solid: MS (ESP) *m*/*z* 321 (M ⁺+1).

### Example 42

### 4-Chloro-8-(2-morpholin-4-ylethoxy)quinazoline

To a solution of 4-chloroquinazolin-8-ol (203 mg, 1.12 mmol; described in US 5270466), triphenylphosphine (442 mg, 1.69 mmol), and *N*-(2-hydroxyethyl)morpholine (221 mg, 1.69 mmol) in anhydrous tetrahydrofuran (5 mL) was added dropwise a solution of diethyl azodicarboxylate (294 mg, 1.69 mmol) in tetrahydrofuran (3 mL). The reaction mixture was stirred at ambient temperature for 45 min. The solvent was removed *in vacuo*, and the residue was purified by column chromatography on silica using chloroform/ethanol, 95:5, as the eluent to give 280 g (85% yield) of the title compound as a orange oil: MS (ESP) *m*/*z* 294 (M⁺+1).

### Example 43

### 3-[7-(3-Morpholin-4-yl-propoxy)quinazolin-4-yl]-2-oxo-2,3-dihydro-1H-indole-5-carboxylic acid dimethylamide

To a suspension of sodium hydride (20 mg, 0.495 mmol, 60% dispersion in oil) in *N,N*-dimethylformamide (2 mL) was added 2-oxo-2,3-dihydro-1*H*-indole-5-carboxylic acid dimethylamide (81 mg, 0.396 mmol) under argon atmosphere. The mixture was stirred for 20 min, and then a solution of 4-chloro-7-(3-morpholin-4-ylpropoxy)quinazoline (61 mg, 0.198 mmol) in *N,N*-dimethylformamide (2.2 mL) was added dropwise over 5 min. The obtained red solution was stirred at room temperature for 3 h. The solvent was removed *in vacuo*, and the mixture was partitioned between ethyl acetate and water. A few drops of an aqueous hydrochloric acid solution (2 M) was added and the pH was adjusted to 8 by the addition of an aqueous solution of saturated NaHCO₃. The product was separated in a separator funnel, and filtered and washed with water and diethyl ether. After drying *in vacuo*, 55 mg (60% yield) of the title compound was obtained as a yellow powder: mp 167.5-168.7 °C; MS (ESP) *m*/*z* 476 (M⁺+1).

### Example 44

### 3-[7-(3-Morpholin-4-yl-propoxy)-quinazolin-4-yl]-2-oxo-2,3-dihydro-1H-indole-5-carboxylic acid methylamide

To a suspension of sodium hydride (21 mg, 0.526 mmol, 60% dispersion in oil) in *N,N*-dimethylformamide (4 mL) was added 2-oxo-2,3-dihydro-1*H*-indole-5-carboxylic acid methylamide (80 mg, 0.421 mmol). 4-Chloro-7-(3-morpholin-4-ylpropoxy)quinazoline (65 mg, 0.210 mmol; described in WO 97/42187) was added in portions after 25 min. The dark red solution was stirred at room temperature over night. The solvent was removed *in vacuo*, and the residue was dissolved in a mixture of methanol, ethyl acetate, and chloroform. The HCl-salt was prepared by adding HCl in diethyl ether (3 M) followed by evaporation of about 75% of the solvent volume. Ethyl acetate was added, and the precipitated HCl-salt was filtered, washed with ethyl acetate, and dried. The HCl-salt was dissolved in an aqueous 2 M NaOH solution and extracted two times with ethyl acetate. The pH was adjusted to 8 followed by three extractions with ethyl acetate. The phases were combined and dried (Na₂SO₄). After filtration, the solvent was removed *in vacuo* affording 40 mg (41 % yield) of the title compound as a yellow solid: mp 195-198 °C; MS (ESP) *m*/*z* 462 (M⁺+1).

### Example 45

### 3-{7-[3-(4-Methylpiperazin-1-yl)propoxy]quinazolin-4-yl}-2-oxo-2,3-dihydro-1H-indole-5-carbonitrile dihydrochloride

To a suspension of sodium hydride (22 mg, 0.538 mmol, 60% dispersion in oil) in *N,N*-dimethylformamide (1.5 mL) was added 5-cyanooxindole (68 mg, 0.430 mmol). A dark yellow solution was obtained after 15 min, and a solution of 4-chloro-7-[3-(4-methylpiperazin-1-yl)propoxy]quinazoline (69 mg, 0.215 mmol) in *N,N*-dimethylformamide (1.5 m) was added dropwise. A clear red solution was initially formed and by the end of the addition a precipitate was formed. The reaction mixture was stirred at room temperature for 2 h. The solvent was removed *in vacuo*, and the residue was partitioned between ethyl acetate and an aqueous hydrochloric acid solution (2 M). The aqueous layer was washed with another portion of ethyl acetate. The pH of the aqueous layer was adjusted to 6 by adding a 2 M NaOH_{(aq)} solution. After washing with ethyl acetate, the aqueous layer was alkalized to pH 8 by addition of a saturated NaHCO_{3(aq)} solution followed by two extractions of chloroform and one with tetrahydrofuran. The chloroform- and tetrahydrofuran layers were dried (Na₂SO₄), combined, and the solvent was removed *in vacuo*. The residue was dissolved in a mixture of methanol, chloroform, and ethyl acetate. A solution of HCl in diethyl ether was added at 0°C. Half the volume was removed *in vacuo,* and ethyl acetate was added. The precipitated HCl-salt was filtered and washed with ethyl acetate. Drying *in vacuo* at 40 °C afforded 51 mg (43% yield) of the title compound as an orange solid: mp 200 °C (decomp.); MS (ESP) *m*/*z* 443 (M⁺+1).

### Example 46

### 2-Hydroxy-3-[8-(2-morpholin-4-ylethoxy)quinazolin-4-yl]-1H-indole-5-carbonitrile hydrochloride

To a solution of 5-cyanooxindole (226 mg, 1.43 mmol) in *N,N*-dimethylformamide (4 mL) was added sodium hydride (76 mg, 1.91 mmol, 60% dispersion in oil). A solution of 4-chloro-8-(2-morpholin-4-ylethoxy)quinazoline (0.28 g, 0.95 mmol) in *N,N*-dimethylformamide (3 mL) was added dropwise after 10 min. The colour changed to red, and the mixture was stirred at room temperature for 30 min. The solvent was removed *in vacuo*, and the residue was suspended in an aqueous hydrochloric acid solution (2 M) and ethyl acetate. The heterogeneous aqueous layer was washed with another portion of ethyl acetate. To the aqueous mixture was added NaHCO₃(s) until pH 8, followed by five extractions with chloroform. The combined chloroform layers were dried (Na₂SO₄), and the solvent was removed *in vacuo* affording 256 mg of an orange crude product. Part of this material (61 mg) was dissolved in a 1:1 mixture of methanol and dichloromethane, and a solution of HCl in diethyl ether (3 M) was added dropwise at 0°C until slightly acidic. About 60-75% of the solvent volume was removed *in vacuo*, and ethyl acetate was added. The precipitated HCl-salt was filtered and washed with ethyl acetate and dried *in vacuo* affording 49 mg of the title compound as a red solid: MS (ESP) m/z 416 (M ⁺+1).

### Example 47

### 3-[7-(3-Morpholin-4-yl-propoxy)quinazolin-4-yl]-6-propyl-1H-indol-2-ol hydrochloride

To a suspension of sodium hydride (30 mg, 0.756 mmol, 60% dispersion in oil) in *N,N*-dimethylformamide (2 mL) was added 6-propyl-1,3-dihydro-indol-2-one (106 mg, 0.605 mmol). The mixture was stirred at room temperature for 1 h, and a solution of 4-chloro-7-(3-morpholin-4-ylpropoxy)quinazoline (93 mg, 0.302 mmol; described in WO 97/42187) in *N,N*-dimethylformamide (1.2 mL) was added dropwise. The obtained red solution was stirred for 2 h. The solvent was removed *in vacuo*, and the residue was partitioned between an aqueous hydrochloric acid solution (2 M) and ethyl acetate. Some solid material remained. The aqueous layer together with insoluble material was washed with another portion of ethyl acetate. The aqueous mixture was alkalized to pH 8 by adding an aqueous 45% NaOH solution and extracted with ethyl acetate. The organic phase was dried (Na₂SO₄), and the solvent was removed *in vacuo.* The crude product was dissolved in ethyl acetate, isopropyl ether, and a few drops of ethanol. A solution of HCl in diethyl ether (3 M) was added at 0°C. The precipitated HCl-salt was filtered and washed with ethyl acetate affording 98 mg (62% yield) of the title compound as an orange powder: MS (ESP) *m*/*z* 447 (M⁺+1)_{.}

### Example 48

### 6-Bromo-5-(2-chloroacetyl)1,3-dihydroindol-2-one

6-Bromooxindole (500 mg, 2.4 mmol) was added to aluminum trichloride (1.08 g, 8.1 mmol) in dichloroethane (1.5 mL). Chloroacetylchloride was slowly added under gas evolution and the mixture was heated at 50 °C for 18 h. The reaction was cooled to room temperature and poured into ice water. The precipitate was collected and purified by flash silica gel chromatography using chloroform/methanol (50:1->20:1->10:1->5:1) as the eluent to give 429 mg (63% yield) the title compound as a white powder: mp 238-239 °C; ESMS *m*/*z* 289.92 (M⁺+1).

### Example 49

### 6-Bromo-2-oxo-2,3-dihydro-1H-indole-5-carboxylic acid

6-Bromo-5-(2-chloroacetyl)1,3-dihydroindol-2-one (422 mg, 1.46 mmol) in pyridine (5 mL) was heated at 70 °C for 3.5 h. The mixture was allowed to cool, the precipitate was filtered and washed with ethanol. The solid was dissolved in 2 M NaOH_{(aq)} (8 mL) and heated to 70 °C for 1 h and 20 min, cooled on an ice bath and acidified with conc. HCl_{(aq)} to pH 2. The brown precipitate was collected by filtration. This, and additional precipitate which was formed in the motherliquor over night, was dried in *vacuo* at 35 °C to give 315 mg (84% yield) of the title compound as a brown powder: mp 294 °C (decomp.); MS (TSP) *m*/*z* 257 (M⁺+1).

### Example 50

### 6-Bromo-2-oxo-2.3-dihydro-1H-indole-5-carboxylic acid methylamide

6-Bromo-2-oxo-2,3-dihydro-1*H*-indole-5-carboxylic acid (510 mg, 2.0 mmol) was dissolved in *N*,*N*-dimethylformamide (20 mL) and methylamine in tetrahydrofuran (2 M, 1095 µL), 1-(3-dimethylaminopropyl)-3-ethylcarbodiimide hydrochloride (840 mg, 4.4 mmol) and dimethylaminopyridin (1.062 g, 8.8 mmol) were added sequentially and the mixture was stirred at room temperature for 19 h. The mixture was acidified with an aqueous hydrochloric acid solution (1 M), concentrated and co-evaporated with toluene three times. The residue was dissolved in an aqueous hydrochloric acid solution (1 M), NaCl (s) was added and the solution was extracted several times with tetrahydrofuran. The organic phase was washed with an aqueous hydrochloric acid solution (1 M) and the combined aqueous phases were washed with tetrahydrofuran. The combined tetrahydrofuran phases were dried (MgSO₄), filtered and concentrated *in vacuo.* The crude product was purified by flash silica gel chromatography using ethyl acetate/methanol/water (15:1:0->10:1:0->5:1:0->2:1:0->7:2:1). The fractions containing the product were concentrated, dissolved in methanol and filtered to remove silica gel. After evaporation of the solvent, the residue was crystallized from methanol to give 137 mg (26% yield) of the title compound as a pink solid: mp 279 °C (decomp.); ESMS *m*/*z* 271.0 (M⁺+1).

### Example 51

### 6-Bromo-3-[7-(3-morpholin-4-yl-propoxy)quinazolin-4-yl]-2-oxo-2,3-dihydro-1H-indole-5-carboxylic acid methylamide

6-Bromo-2-oxo-2,3-dihydro-1*H*-indole-5-carboxylic acid methylamide (73 mg, 271 µmol) in *N*,*N*-dimethylformamide (3 mL) was added to sodium hydride (12.9 mg, 323 µmol, pre-washed with hexane). After stirring for 10 min, 4-chloro-7-(3-morpholin-4-ylpropoxy)quinazoline (39.8 mg, 123 µmol; described in WO 97/42187) was added. The reaction was stirred at room temperature for 2.5 h, concentrated and co-evaporated with toluene three times. The residue was dissolved in 10% NaOH_{(aq)} and extracted with ethyl acetate and the organic phases were washed with 10% NaOH_{(aq)}. The combined aqueous phases were acidified with 1 M HCl to pH 2 and extracted with ethyl acetate. Using a 10% aqueous solution of NaOH and NaHCO₃ (s) the pH of the aqueous phase was adjusted to 8.5 and extracted with tetrahydrofuran. The organic phase was dried (MgSO₄), filtered and concentrated *in vacuo.* The residue was dissolved in methanol and HCl in diethyl ether (1 M, 3 mL) was added. The hydrochloride salt was precipitated by the addition of diethyl ether, filtered and washed with diethyl ether. After drying *in vacuo* at 35 °C the crude product was purified by preparative HPLC (X-Terra C8 column, 19x 300 mm), using a gradient of A (water 95%, containing NH₄OAc (0.01 M), and 5% acetonitrile) and B (acetonitrile), going from 0% to 100% B over a period of 22 min. The fractions were collected and concentrated *in vacuo* to give 21 mg (30% yield) of the title compound as a yellow semi-solid: ESMS *m*/*z* 542.3 (M⁺+1).

### Example 52

### 2-(4-Ethylphenyl)-N-methoxyacetamide

(4-Ethylphenyl)acetyl chloride (2.9 g, 16 mmol; described in Shah, S. et al. J. Med. Chem., 1992, 35, 3745-3754) was added to a vigorously stirred solution of methoxyamine hydrochloride (1.5 g, 17.6 mmol) and sodium carbonate (3.4 g, 32 mmol) in a mixture of toluene (15 mL) and water (15 mL). The reaction mixture was stirred for 4 h at room temperature and then extracted with ethyl acetate (3×30 mL). The combined extracts were washed with brine (30 mL), dried (NgSO₄), and the solvent was removed *in vacuo* and dried at 25 °C *in vacuo* over night to afford 2.7 g. The residue was purified on a silica gel column using ethyl acetate as the eluent to afford 2.4 g (77% yield) of title compound as a colorless oil: MS (TSP) *m*/*z* 194 (M⁺+1).

### Example 53

### 6-Ethyl-1-methoxy-1,3-dihydroindol-2-one

To a solution of 2-(4-ethylphenyl)-*N*-methoxyacetamide (2.4 g, 12.4 mmol) in methylene chloride (35 mL), cooled on an ice-bath, was added *tert*-butyl hypochlorite (1.7 mL, 14.9 mmol). The ice-bath was removed and the solution was allowed to reach room temperature. After 1.5 h reaction time, the solvent was removed *in vacuo.* The residue was dissolved in nitromethane (3 mL) and added to nitromethane (100 mL) containing zinc acetate (11.4 g, 62 mmol) at 95 °C. After 30 min, insoluble materials were filtered off, washed with ethyl acetate, and the combined solvents were removed *in vacuo.* The residue was dissolved in ethyl acetate (200 mL) and washed with 5% aqueous solution of NaHCO₃ (150 mL), brine (50 mL), dried (MgSO₄), and concentrated to afford 2.2 g of a crude residue. The residue was purified on a silica gel column using diethyl ether as the eluent to afford 1.8 g (76% yield) of title compound as a brownish oil: MS (TSP) *m*/*z* 192 (M⁺+1).

### Example 54

### 6-Ethyl-1,3-dihydroindol-2-one

6-Ethyl-1-methoxy-1,3-dihydroindol-2-one (0.5 g, 2.6 mmol) was dissolved in methanol (15 mL), palladium (10%) on charcoal (0.3 g) was added and the mixture was hydrogenated at atmospheric pressure and room temperature. After 3 h the mixture was filtered through silica to remove the catalyst and the solvent was evaporated *in vacuo.* The residue was dried at 25 °C *in vacuo* over night to afford 0.27 g (64% yield) of title compound as a white solid: MS (TSP) *m*/*z* 162 (M⁺+1).

### Example 55

### 6-Ethyl-3-[7-(3-morpholin-4-ylpropoxy)quinazolin-4-yl]-1H-indol-2-ol hydrochloride

A mixture of sodium hydride (31 mg, 0.78 mmol, pre-washed with hexane) in *N*,*N*-dimethylformamide (1 mL) was added to 6-ethyl-1,3-dihydroindol-2-one (100 mg, 0.62 mmol) in *N,N*-dimethylformamide (2 mL). The formed yellow mixture was stirred at room temperature for 5 min and 4-chloro-7-(3-morpholin-4-yl-propoxy)quinazoline (95 mg, 0.31 mmol; described in: WO 97/42187) in *N*,*N*-dimethylformide (1 mL) was added. The obtained red solution was stirred for 1 h and the solvent was removed *in vacuo.* The residue was partitioned between an aqueous solution of hydrochloric acid (2 M) and ethyl acetate. Some solid material remained and the aqueous layer together with the insoluble material was washed with another portion of ethyl acetate. The aqueous mixture was alkalized to pH 10 by adding 2 M NaOH_{(aq)}, and extracted with ethyl acetate (3x5 mL). The combined organic layers were dried (Na₂SO₄), and the solvent was removed *in vacuo* and dried at 25 °C *in vacuo* over night to afford 80 mg. The residue was purified on a silica gel column using chloroform/ methanol, 23:2, as an eluent. Fractions containing the product were collected and evaporated *in vacuo* and dried at 25 °C *in vacuo* over night. The residue was dissolved in diethyl ether and treated with HCl in diethyl ether (5 M). The hydrochloride was dried at 25 °C *in vacuo* over night to afford 30 mg (19% yield) of title compound as an orange powder: MS (ESP) *m*/*z* 433 (M⁺+1).

### Example 56

### N-Methoxy-2-(4-propylphenyl)acetamide

The reaction was performed as described in Example 52 using (4-propylphenyl)acetic acid (described in Kindler, et al *Chem*. *Ber*., 1943, 76, 308) that was converted to the acid chloride using thionyl chloride to afford 5.8 g (58% yield) of title compound as a colorless oil: MS (TSP) *m*/*z* 208 (M⁺+1).

### Example 57

### 1-Methoxy-6-propyl-1.3-dihydroindol-2-one

The reaction was performed as described in Example 53 using *N*-methoxy-2-(4-propylphenyl)acetamide to afford 4.4 g (76% yield) of title compound as a brownish oil: MS (TSP) *m*/*z* 206 (M⁺+1).

### Example 58

### 6-Propyl-1,3-dihydroindol-2-one

1-Methoxy-6-propyl-1,3-dihydroindol-2-one (1.54 g, 7.5 mmol) was dissolved in methanol (10 mL), palladium (10%) on charcoal (0.7 g) was added and the mixture was hydrogenated at 3.5 kg pressure and at room temperature. After 20 h the mixture was filtered through silica to remove the catalyst and the solvent was evaporated in *vacuo* to afford 1.18 g (90% yield) of title compound: MS (TSP) *m*/*z* 176 (100, M⁺+1).

### Example 59

### 6-Methyl-3-[7-(3-morpholin-4-yl-propoxy)-quinazolin-4-yl]-1H-indol-2-ol

The reaction was performed as described in Example 55 using 6-methyl-1,3-dihydroindol-2-one (described in Kawase M. et al J. Org. Chem., 1989, 54, 3394-3403) and 4-chloro-7-(3-morpholin-4-yl-propoxy)quinazoline (described in: WO 97/42187). The crude product was purified by preparative HPLC (X-Terra C8 column, 19x 300 mm), using a gradient of water/acetonitrile, 70/30, to water/acetonitrile, 30/70, to afford 8 mg (6% yield) of title compound as a orange solid: MS (ESP) *m*/*z* 419 (M⁺+1).

### Example 60

### 5-Bromo-6-methyl-1,3-dihydroindol-2-one.

6-Methyl-1,3-dihydro-2*H*-indol-2-one (0.107 g, 0.73 mmol) was dissolved in acetonitrile (3 mL) and stirred for 5 min at 0 °C. *N*-Bromosuccinimide (0.129 g, 0.73 mmol) was added and the resulting reaction mixture was stirred for 4 h at 0 °C. The reaction mixture was poured onto water and the resulting crystals were collected by vacuum filtration, followed by drying over night in a vacuum desiccator over MgSO₄/CaSO₄ to afford 0.091 g (55% yield) of the title compound as a reddish-brown solid: ¹H NMR (400 MHz, DMSO-*d6*) δ 10.50 (s, 1 H), 7.41 (s, 1 H), 6.82 (s, 1 H), 3.49 (s, 2 H), 2.34 (s, 3 H).

### Example 61

### 6-Bromo-5-nitro-1,3-dihydroindol-2-one.

6-Bromo-1,3-dihydro-2*H*-indol-2-one (0.500 g, 2.36 mmol) was dissolved in conc. sulfuric acid (6 mL) and stirred for 10 min at room temperature and solid potassium nitrate (0.238 g, 2.36 mmol) was added in two portions. The resulting reaction mixture was stirred over night at room temperature. The mixture was poured onto ice-water and the reddish-brown precipitates were collected by vacuum filtration and dried over night at a vacuum desiccator over MgSO₄/CaSO₄ to afford 0.535 g (88 % yield) of the title compound: ¹H NMR (400 MHz, DMSO-*d6*) δ 11.04 (s, 1 H), 8.01 (s, 1 H), 7.20 (s, 1 H), 3.62 (s, 2 H).

### Example 62

### 5-Bromo-6-methyl-3-[7-(3-morpholin-4-ylpropoxy)quinazolin-4-yl]-1H-indol-2-ol dihydrochloride.

To a *N,N*-dimethylformamide (1.3 mL) suspension of sodium hydride (0.011 mg, 0.44 mmol, 60% in oil, pre-washed with hexane) was added 5-bromo-6-methyl-1,3-dihydroindol-2-one (0.079 g, 0.35 mmol). The formed red mixture was stirred for 10 min at room temperature and 4-chloro-7-(3-morpholin-4-ylpropoxy)quinazoline (0.055 g, 0.18 mmol; described in WO97/42187) was added. The resulting reaction mixture was stirred for 2 h and then the solvent was removed *in vacuo.* The residue was purified on a silica gel column using dichloromethane/methanol, 10:1, as an eluent to afford, after drying, 0.036 g (40% yield) of the title compound as the free base (yellow solid). This residue (0.026 g, 0.05 mmol) was dissolved in dichloromethane/methanol, 1:1, and treated with 1 M HCl in diethyl ether at 0 °C. The resulting orange solids were collected by vacuum filtration and washed with diethyl ether to afford 0.016 g of the title compound: MS (CI) *m*/*z* 498 (M⁺ +1).

### Example 63

### 6-Bromo-3-[7-(3-morpholin-4-ylpropoxy)quinazolin-4-yl]-5-nitro-1H-indol-2-ol dihydrochloride.

Sodium hydride (0.013 mg, 0.5 mmol, 60% in oil, pre-washed with hexane) was suspended in *N*,*N*-dimethylformamide (2.0 mL) and to this stirred mixture, 6-bromo-5-nitro-1,3-dihydroindol-2-one (0.103 g, 0.4 mmol) was added. The formed red mixture was stirred for 10 min at room temperature and 4-chloro-7-(3-morpholin-4-ylpropoxy)quinazoline (0.062 g, 0.2 mmol; described in WO97/42187) was added. The resulting reaction mixture was stirred for 2 h and the solvent was removed *in vacuo.* The residue was purified on a silica gel column using dichloromethane/methanol, 15:1, as eluent to afford, after drying, 0.039 g (37% yield) of the title compound as the free base (yellow solid). This residue (0.03 g, 0.057 mmol) was dissolved in dichloromethane/methanol, 1:1, and treated with 1 M HCl in diethyl ether at 0 °C. The resulting orange crystals were collected by vacuum filtration and washed with diethyl ether to afford 0.028 g (81% yield) of the title compound: MS (CI) *mlz* 529 (M⁺ +1).

### Example 64

### 7-(3-Dimethylaminopropoxy)-3H-quinazolin-4-one.

3-Dimethylaminopropan-1-ol (0.95 mL, 8.0 mmol) was dissolved in *N*,*N-*dimethylformamide (5 mL) under N₂ atmosphere and stirred for 10 min at 0 °C. To this solution, sodium hydride (60 % dispersion in oil, 0.35 g, 8.8 mmol) was added and the resulting reaction mixture was allowed to warm up to room temperature and stirred for 1 h. A solution of 7-fluoro-3*H*-quinazolin-4-one (328 mg, 2 mmol; described in Rewcastle G. et al J. Med Chem., 1996, 39, 4, 918-928) in *N*,*N*-dimethylformamide (5 mL) was added and the mixture was stirred for 2 h at 140 °C. The reaction mixture was cooled down to room temperature and diluted with aqueous saturated NaHCO₃ solution followed by extraction with ethyl acetate (3x50mL). The combined organic layer was dried over anhydrous Na₂SO₄ and concentrated in *vacuo.* The residual oil was re-concentrated with toluene and the semisolid product crystallised upon treatment with diethyl ether. The crystals were collected by vacuum filtration and dried in a desiccator to give 0.41 g (83 % yield) of the title compound as a solid: ¹H NMR (DMSO-d6, 300 MHz) δ 12.00 (br s, 1 H), 8.04 (s, 1 H), 8.01 (dd, *J*= 8 and 2 Hz, 1 H), 7.09 (d, *J*= 3 Hz, 1 H), 7.06 (s, 1 H), 4.13 (t, *J*= 6 Hz, 2 H), 2.36 (t, *J*= 7 Hz, 2 H), 2.14 (s, 6 H), 1.88 (m, 2 H).

### Example 65

### 7-(2-Dimethylaminoethoxy)-3H-quinazolin-4-one.

The compound was prepared as described in Example 64 using 2-dimethylaminoethanol (1.5 mL, 15 mmol) and 7-fluoro-3*H*-quinazolin-4-one (0.492 g, 3 mmol) affording 0.52 g (75% yield) of the title compound as a solid: MS (ES) *m*/*z* 234 (M⁺+1).

### Example 66

### 7-[2-(Isopropylmethylamino)ethoxy]-3H-quinazolin-4-one.

The compound was prepared as described in Example 64 using 2-(isopropylmethylamino)ethanol (1.168 g, 10.0 mmol) and 7-fluoro-3*H*-quinazolin-4-one (0.41 g, 2.5 mmol) to give 0.512 g (78 % yield) of the title compound as a solid. MS (ES) *m*/*z* 262 (M⁺+1).

### Example 67

### 7-(2-Diisopropylaminoethoxy)-3H-quinazolin-4-one.

The compound was prepared as described in Example 64 using 2-(diisopropylamino)ethanol (1.59 mL, 9.0 mmol) and 7-fluoro-3*H*-quinazolin-4-one (0.492 g, 3.0 mmol) to give 0.66 g (76 % yield) of the title compound as white fluffy crystals: ¹H NMR (DMSO-d6, 300 MHz) δ 12.07 (br s, 1 H), 8.04 (s, 1 H), 8.01 (dd, *J*= 6 and 4 Hz, 1 H), 7.07 (d, *J*= 4 Hz, 1 H), 7.05 (s, 1 H), 4.01 (t, *J*= 7 Hz, 2 H), 3.01 (m, 2 H), 2.80 (t, *J*=7 Hz, 2 H), 0.99 (d, *J*=7 Hz, 12 H).

### Example 68

### [3-(4-Chloroquinazolin-7-yloxy)propyl]dimethylamine.

7-(3-Dimethylaminopropoxy)-3*H*-quinazolin-4-one (0.2 g, 0.21 mmol) was added to stirred phosphorus oxychloride (5 mL). The vessel was closed and the mixture was heated to 110 °C and kept stirring for 2 h. The excess of the phosphorus oxychloride was removed *in vacuo* and the residue was dissolved in a water/chloroform mixture. The aqueous layer was alkalyzed with an aqueous saturated NaHCO₃ solution and the organic layer was separated. The water layer was extracted two times with chloroform and the combined organic layer was washed with brine, dried over anhydrous Na₂SO4 and concentrated *in vacuo*. The material was used in the next step without further purification.

### Example 69

### [2-(4-Chloroquinazolin-7-yloxy)ethyl]dimethylamine.

The compound was prepared as described in Example 68 using 7-(3-dimethylaminoethoxy)-3*H*-quinazolin-4-one (0.270g, 1.16 mmol). The material was used in the next step without further purification.

### Example 70

### [2-(4-Chloroquinazolin-7-yloxy)ethyl]isopropylmethylamine.

The compound was prepared as described in Example 68 using 7-[2-(isopropylmethylamino)ethoxy]-3*H*-quinazolin-4-one (0.300g, 1.15 mmol). The material was used in the next step without further purification.

### Example 71

### [2-(4-Chloro-quinazolin-7-yloxy)ethyl]diisopropylamine.

The compound was prepared as described in Example 68 using 7-(2-diisopropylaminoethoxy)-3H-quinazolin-4-one (0.330g, 1.14 mmol). The material was used in the next step without further purification.

### Example 72

### 3-[7-(3-Dimethylaminopropoxy)quinazolin-4-yl]-2-hydoxy-1H-indol-5-carbonitrile hydrochloride.

To an ice-bath cooled solution of 5-cyano-1,3-dihydroindol-2-one (0.255 g, 1.62 mmol) in *N*,*N*-dimethylformamide (3 mL) was added sodium hydride (60 % dispersion in oil, 0.078 g, 1.94 mmol) and the ice-bath was removed. After 15 min stirring, the ice-bath cooling was resumed and a solution of [3-(4-chloroquinazolin-7-yloxy)propyl]dimethylamine (0.265 g, 0.81 mmol) in *N,N*-dimethylformamide (6 mL) was slowly added. The reaction mixture was stirred for 12 h and quenched with aqueous saturated NaHCO₃ solution followed by extraction with ethyl acetate (3x100 mL). The combined organic extracts were dried over anhydrous Na₂SO₄ and concentrated *in vacuo* affording the free base as a yellow solid. The residue was dissolved in chloroform/ethanol, 1:1, and treated with 1 M HCl in diethyl ether at 0 °C. The resulting solid were collected by vacuum filtration and washed with diethyl ether to obtain 0.14 g (41 % yield) of the title compound as a solid: ¹H NMR (DMSO-d6, 300 MHz) δ 11.02 (br s, 1 H), 10.42 (br s, 1 H), 8.72 (br s, 1 H), 8.49 (s, 1 H), 8.15 (s, 1 H), 7.44 (dd, *J*=8 and 1 Hz, 1 H), 7.22 (d, *J*=9 Hz, 1 H), 7.11 (s, 1 H), 7.01 (d, *J*=8 Hz, 1 H), 4.29 (t, *J*=6 Hz, 2 H), 3.25 (m, 2 H), 2.80 (s, 6 H), 2.24 (m, 2 H).

### Example 73

### 3-[7-(2-Dimethylaminoethoxy)quinazolin-4-yl]-2-hydoxy-1H-indol-5-carbonitrile fumarate.

The compound was prepared as described in Example 72 using [2-(4-chloroquinazolin-7-yloxy)-ethyl]dimethylamine (1.15 mmol) and fumaric acid to give 0.22 g (40% yield) of the title compound as a solid. ¹H NMR (DMSO-d6, 300 MHz) δ 10.85 (br s, 1 H), 8.81 (br s, 1 H), 8.47 (s, 1 H), 8.20 (b s, 1 H), 7.38 (d, *J*=8 Hz, 1 H), 7.18 (d, *J*=6 Hz, 1 H), 7.07 (s, 1 H), 6.97 (d, *J*=8 Hz, 1 H), 6.58 (s, 1 H), 4.30 (t, J=5 Hz, 2 H), 2.87 (t, *J*=5 Hz, 2 H), 2.37 (s, 6 H).

### Example 74

### 3-[7-[2-(Isopropylmethylamino)ethoxylquinazolin-4-yl]-2-oxo-2,3-dihydro-1H-indol-5-carbonitrile fumarate.

The compound was prepared as described in Example 72 using [2-(4-chloroquinazolin-7-yloxy)ethyl]-isopropylmethylamine (1.15 mmol) and fumaric acid to give 0.185 g (32% yield) of the title compound as a solid: ¹H NMR (DMSO-d6, 300 MHz) δ 10.84 (br s, 1 H), 8.80 (br s, 1 H), 8.46 (s, 1 H), 8.19 (br s, 1 H), 7.37 (d, *J*=8 Hz, 1 H), 7.17 (d, *J*=9 Hz, 1 H), 7.06 (s, 1 H), 6.95 (d, *J*=8 Hz, 1 H), 6.56 (s, 1 H), 4.29 (t, *J*=5 Hz, 2 H), 3.08 (t, *J*=7 Hz, 1 H), 3.00 (t, *J*=6 Hz, 2 H), 2.40 (s, 3 H). 1.06 (d, *J*=6 Hz, 6 H).

### Example 75

### 3-[7-(2-Diisopronylamino)ethoxy)quinazolin-4-yl]-2-hydroxy-1H-indol-5-carbonitrile fumarate.

The compound was prepared as described in Example 72 using [2-(4-chloroquinazolin-7-yloxy)-ethyl]diisopropylamine (1.14 mmol) and fumaric acid to give 0.20 g (35% yield) of the title compound as a solid: ¹H NMR (DMSO-d6, 300 MHz) δ 11.00 (b s, 1 H), 8.84 (br s, 1 H), 8.57 (s, 1 H), 8.29 (br s, 1 H), 7.50 (d, *J*=8 Hz, 1 H), 7.29 (d, *J*=9 Hz, 1 H), 7.15 (s, 1 H), 7.08 (d, *J*=8 Hz, 1 H), 6.70 (s, 1 H), 4.26 (br s, 2 H), 3.29 (t, *J*=6 Hz, 2 H), 3.10 (br s, 2 H), 1.19 (d, *J*=6 Hz, 6 H), 1.16 (d, *J*=7 Hz, 6 H).

### Pharmaceutical composition

The composition may be in a form suitable for oral administration, for example as a tablet, pill, syrup, powder, granule or capsule, for parenteral injection (including intravenous, subcutaneous, intramuscular, intravascular or infusion) as a sterile solution, suspension or emulsion, for topical administration e.g. as an ointment, patch or cream or for rectal administration e.g. as a suppository.

In general the above compositions may be prepared in a conventional manner using pharmaceutically acceptable carriers or diluents.
Suitable daily doses of the compounds of formula **I** in the treatment of a mammal, including man, are approximately 0.01 to 250 mg/kg bodyweight at peroral administration and about 0.001 to 250 mg/kg bodyweight at parenteral administration. The typical daily dose of the active ingredients varies within a wide range and will depend on various factors such as the relevant indication, severity of the illness treated, the specific compound used, the route of administration, the age, weight and sex of the patient and may be determined by a physician.

Illustrate representative pharmaceutical dosage forms containing a compound of formula I, as a free base or a pharmaceutically acceptable salt thereof, are described in WO 97/42187.

### Medical use

Surprisingly, it has been found that the compounds defined in the present invention, as hereinbefore defined, are useful in therapy. The compounds of the present invention are well suited for inhibiting glycogen synthase kinase-3 (GSK3). Accordingly, the compounds of the present invention are expected to be useful in the prevention and/or treatment of conditions associated with glycogen synthase kinase-3 activity, i.e. the compounds may be used to produce an inhibitory effect of GSK3 in mammals, including man, in need of such prevention and/or treatment.
GSK3 is highly expressed in the central and peripheral nervous system and in other tissues. Thus, it is expected that compounds of the invention are well suited for the prevention and/or treatment of conditions associated with glycogen synthase kinase-3 in the central and peripheral nervous system. In particular, the compounds of the invention are expected to be suitable suitable in the manufacture of a medicament for the prevention and/or treatment of dementia related diseases and Alzheimer's Disease.
The dementia related diseases are selected from the group consisting of Frontotemporal dementia Parkinson's Type, Parkinson dementia complex of Guam, HIV dementia, predemented states, vascular dementia, dementia with Lewy bodies, Frontotemporal dementia and dementia pugilistica.

The compounds of the invention are also expected to be suitable in the manufacture of a medicament for the prevention and/or treatment of amyotrophic lateral sclerosis, corticobasal degeneration, Down syndrome, Huntington's Disease, Parkinson's Disease, postencephelatic parkinsonism, progressive supranuclear palsy, Pick's Disease, Niemann-Pick's Disease, stroke, head trauma and other chronic neurodegenerative diseases, Bipolar Disease, affective disorders, depression, schizophrenia, cognitive disorders and hair loss.

The compounds of the invention are further expected to be suitable in the manufacture of a medicament for the prevention and/or treatment of Mild Cognitive Impairment, Age-Associated Memory Impairment, Age-Related Cognitive Decline, Cognitive Impairement No Dementia, mild cognitive decline, mild neurocognitive decline, Late-Life Forgetfulness, memory impairment and cognitive impairment and androgenetic alopecia.

The present invention relates also to the use of a compound of the present invention as defined hereinbefore, in the manufacture of a medicament for the prevention and/or treatment of conditions as hereinbefore defined.

In the context of the present specification, the term "therapy" includes treatment as well as prevention, unless there are specific indications to the contrary. The terms "therapeutic" and "therapeutically" should be construed accordingly.

### Non- Medical use

In addition to their use in therapeutic medicine, the compounds of the present invention as a free base or salts thereof, are also useful as pharmacological tools in the development and standardisation of *in vitro* and *in vivo* test systems for the evaluation of the effects of inhibitors of GSK3 related activity in laboratory animals such as cats, dogs, rabbits, monkeys, rats and mice, as part of the search for new therapeutics agents.

### Pharmacology

### Determination of ATP competition in Scintillation Proximity GSK3βAssay.

### GSK3β scintillation Proximity assay.

The competition experiments were carried out in duplicate with 10 different concentrations of the inhibitors in clear-bottom microtiter plates (Wallac, Finland). A biotinylated peptide substrate, Biotin-Ala-Ala-Glu-Glu-Leu-Asp-Ser-Arg-Ala-Gly-Ser(PO₃H₂)-Pro-Gln-Leu (AstraZeneca, Lund), was added at a final concentration of 1 µM in an assay buffer containing 1 mU recombinant human GSK3β (Dundee University, UK), 12 mM morpholinepropanesulfonic acid (MOPS), pH 7.0,0.3 mM EDTA, 0.01% β-mercaptorethanol, 0.004 % Brij 35 (a natural detergent), 0.5 % glycerol and 0:5 µg BSA/25 µl. The reaction was initiated by the addition of 0.04 µCi [γ-³³P]ATP (Amersham, UK) and unlabelled ATP at a final concentration of 1 µM and assay volume of 25 µl. After incubation for 20 min at room temperature, each reaction was terminated by the addition of 25 µl stop solution containing 5 mM EDTA, 50 µM ATP, 0.1 % Triton X-100 and 0.25 mg streptavidin coated Scintillation Proximity Assay (SPA) beads (Amersham, UK). After 6 hours the radioactivity was determined in a liquid scintillation counter (1450 MicroBeta Trilux, Wallac). The inhibition curves were analysed by non-linear regression using GraphPad Prism, USA. The Kₘ value of ATP for GSK3β, used to calculate the inhibition constants (Kᵢ) of the various compounds, was 20 µM.

The following abbreviations have been used:
- ATP: Adenosine Triphophatase
- BSA: Bovin Serum Albumin
- EDTA: Ethylenediaminetetraacetic acid
- GSK3: Glycogen synthase kinase 3
- MOPS: Morpholinepropanesulfonic acid
- SPA: Scintillation Proximity Assay

### Results

Typical Kᵢ values for the compounds of the present invention are in the range of about 0.001 to about 10,000 nM. Other values for Kᵢ are in the range of about 0.001 to about 1000 nM. Further values for Kᵢ are in the range of about 0.001 nM to about 300 nM.

## Claims

1. Use of a compound of formula **I** 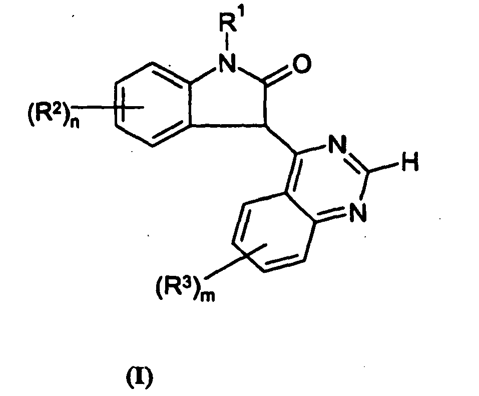 wherein:
R¹ is hydrogen or C₁₋₃alkyl;
R² is hydroxy, halogeno, trifluoromethyl, cyano, amino, nitro, carboxy, C₁₋₃alkyl, C₁₋₃alkoxy, C₁₋₃akanoyloxy, C₂₋₄alkanoyl, C₁₋₄alkanoylamino, C₁₋₄alkoxycarbonyl, C₁₋₄alkylthio, C₁₋₄alkylsulphinyl, C₁₋₄alkylsulphonyl, carbamoyl, *N*-C₁₋₄alkylcarbamoyl, *N*,*N*-di(C₁₋₄alkyl)carbamoyl, aminosulphonyl, *N*-C₁₋₄alkylaminosulphonyl, *N*,*N-*di(C₁₋₄alkyl)aminosulphonyl, C₁₋₄alkylsulphonylamino, or a group R⁴X¹,
wherein X¹ is a direct bond, C₂₋₄alkanoyl, CONR⁵R⁶, SO₂NR⁷R⁸ or SO₂R⁹ (wherein R⁵ and R⁷ each independently are hydrogen or C₁₋₂alkyl, and R⁶, R⁸ and R⁹ each independently are C₁₋₄alkyl, and wherein R⁴ is linked to R⁶, R⁸ or R⁹); and
R⁴ is phenyl or a 5 or 6 membered heterocyclic group with one or two heteroatoms, selected independently from O, S and N, which heterocyclic group may be saturated or unsaturated and which phenyl or heterocyclic group may be substituted with one or two substituents selected independently from hydroxy, halogeno, C₁₋₃alkyl,
C₁₋₃alkoxy, C₁₋₃alkanoyloxy, trifluoromethyl, cyano, amino, nitro and
C₁₋₄alkoxycarbonyl;
R³ is hydroxy, halogeno, nitro, trifluoromethyl, C₁₋₃alkyl, cyano, amino or R¹⁰X²,
wherein X² is O, CH₂, S, SO, SO₂, NR¹¹CO, CONR¹², SO₂NR¹³, NR¹⁴SO₂ or NR¹⁵ (wherein R¹¹, R¹², R¹³, R¹⁴ and R¹⁵ each independently are hydrogen, C₁₋₃alkyl or
C₁₋₃alkoxyC₂₋₃alkyl), or X² is a direct bond; and
R¹⁰ is selected from one of the following groups:
1) hydrogen or C₁₋₅alkyl which may be substituted with one or more groups selected independently from hydroxy, fluoro and amino;
2) C₁₋₅alkylX³COR¹⁶ (wherein X³ is O or NR¹⁷ (wherein R¹⁷ is hydrogen, C₁₋₃alkyl or C₁₋₃alkoxyC₂₋₃alkyl) and R¹⁶ is C₁₋₃alkyl, NR¹⁸R¹⁹ or OR²⁰ (wherein R¹⁸, R¹⁹ and R²⁰ each independently are hydrogen, C₁₋₃alkyl or C₁₋₃alkoxyC₂₋₃alkyl);
3) C₁₋₅alkylX⁴R²¹ (wherein X⁴ is O, S, SO, SO₂, OCO, NR²²CO, CONR²³, SO₂NR²⁴, NR²⁵O₂ or NR²⁶ (wherein R²², R²³, R²⁴, R²⁵ and R²⁶ each independently are hydrogen, C₁₋₃alkyl or C₁₋₃alkoxyC₂₋₃alkyl) and R²¹ is hydrogen, C₁₋₃alkyl, cyclopentyl, cyclohexyl or a 5 or 6 membered saturated heterocyclic group with one or two heteroatoms selected independently from O, S and N, which C₁₋₃alkyl group may be substituted with one or two substituents selected independently from oxo, hydroxy, halogeno and C₁₋₄alkoxy and which heterocyclic group may be substituted with one or two substituents selected independently from oxo, hydroxy, halogeno, C₁₋₄alkyl, C₁₋₄hydroxyalkyl and C₁₋₄alkoxy);
4) C₁₋₅alkylX⁵C₁₋₅alkylX⁶R²⁷ (wherein X⁵ and X⁶ each independently are O, S, SO, SO₂, NR²⁸CO, CONR²⁹, SO₂NR³⁰, NR³¹SO₂ or NR³² (wherein R²⁸, R²⁹, R³⁰, R³¹ and R³² each independently are hydrogen, C₁₋₃alkyl or C₁₋₃alkoxyC₂₋₃alkyl) and R²⁷ is hydrogen or C₁₋₃alkyl);
5) C₁₋₅alkylR³³ (wherein R³³ is a 5 or 6 membered saturated heterocyclic group with one or two heteroatoms selected independently from O, S and N, which heterocyclic group may be substituted with one or two substituents selected independently from oxo, hydroxy, halogeno, C₁₋₄alkyl, C₁₋₆carbonyl, C₁₋₄hydroxyalkyl and C₁₋₄alkoxy);
6) C₂₋₅alkenylR³³ (wherein R³³ is as defined hereinbefore);
7) C₂₋₅alkynylR³³ (wherein R³³ is as defined hereinbefore);
8) R³⁴ (wherein R³⁴ is a pyridone group, a phenyl group or a 5 or 6 membered aromatic heterocyclic group with 1 to 3 heteroatoms selected independently from O, N and S, which pyridone, phenyl or heterocyclic group may carry up to 5 substituents selected independently from hydroxy, halogeno, amino, C₁₋₄alkyl, C₁₋₄alkoxy, C₁₋₄hydroxyalkyl, C₁₋₄aminoalkyl, C₁₋₄alkylamino, C₁₋₄hydroxyalkoxy, carboxy, cyano, CONR³⁵R³⁶ and NR³⁷COR³⁸ (wherein R³⁵, R³⁶, R³⁷ and R³⁸ each independently are hydrogen, C₁₋₄alkyl or C₁₋₃alkoxyC₂₋₃alkyl));
9) C₁₋₅alkylR³⁴ (wherein R³⁴ is as defined hereinbefore);
10) C₂₋₅alkenylR³⁴ (wherein R³⁴ is as defined hereinbefore);
11) C₂₋₅alkynylR³⁴ (wherein R³⁴ is as defined hereinbefore);
12) C₁₋₅alkylX⁷R³⁴ (wherein X⁷ is O, S, SO, SO₂, NR³⁹CO, CONR⁴⁰, SO₂NR⁴¹, NR⁴²SO₂ or NR⁴³ (wherein R³⁹, R⁴⁰, R⁴¹, R⁴² and R⁴³ each independently are hydrogen, C₁₋₃alkyl or C₁₋₃alkoxyC₂₋₃alkyl) and R³⁴ is as defined hereinbefore);
13) C₂₋₅alkenylX⁸R³⁴ (wherein X⁸ is O, S, SO, SO₂, NR⁴⁴CO, CONR⁴⁵, SO₂NR⁴⁶, NR⁴⁷SO₂ or NR⁴⁸ (wherein R⁴⁴, R⁴⁵, R⁴⁶, R⁴⁷ and R⁴⁸ each independently are hydrogen, C₁₋₃alkyl or C₁₋₃alkoxyC₂₋₃alkyl) and R³⁴ is as defined hereinbefore);
14) C₂₋₅alkynylX⁹R³⁴ (wherein X⁹ is O, S, SO, SO₂, NR⁴⁹CO, CONR⁵⁰, SO₂NR⁵¹, NR⁵²SO₂ or NR⁵³ (wherein R⁴⁹, R⁵⁰, R⁵¹, R⁵² and R⁵³ each independently are hydrogen, C₁₋₃alkyl or C₁₋₃alkoxyC₂₋₃alkyl) and R³⁴ is as defined hereinbefore);
15) C₁₋₃alkylX¹⁰C₁₋₃alkylR³⁴ (wherein X¹⁰ is O, S, SO, SO₂, NR⁵⁴CO, ONR⁵⁵, SO₂NR⁵⁶, NR⁵⁷SO₂ or NR⁵⁸ (wherein R⁵⁴, R⁵⁵, R⁵⁶, R⁵⁷ and R⁵⁸ each independently are hydrogen, C₁₋₃alkyl or C₁₋₃alkoxyC₂₋₃alkyl) and R³⁴ is as defined hereinbefore);
16) R³³ (wherein R³³ is as defined hereinbefore); and
17) C₁₋₃alkylX¹⁰C₁₋₃alkylR³³ (wherein X¹⁰ and R³³ are as defined hereinbefore);
18) C₁₋₅alkylCOR³³ (wherein R³³ is as defined hereinbefore);
n is 0, 1, 2, 3 or 4;
m is 0, 1, 2, 3 or 4;
as a free base or a pharmaceutically acceptable salt thereof, in the manufacturing of a medicament for the prevention and/or treatment of Alzheimer's disease.

2. Use of a compound of formula **I** as defined in claim 1, in the manufacture of a medicament for the prevention and/or treatment of dementia related diseases selected from Frontotemporal dementia Parkinson's Type, Parkinson dementia complex of Guam, HIV dementia, predemented states, vascular dementia, dementia with Lewy bodies, Frontotemporal dementia and dementia pugilistica.

3. Use of a compound of formula **I** as defined in claim 1, in the manufacture of a medicament for the prevention and/or treatment of amyotrophic lateral sclerosis, corticobasal degeneration, Down syndrome, Huntington's Disease, Parkinson's Disease, postencephelatic parkinsonism, progressive supranuclear palsy, Pick's Disease, Niemann-Pick's Disease, stroke, head trauma and other chronic neurodegenerative diseases, Bipolar Disease, affective disorders, depression, schizophrenia, cognitive disorders, and hair loss.

4. Use of a compound of formula **I** as defined in claim 1, in the manufacture of a medicament for the prevention and/or treatment of Mild Cognitive Impairment, Age-Associated Memory Impairment, Age-Related Cognitive Decline, Cognitive Impairement No Dementia, mild cognitive decline, mild neurocognitive decline, Late-Life Forgetfulness, memory impairment and cognitive impairment and androgenetic alopecia.

5. The use of a compound according to any of claims 1 to 4, wherein R³ is R¹⁰X²,
wherein X² is O, CH₂, S, SO, SO₂, NR¹¹CO, CONR¹², SO₂NR¹³, NR¹⁴SO₂ or NR¹⁵ (wherein R¹¹, R¹², R¹³, R¹⁴ and R¹⁵ each independently are hydrogen, C₁₋₃alkyl or C₁₋₃alkoxyC₂₋₃alkyl), or X² is a direct bond; and
R¹⁰ is selected from one of the following groups:
1) hydrogen or C₁₋₅alkyl which may be substituted with one or more groups selected independently from hydroxy, fluoro and amino;
2) C₁₋₅alkylX³COR¹⁶ (wherein X³ is O or NR¹⁷ (wherein R¹⁷ is hydrogen, C₁₋₃alkyl or C₁₋₃alkoxyC₂₋₃alkyl) and R¹⁶ is C₁₋₃alkyl, NR¹⁸R¹⁹ or OR²⁰ (wherein R¹⁸, R¹⁹ and R²⁰ each independently are hydrogen, C₁₋₃alkyl or C₁₋₃alkoxyC₂₋₃alkyl));
3) C₁₋₅alkylX⁴R²¹ (wherein X⁴ is O, S, SO, SO₂, OCO, NR²²CO, CONR²³, SO₂NR¹⁴, NR²⁵SO₂ or NR²⁶ (wherein R²², R²³, R²⁴, R²⁵ and R²⁶ each independently are hydrogen, C₁₋₃alkyl or C₁₋₃alkoxyC₂₋₃alkyl) and R²¹ is hydrogen, C₁₋₃alkyl, cyclopentyl, cyclohexyl or a 5 or 6 membered saturated heterocyclic group with one or two heteroatoms selected independently from O, S and N, which C₁₋₃alkyl group may be substituted with one or two substituents selected independently from oxo, hydroxy, halogeno and C₁₋₄alkoxy and which heterocyclic group may be substituted with one or two substituents selected independently from oxo, hydroxy, halogeno, C₁₋₄alkyl, C₁₋₄hydroxyalkyl and C₁₋₄alkoxy);
4) C₁₋₅alkylX⁵C₁₋₅alkylX⁶R²⁷ (wherein X⁵ and X⁶ each independently are O, S, SO, SO₂, NR²⁸CO, CONR²⁹, SO₂NR³⁰, NR³¹SO₂ or NR³² (wherein R²⁸, R²⁹, R³⁰, R³¹ and R³² each independently are hydrogen, C₁₋₃alkyl or C₁₋₃alkoxyC₂₋₃alkyl) and R²⁷ is hydrogen or C₁₋₃alkyl);
5) C₁₋₅alkylR³³ (wherein R³³ is a 5 or 6 membered saturated heterocyclic group with one or two heteroatoms selected independently from O, S and N, which heterocyclic group may be substituted with one or two substituents selected independently from oxo, hydroxy, halogeno, C₁₋₄alkyl, C₁₋₄hydroxyalkyl and C₁₋₄alkoxy);
6) C₂₋₅alkenylR³³ (wherein R³³ is as defined hereinbefore);
7) C₂₋₅alkynylR³³ (wherein R³³ is as defined hereinbefore);
8) R³⁴ (wherein R³⁴ is a pyridone group, a phenyl group or a 5 or 6 membered aromatic heterocyclic group with 1 to 3 heteroatoms selected independently from O, N and S, which pyridone, phenyl or heterocyclic group may carry up to 5 substituents selected independently from hydroxy, halogeno, amino, C₁₋₄alkyl, C₁₋₄alkoxy, C₁₋₄hydroxyalkyl, C₁₋₄aminoalkyl, C₁₋₄alkylamino, C₁₋₄hydroxyalkoxy, carboxy, cyano, CONR³⁵R³⁶ and NR³⁷COR³⁸ (wherein R³⁵, R³⁶, R³⁷ and R³⁸ each independently are hydrogen, C₁₋₄alkyl or C₁₋₃alkoxyC₂₋₃alkyl));
9) C₁₋₅alkylR³⁴ (wherein R³⁴ is as defined hereinbefore);
10) C₂₋₅alkenylR³⁴ (wherein R³⁴ is as defined hereinbefore);
11) C₂₋₅alkynylR³⁴ (wherein R³⁴ is as defined hereinbefore);
12) C₁₋₅alkylX⁷R³⁴ (wherein X⁷ is O, S, SO, SO₂, NR³⁹CO, CONR⁴⁰, SO₂NR^{4t}, NR⁴²SO₂ or NR⁴³ (wherein R³⁹, R⁴⁰, R⁴¹, R⁴² and R⁴³ each independently are hydrogen, C₁₋₃alkyl or C₁₋₃alkoxyC₂₋₃alkyl) and R³⁴ is as defined hereinbefore);
13) C₂₋₅alkenylX⁸R³⁴ (wherein X⁸ is O, S, SO, SO₂, NR⁴⁴CO, CONR⁴⁵, SO₂NR⁴⁶, NR⁴⁷SO₂ or NR⁴⁸ (wherein R⁴⁴, R⁴⁵, R⁴⁶, R⁴⁷ and R⁴⁸ each independently are hydrogen, C₁₋₃akyl or C₁₋₃alkoxyC₂₋₃alkyl) and R³⁴ is as defined hereinbefore);
14) C₂₋₅alkynylX⁹R³⁴ (wherein X⁹ is O, S, SO, SO₂, NR⁴⁹CO, CONR⁵⁰, SO₂NR⁵¹, NR¹²SO₂ or NR⁵³ (wherein R⁴⁹, R⁵⁰, R⁵¹, R⁵² and R⁵³ each independently are hydrogen, C₁₋₃alkyl or C₁₋₃alkoxyC₂₋₃alkyl) and R³⁴ is as defined hereinbefore);
15) C₁₋₃alkylX¹⁰C₁₋₃alkylR³⁴ (wherein X¹⁰ is O, S, SO, SO₂, NR⁵⁴CO, ONR⁵⁵, SO₂NR⁵⁶, NR⁵⁷SO₂ or NR⁵⁸ (wherein R⁵⁴, R⁵⁵, R⁵⁶, R¹⁷ and R⁵⁸ each independently are hydrogen, C₁₋₃alkyl or C₁₋₃alkoxyC₂₋₃alkyl) and R³⁴ is as defined hereinbefore);
16) R³³ (wherein R³³ is as defined hereinbefore); and
17) C₁₋₃alkylX¹⁰C₁₋₃alkylR³³ (wherein X¹⁰ and R³³ are as defined hereinbefore).

6. The use of a compound according to any one of claims 1 to 5, wherein R¹ is hydrogen.

7. The use of a compound according to any one of claims 1 to 6, wherein R² is halogeno, cyano, nitro, carboxy, C₁₋₄alkoxycarbonyl, trifluoromethyl, C₁₋₃alkyl, C₁₋₃alkoxy, *N*-C₁₋₄alkylcarbamoyl, *N,N*-di(C₁₋₄alkyl)carbamoyl, aminosulphonyl, or a group R⁴X¹,
wherein X¹ is CONR⁵R⁶, (wherein R⁵ is hydrogen or C₁₋₂alkyl, and R⁶ is C₁₋₄alkyl, and
wherein R⁴ is linked to R⁶); and
R⁴ is phenyl or a 5 or 6 membered heterocyclic group with one or two heteroatoms, selected independently from O and N, which heterocyclic group may be saturated or unsaturated and which phenyl or heterocyclic group may be substituted with one or two substituents selected independently from hydroxy, halogeno, C₁₋₃alkyl, C₁₋₃alkoxy, C₁₋₃alkanoyloxy, trifluoromethyl, cyano, amino, nitro and C₁₋₄alkoxycarbonyl;
n is 0, 1 or2.

8. The use of a compound according to any one of claims 1 to 7, wherein R³ is R¹⁰X²,
wherein X² is O; and
R¹⁰ is selected from one of the following groups:
3) C₁₋₅alkylX⁴R²¹ (wherein X⁴ is O or NR²⁶ (wherein R²¹ and R²⁶ each independently are hydrogen, C₁₋₃alkyl, cyclopentyl or cyclohexyl));
4) C₁₋₅alkylX⁵C₁₋₅alkylX⁶R²⁷ (wherein X⁵ and X⁶ are O and R²⁷ is hydrogen or C₁₋₃alkyl);
5) C₁₋₅alkylR³³ (wherein R³³ is a 6 membered saturated heterocyclic group with one or two heteroatoms, selected independently from O and N, which heterocyclic group may be substituted with one or two substituents selected independently from oxo, hydroxy, halogeno, C₁₋₄alkyl, C₁₋₄hydroxyalkyl and C₁₋₄alkoxy);
9) C₁₋₅alkylR³⁴ (wherein R³⁴ is a 5 membered aromatic heterocyclic group with 1 to 3 heteroatoms selected independently from O and N, which heterocyclic group may carry up to 5 substituents selected independently from halogeno, amino, C₁₋₄alkyl, C₁₋₄alkoxy, C₁₋₄hydroxyalkyl, C₁₋₄aminoalkyl, C₁₋₄alkylamino, C₁₋₄hydroxyalkoxy, carboxy, hydoxy, cyano, CONR³⁵R³⁶ and NR³⁷COR³⁸ (wherein R³⁵, R³⁶, R³⁷ and R³⁸ each independently are hydrogen, C₁₋₄alkyl or C₁₋₃alkoxyC₂₋₃alkyl)); and
17) C₁₋₃alkylX¹⁰C₁₋₃alkylR³³, (wherein X¹⁰ is O and R³³ are as defined hereinbefore);
m is 0, 1 or 2.

9. The use of a compound according to any one of claims 1 to 4, 6 and 7, wherein R³ is R¹⁰X²,
wherein X² is O; and R¹⁰ is selected from one of the following groups:
1) hydrogen or C₁₋₅alkyl;
5) C₁₋₅alkylR³³ (wherein R³³ is a 5 or 6 membered saturated heterocyclic group with one or two heteroatoms, selected independently from O, S and N, which heterocyclic group may be substituted with one or two substituents selected independently from oxo, hydroxy, halogeno, C₁₋₄alkyl, C₁₋₆carbonyl, C₁₋₄hydroxyalkyl and C₁₋₄alkoxy);
18) C₁₋₅alkylCOR³³ (wherein R³³ is as defined hereinbefore).

10. The use of a compound according to any of claims 1 to 8, wherein R³ is R¹⁰X²,
wherein X² is O; and R¹⁰ is
4) C₁₋₅alkylX⁵C₁₋₅alkylX⁶R²⁷ (wherein X⁵ and X⁶ are O and R²⁷ is hydrogen or C₁₋₃alkyl).

11. Use of a compound according to any one of claims 1 to 10, wherein R² is substituted on position 5 and/or 6 and R³ is substituted on position 6, 7 and/or 8.

12. A compound which is
3-[7-(3-Dimethylaminopropoxy)quinazolin-4-yl]-2-hydoxy-1H-indol-5-carbonitrile hydrochloride,
3-[7-(2-Dimethylaminoethoxy)quinazolin-4-yl]-2-hydoxy-1*H*-indol-5-carbonitrile fumarate,
3-{7-[2-(Isopropylmethylamino)ethoxy]quinazolin-4-yl]-2-oxo-2,3-dihydro-1*H*-indol-5-carbonitrile fumarate and
3-[7-(2-Diisopropylamino)ethoxy)quinazolin-4-yl]-2-hydroxy-1*H*-indol-5-carbonitrile fumarate,
as a free base or salts thereof.

13. Compounds according to claim 12 for use in therapy.

14. A use of a compound according to claim 12 in the manufacturing of a medicament for the prevention and/or treatment of Alzheimer's Disease.

15. A use of a compound according to claim 12 in the manufacturing of a medicament for the prevention and/or treatment of a dementia related diseases selected from the group consisting of Frontotemporal dementia Parkinson's Type, Parkinson dementia complex of Guam, HIV dementia, predemented states, vascular dementia, dementia with Lewy bodies, Frontotemporal dementia and dementia pugilistica.

16. A use of a compound according to claim 12 in the manufacturing of a medicament for the prevention and/or treatment of amyotrophic lateral sclerosis, corticobasal degeneration, Down syndrome, Huntington's Disease, Parkinson's Disease, postencephelatic parkinsonism, progressive supranuclear palsy, Pick's Disease, Niemann-Pick's Disease, stroke, head trauma and other chronic neurodegenerative diseases, Bipolar Disease, affective disorders, depression, schizophrenia, cognitive disorders, and hair loss.

17. A use of a compound according to claim 12 in the manufacturing of a medicament for the prevention and/or treatment of Mild Cognitive Impairment, Age-Associated Memory Impairment, Age-Related Cognitive Decline, Cognitive Impairement No Dementia, mild cognitive decline, mild neurocognitive decline, Late-Life Forgetfulness, memory impairment and cognitive impairment and androgenetic alopecia.

## Patentansprüche

1. Verwendung einer Verbindung der Formel I 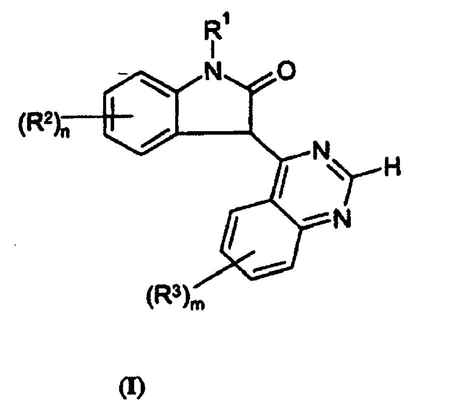 in welcher:
R¹ für Wasserstoff oder C₁₋₃-Alkyl steht;
R² für Hydroxy, Halogen, Trifluormethyl, Cyano, Amino, Nitro, Carboxy, C₁₋₃-Alkyl, C₁₋₃-Alkoxy, C₁₋₃-Alkanoyloxy, C₂₋₄-Alkanoyl, C₁₋₄-Alkanoylamino, C₁₋₄-Alkoxycarbonyl, C₁₋₄-Alkylthio, C₁₋₄-Alkylsulfinyl, C₁₋₄-Alkylsulfonyl, Carbamoyl, N-C₁₋₄-Alkylcarbamoyl, *N*,*N*-Di(C₁₋₄-alkyl)carbamoyl, Aminosulfonyl, *N*-C₁₋₄-Alkylaminosulfonyl, *N,N*-Di(C₁₋₄-alkyl)aminosulfonyl, C₁₋₄-Alkylsulfonylamino oder eine Gruppe R⁴X¹ steht,
wobei X¹ für eine direkte Bindung, C₂₋₄-Alkanoyl, CONR⁵R⁶, SO₂NR⁷R⁸ oder SO₂R⁹ steht (wobei R⁵ und R⁷ jeweils unabhängig voneinander für Wasserstoff oder C₁₋₂-Alkyl stehen und R⁶, R⁸ und R⁹ jeweils unabhängig voneinander für C₁₋₄-Alkyl stehen und
wobei R⁴ an R⁶, R⁸ oder R⁹ gebunden ist); und
R⁴ für eine Phenylgruppe oder eine 5- oder 6-gliedrige heterocyclische Gruppe mit einem oder zwei Heteroatomen unabhängig voneinander ausgewählt aus O, S und N steht, wobei diese heterocyclische Gruppe gesättigt oder ungesättigt sein kann und wobei diese Phenylgruppe bzw. heterocyclische Gruppe durch einen oder zwei Substituenten unabhängig voneinander ausgewählt aus Hydroxy, Halogen, C₁₋₃-Alkyl, C₁₋₃-Alkoxy, C₁₋₃-Alkanoyloxy, Trifluoromethyl, Cyano, Amino, Nitro und C₁₋₄-Alkoxycarbonyl substituiert sein kann;
R³ für Hydroxy, Halogen, Nitro, Trifluormethyl, C₁₋₃-Alkyl, Cyano, Amino oder R¹⁰X² steht, wobei X² für O, CH₂, S, SO, SO₂, NR¹¹CO, CONR¹², SO₂NR¹³, NR¹⁴SO₂ oder NR¹⁵ steht (wobei R¹¹, R¹², R¹³, R¹⁴ und R¹⁵ jeweils unabhängig voneinander für Wasserstoff, C₁₋₃-Alkyl oder C₁₋₃-Alkoxy-C₂₋₃-alkyl stehen) oder X² für eine direkte Bindung steht; und
R¹⁰ aus einer der folgenden Gruppen ausgewählt ist:
1) Wasserstoff oder C₁₋₅-Alkyl, das durch eine oder mehrere Gruppen unabhängig voneinander ausgewählt aus Hydroxy, Fluor und Amino substituiert sein kann;
2) C₁₋₅-Alkyl-X³COR¹⁶ (wobei X³ für 0 oder NR¹⁷ steht (wobei R¹⁷ für Wasserstoff, C₁₋₃-Alkyl oder C₁₋₃-Alkoxy-C₂₋₃-alkyl steht) und R¹⁶ für C₁₋₃-Alkyl, NR¹⁸R¹⁹ oder OR²⁰ steht (wobei R¹⁸, R¹⁹ und R²⁰ jeweils unabhängig voneinander für Wasserstoff, C₁₋₃-Alkyl oder C₁₋₃-Alkoxy-C₂₋₃-alkyl stehen));
3) C₁₋₅-Alkyl-X⁴R²¹ (wobei X⁴ für O, S, SO, SO₂, OCO, NR²²CO, CONR²³, SO₂NR²⁴, NR²⁵SO₂ oder NR²⁶ steht (wobei R²², R²³, R²⁴, R²⁵ und R²⁶ jeweils unabhängig für Wasserstoff, C₁₋₃-Alkyl oder C₁₋₃-Alkoxy-C₂₋₃-alkyl stehen) und R²¹ für Wasserstoff, C₁₋₃-Alkyl, Cyclopentyl, Cyclohexyl oder eine 5- oder 6-gliedrige gesättigte heterocyclische Gruppe mit einem oder zwei Heteroatomen unabhängig voneinander ausgewählt aus O, S und N steht, wobei diese C₁₋₃-Alkylgruppe durch einen oder zwei Substituenten unabhängig voneinander ausgewählt aus Oxo, Hydroxy, Halogen und C₁₋₄-Alkoxy substituiert sein kann und wobei diese heterocyclische Gruppe durch einen oder zwei Substituenten unabhängig voneinander ausgewählt aus Oxo, Hydroxy, Halogen, C₁₋₄-Alkyl, C₁₋₄-Hydroxyalkyl und C₁₋₄-Alkoxy substituiert sein kann);
4) C₁₋₅-Alkyl-X⁵-C₁₋₅-alkyl-X⁶R²⁷ (wobei X⁵ und X⁶ jeweils unabhängig voneinander für O, S, SO, SO₂, NR²⁸CO, CONR²⁹, SO₂NR³⁰, NR³¹SO₂ oder NR³² stehen (wobei R²⁸, R²⁹, R³⁰, R³¹ und R³² jeweils unabhängig voneinander für Wasserstoff, C₁₋₃-Alkyl oder C₁₋₃-Alkoxy-C₂₋₃-alkyl stehen) und R²⁷ für Wasserstoff oder C₁₋₃-Alkyl steht);
5) C₁₋₅-Alkyl-R³³ (wobei R³³ für eine 5- oder 6-gliedrige gesättigte heterocyclische Gruppe mit einem oder zwei Heteroatomen unabhängig voneinander ausgewählt aus 0, S und N steht, wobei diese heterocyclische Gruppe durch einen oder zwei Substituenten unabhängig voneinander ausgewählt aus Oxo, Hydroxy, Halogen, C₁₋₄-Alkyl, C₁₋₆-Carbonyl, C₁₋₄-Hydroxyalkyl und C₁₋₄-Alkoxy substituiert sein kann);
6) C₂₋₅-Alkenyl-R³³ (wobei R³³ wie oben definiert ist);
7) C₂₋₅-Alkinyl-R³³ (wobei R³³ wie oben definiert ist);
8) R³⁴ (wobei R³⁴ für eine Pyridongruppe, eine Phenylgruppe oder eine 5- oder 6gliedrige aromatische heterocyclische Gruppe mit 1 bis 3 Heteroatomen unabhängig voneinander ausgewählt aus O, N und S steht, wobei diese Pyridongruppe, Phenylgruppe oder heterocyclische Gruppe bis zu fünf Substituenten unabhängig voneinander ausgewählt aus Hydroxy, Halogen, Amino, C₁₋₄-Alkyl, C₁₋₄-Alkoxy, C₁₋₄-Hydroxyalkyl, C₁₋₄-Aminoalkyl, C₁₋₄-Alkylamino, C₁₋₄-Hydroxyalkoxy, Carboxy, Cyano, CONR³⁵R³⁶ und NR³⁷COR³⁸ tragen kann (wobei R³⁵, R³⁶, R³⁷ und R³⁸ jeweils unabhängig voneinander für Wasserstoff, C₁₋₄-Alkyl oder C₁₋₃-Alkoxy-C₂₋₃-alkyl stehen));
9) C₁₋₅-Alkyl-R³⁴ (wobei R³⁴ wie oben definiert ist);
10) C₂₋₅-Alkenyl-R³⁴ (wobei R³⁴ wie oben definiert ist);
11) C₂₋₅-Alkinyl-R³⁴ (wobei R³⁴ wie oben definiert ist);
12) C₁₋₅-Alkyl-X⁷R³³ (wobei X⁷ für O, S, SO, SO₂, NR³⁹CO, CONR⁴⁰, SO₂NR⁴¹, NR⁴²SO₂ oder NR⁴³ steht (wobei R³⁹, R⁴⁰, R⁴¹, R⁴² und R⁴³ jeweils unabhängig voneinander für Wasserstoff, C₁₋₃-Alkyl oder C₁₋₃-Alkoxy-C₂₋₃-alkyl stehen) und R³⁴ wie oben definiert ist);
13) C₂₋₅-Alkenyl-X⁸R³⁴ (wobei X⁸ für O, S, SO, SO₂, NR⁴⁴CO, CONR⁴⁵, SO₂NR⁴⁶, NR⁴⁷SO₂ oder NR⁴⁸ steht (wobei R⁴⁴, R⁴⁵, R⁴⁶, R⁴⁷ und R⁴⁸ jeweils unabhängig voneinander für Wasserstoff, C₁₋₃-Alkyl oder C₁₋₃-Alkoxy-C₂₋₃-alkyl stehen) und R³⁴ wie oben definiert ist);
14) C₂₋₅-Alkinyl-X⁹R³⁴ (wobei X⁹ für O, S, SO, SO₂, NR⁴⁹CO, CONR⁵⁰, SO₂NR⁵¹, NR⁵²SO₂ oder NR⁵³ steht (wobei R⁴⁹, R⁵⁰, R⁵¹, R⁵² und R⁵³ jeweils unabhängig voneinander für Wasserstoff, C₁₋₃-Alkyl oder C₁₋₃-Alkoxy-C₂₋₃-alkyl stehen) und R³⁴ wie oben definiert ist); und
15) C₁₋₃-Alkyl-X¹⁰-C₁₋₃-alkyl-R³⁴ (wobei X¹⁰ für O, S, SO, SO₂, NR⁵⁴CO, ONR⁵⁵, SO₂NR⁵⁶, NR⁵⁷SO₂ oder NR⁵⁸ steht (wobei R⁵⁴, R⁵⁵, R⁵⁶, R⁵⁷ und R⁵⁸ jeweils unabhängig voneinander für Wasserstoff, C₁₋₃-Alkyl oder C₁₋₃-Alkoxy-C₂₋₃-alkyl stehen) und R³⁴ wie oben definiert ist);
16) R³³ (wobei R³³ wie oben definiert ist); und
17) C₁₋₃-Alkyl-X¹⁰-C₁₋₃-alkyl-R³³ (wobei X¹⁰ und R³³ wie oben definiert sind);
18) C₁₋₅-Alkyl-COR³³ (wobei R³³ wie oben definiert ist);
n für 1, 2, 3 oder 4 steht;
m für 1, 2, 3 oder 4 steht;
als freie Base oder pharmazeutisch annehmbares Salz davon bei der Herstellung eines Medikaments zur Prävention und/oder Behandlung von Alzheimer-Krankheit.

2. Verwendung einer wie in Anspruch 1 definierten Verbindung der Formel I bei der Herstellung eines Medikaments zur Prävention und/oder Behandlung von demenziellen Krankheiten ausgewählt aus frontotemporaler Erkrankung des Parkinsontyps, Guam-Parkinsondemenz-Komplex, HIV-Demenz, Prädemenz-Zuständen, vaskulärer Demenz, Demenz mit Lewy-Körperchen, frontotemporaler Demenz und Dementia Pugilistica.

3. Verwendung einer wie in Anspruch 1 definierten Verbindung der Formel I bei der Herstellung eines Medikaments zur Prävention und/oder Behandlung von amyotropher Lateralsklerose, kortikobasaler Degeneration, Down-Syndrom, Chorea Huntington, Parkinson-Krankheit, postenzephalitischem Parkinsonismus, progressiver supranukleärer Lähmung, Pick-Krankheit, Niemann-Pick-Krankheit, Schlaganfall, Kopftrauma und anderen chronischen neurodegenerativen Erkrankungen, Bipolarerkrankung, affektiven Störungen, Depression, Schizophrenie, kognitiven Störungen und Haarausfall.

4. Verwendung einer wie in Anspruch 1 definierten Verbindung der Formel I bei der Herstellung eines Medikaments zur Prävention und/oder Behandlung von schwacher kognitiver Störung, altersbedingter Gedächtnisstörung, altersbedingter kognitiver Abnahme, kognitiver Störung ohne Demenz, schwacher kognitiver Abnahme, schwacher neurokognitiver Abnahme, Vergeßlichkeit im hohen Alter, Gedächtnisstörung und kognitiver Störung und androgenetischer Alopezie.

5. Verwendung einer Verbindung nach einem der Ansprüche 1 bis 4, wobei R³ für R¹⁰X² steht,
wobei X² für O, CH₂, S, SO, SO₂, NR¹¹CO, CONR¹², SO₂NR¹³, NR¹⁴SO₂ oder NR¹⁵ steht (wobei R¹¹, R¹², R¹³, R¹⁴ und R¹⁵ jeweils unabhängig für Wasserstoff, C₁₋₃-Alkyl oder C₁₋₃-Alkoxy-C₂₋₃-alkyl stehen), wobei X² für eine direkte Bindung steht; und
R¹⁰ aus einer der folgenden Gruppen ausgewählt ist:
1) Wasserstoff oder C₂₋₅-Alkyl, das durch eine oder mehrere Gruppen unabhängig voneinander ausgewählt aus Hydroxy, Fluor und Amino substituiert sein kann;
2) C₁₋₅-Alkyl-X³COR¹⁶ (wobei X³ für O oder NR¹⁷ steht (wobei R¹⁷ für Wasserstoff, C₁₋₃-Alkyl oder C₁₋₃-Alkoxy-C₂₋₃-alkyl steht) und R¹⁶ für C₁₋₃-Alkyl, NR¹⁸R¹⁹ oder OR²⁰ steht (wobei R¹⁸, R¹⁹ und R²⁰ jeweils unabhängig voneinander für Wasserstoff, C₁₋₃-Alkyl oder C₁₋₃-Alkoxy-C₂₋₃-alkyl stehen));
3) C₁₋₅-Alkyl-X⁴R²¹ (wobei X⁴ für 0, S, SO, SO₂, OCO, NR²²CO, CONR²³, SO₂NR²⁴, NR²⁵SO₂ oder NR²⁶ steht (wobei R²², R²³, R²⁴, R²⁵ und R²⁶ jeweils unabhängig für Wasserstoff, C₁₋₃-Alkyl oder C₁₋₃-Alkoxy-C₂₋₃-alkyl stehen) und R²¹ für Wasserstoff, C₁₋₃-Alkyl, Cyclopentyl, Cyclohexyl oder eine 5- oder 6-gliedrige gesättigte heterocyclische Gruppe mit einem oder zwei Heteroatomen unabhängig voneinander ausgewählt aus O, S und N steht, wobei diese C₁₋₃-Alkylgruppe durch einen oder zwei Substituenten unabhängig voneinander ausgewählt aus Oxo, Hydroxy, Halogen und C₁₋₄-Alkoxy substituiert sein kann und wobei diese heterocyclische Gruppe durch einen oder zwei Substituenten unabhängig voneinander ausgewählt aus Oxo, Hydroxy, Halogen, C₁₋₄-Alkyl, C₁₋₄-Hydroxyalkyl und C₁₋₄-Alkoxy substituiert sein kann);
4) C₁₋₅-Alkyl-X⁵-C₁₋₅-alkyl-X⁶R²⁷ (wobei X⁵ und X⁶ jeweils unabhängig voneinander für O, S, SO, SO₂, NR²⁸CO, CONR²⁹, SO₂NR³⁰, NR³¹SO₂ oder NR³² stehen (wobei R²⁸, R²⁹, R³⁰, R³¹ und R³² jeweils unabhängig voneinander für Wasserstoff, C₁₋₃-Alkyl oder C₁₋₃-Alkoxy-C₂₋₃-alkyl stehen) und R²⁷ für Wasserstoff oder C₁₋₃-Alkyl steht);
5) C₁₋₅-Alkyl-R³³ (wobei R³³ für eine 5- oder 6-gliedrige gesättigte heterocyclische Gruppe mit einem oder zwei Heteroatomen unabhängig voneinander ausgewählt aus O, S und N steht, wobei diese heterocyclische Gruppe durch einen oder zwei Substituenten unabhängig voneinander ausgewählt aus Oxo, Hydroxy, Halogen, C₁₋₄-Alkyl, C₁₋₄-Hydroxyalkyl und C₁₋₄-Alkoxy substituiert sein kann);
6) C₂₋₅-Alkenyl-R³³ (wobei R³³ wie oben definiert ist);
7) C₂₋₅-Alkinyl-R³³ (wobei R³³ wie oben definiert ist);
8) R³⁴ (wobei R³⁴ für eine Pyridongruppe, eine Phenylgruppe oder eine 5- oder 6gliedrige aromatische heterocyclische Gruppe mit 1 bis 3 Heteroatomen unabhängig voneinander ausgewählt aus O, N und S steht, wobei diese Pyridongruppe, Phenylgruppe oder heterocyclische Gruppe bis zu fünf Substituenten unabhängig voneinander ausgewählt aus Hydroxy, Halogen, Amino, C₁₋₄-Alkyl, C₁₋₄-Alkoxy, C₁₋₄-Hydroxyalkyl, C₁₋₄-Aminoalkyl, C₁₋₄-Alkylamino, C₁₋₄-Hydroxyalkoxy, Carboxy, Cyano, CONR³⁵R³⁶ und NR³⁷COR³⁸ tragen kann (wobei R³⁵, R³⁶, R³⁷ und R³⁸ jeweils unabhängig voneinander für Wasserstoff, C₁₋₄-Alkyl oder C₁₋₃-Alkoxy-C₂₋₃-alkyl stehen));
9) C₁₋₅-Alkyl-R³⁴ (wobei R³⁴ wie oben definiert ist);
10) C₂₋₅-Alkenyl-R³⁴ (wobei R³⁴ wie oben definiert ist);
11) C₂₋₅-Alkinyl-R³⁴ (wobei R³⁴ wie oben definiert ist);
12) C₁₋₅-Alkyl-X⁷R³³ (wobei X⁷ für O, S, SO, SO₂, NR³⁹CO, CONR⁴⁰, SO₂NR⁴¹, NR⁴²SO₂ oder NR⁴³ steht (wobei R³⁹, R⁴⁰, R⁴¹, R⁴² und R⁴³ jeweils unabhängig voneinander für Wasserstoff, C₁₋₃-Alkyl oder C₁₋₃-Alkoxy-C₂₋₃-alkyl stehen) und R³⁴ wie oben definiert ist);
13) C₂₋₅-Alkenyl-X⁸R³⁴ (wobei X⁸ für O, S, SO, SO₂, NR⁴⁴CO, CONR⁴⁵, SO₂NR⁴⁶, NR⁴⁷SO₂ oder NR⁴⁸ steht (wobei R⁴⁴, R⁴⁵, R⁴⁶, R⁴⁷ und R⁴⁸ jeweils unabhängig voneinander für Wasserstoff, C₁₋₃-Alkyl oder C₁₋₃-Alkoxy-C₂₋₃-alkyl stehen) und R³⁴ wie oben definiert ist);
14) C₂₋₅-Alkinyl-X⁹R³⁴ (wobei X⁹ für 0, S, SO, SO₂, NR⁴⁹CO, CONR⁵⁰, SO₂NR⁵¹, NR⁵²SO₂ oder NR⁵³ steht (wobei R⁴⁹, R⁵⁰, R⁵¹, R⁵² und R⁵³ jeweils unabhängig voneinander für Wasserstoff, C₁₋₃-Alkyl oder C₁₋₃-Alkoxy-C₂₋₃-alkyl stehen) und R³⁴ wie oben definiert ist); und
15) C₁₋₃-Alkyl-X¹⁰-C₁₋₃-alkyl-R³⁴ (wobei X¹⁰ für O, S, SO, SO₂, NR⁵⁴CO, ONR⁵⁵, SO₂NR⁵⁶, NR⁵⁷SO₂ oder NR⁵⁸ steht (wobei R⁵⁴, R⁵⁵, R⁵⁶, R⁵⁷ und R⁵⁸ jeweils unabhängig voneinander für Wasserstoff, C₁₋₃-Alkyl oder C₁₋₃-Alkoxy-C₂₋₃-alkyl stehen) und R³⁴ wie oben definiert ist);
16) R³³ (wobei R³³ wie oben definiert ist); und
17) C₁₋₃-Alkyl-X¹⁰-C₁₋₃-alkyl-R³³ (wobei X¹⁰ und R³³ wie oben definiert sind)).

6. Verwendung einer Verbindung nach einem der Ansprüche 1 bis 5, wobei R¹ für Wasserstoff steht.

7. Verwendung einer Verbindung nach einem der Ansprüche 1 bis 6, wobei R² für Halogen, Cyano, Nitro, Carboxy, C₁₋₄-Alkoxycarbonyl, Trifluoromethyl, C₁₋₃-Alkyl, C₁₋₃-Alkoxy, *N*-C₁₋₄-Alkylcarbamoyl, *N,N*-Di(C₁₋₄-alkyl)carbamoyl, Aminosulfonyl oder eine Gruppe R⁴X¹ steht,
wobei X¹ für CONR⁵R⁶ steht (wobei R⁵ für Wasserstoff oder C₁₋₂-Alkyl steht und R⁶ für C₁₋₄-Alkyl steht, und wobei R⁴ an R⁶ gebunden ist); und
R⁴ für eine Phenylgruppe oder eine 5- oder 6-gliedrige heterocyclische Gruppe mit einem oder zwei Heteroatomen unabhängig voneinander ausgewählt aus O und N steht, wobei diese heterocyclische Gruppe gesättigt oder ungesättigt sein kann und wobei diese Phenylgruppe bzw. heterocyclische Gruppe durch einen oder zwei Substituenten unabhängig voneinander ausgewählt aus Hydroxy, Halogen, C₁₋₃-Alkyl, C₁₋₃-Alkoxy, C₁₋₃-Alkanoyloxy, Trifluoromethyl, Cyano, Amino, Nitro und C₁₋₄-Alkoxycarbonyl substituiert sein kann;
n für 0, 1 oder 2 steht.

8. Verwendung einer Verbindung nach einem der Ansprüche 1 bis 7, wobei R³ für R¹⁰X² steht,
wobei X² für O steht; und
R¹⁰ aus einer der folgenden Gruppen ausgewählt ist:
3) C₁₋₅-Alkyl-X⁴R²¹ (wobei X⁴ für O oder NR²⁶ steht (wobei R²¹ und R²⁶ jeweils unabhängig voneinander für Wasserstoff, C₁₋₃-Alkyl, Cyclopentyl oder Cyclohexyl stehen));
4) C₁₋₅-Alkyl-X⁵-C₁₋₅-alkyl-X⁶R²⁷ (wobei X⁵ und X⁶ für O stehen und R²⁷ für Wasserstoff oder C₁₋₃-Alkyl steht);
5) C₁₋₅-Alkyl-R³³ (wobei R³³ für eine 6gliedrige gesättigte heterocyclische Gruppe mit einem oder zwei Heteroatomen unabhängig voneinander ausgewählt aus 0 und N steht, wobei diese heterocyclische Gruppe durch einen oder zwei Substituenten unabhängig voneinander ausgewählt aus Oxo, Hydroxy, Halogen, C₁₋₄-Alkyl, C₁₋₄-Hydroxyalkyl und C₁₋₄-Alkoxy substituiert sein kann);
9) C₁₋₅-Alkyl-R³⁴ (wobei R³⁴ für eine 5gliedrige aromatische heterocyclische Gruppe mit 1 bis 3 Heteroatomen unabhängig voneinander ausgewählt aus O und N steht, wobei diese heterocyclische Gruppe bis zu 5 Substituenten unabhängig voneinander ausgewählt aus Halogen, Amino, C₁₋₄-Alkyl, C₁₋₄-Alkoxy, C₁₋₄-Hydroxyalkyl, C₁₋₄-Aminoalkyl, C₁₋₄-Alkylamino, C₁₋₄-Hydroxyalkoxy, Carboxy, Hydroxy, Cyano, CONR³⁵R³⁶ und NR³⁷COR³⁸ tragen kann (wobei R³⁵, R³⁶, R³⁷ und R³⁸ jeweils unabhängig voneinander für Wasserstoff, C₁₋₄-Alkyl oder C₁₋₃-Alkoxy-C₂₋₃-alkyl stehen)); und
17) C₁₋₃-Alkyl-X¹⁰-C₁₋₃-alkyl-R³³ (wobei X¹⁰ für O steht und R³³ wie oben definiert ist);
m für 0, 1 oder 2 steht.

9. Verwendung einer Verbindung nach einem der Ansprüche 1 bis 4, 6 und 7, wobei R³ für R¹⁰X² steht,
wobei X² für O steht und R¹⁰ aus einer der folgenden Gruppen ausgewählt ist:
1) Wasserstoff oder C₁₋₅-Alkyl;
5) C₁₋₅-Alkyl-R³³ (wobei R³³ für eine 5- oder 6-gliedrige gesättigte heterocyclische Gruppe mit einem oder zwei Heteroatomen unabhängig voneinander ausgewählt aus 0, S und N steht, wobei diese heterocyclische Gruppe durch einen oder zwei Substituenten unabhängig voneinander ausgewählt aus Oxo, Hydroxy, Halogen, C₁₋₄-Alkyl, C₁₋₄-Carbonyl, C₁₋₄-Hydroxyalkyl und C₁₋₄-Alkoxy substituiert sein kann);
18) C₁₋₅-Alkyl-COR³³ (wobei R³³ wie oben definiert ist).

10. Verwendung einer Verbindung nach einem der Ansprüche 1 bis 8, wobei R³ für R¹⁰X² steht,
wobei X² für O steht; und R¹⁰ für
4) C₁₋₅-Alkyl-X⁵-C₁₋₅alkyl-X⁶R²⁷ steht (wobei X⁵ und X⁶ für O stehen und R²⁷ für Wasserstoff oder C₁₋₃-Alkyl steht).

11. Verwendung einer Verbindung nach einem der Ansprüche 1 bis 10, wobei R² in Position 5 und/oder 6 substituiert ist und R³ in Position 6, 7 und/oder 8 substituiert ist.

12. Verbindungen, bei denen es sich um
3-[7-(3-Dimethylaminopropoxy)chinazolin-4-yl]-2-hydroxy-1*H*-indol-5-carbonitrilhydrochlorid,
3-[7-(2-Dimethylaminoethoxy)chinazolin-4-yl]-2-hydroxy-1H-indol-5-carbonitrilfumarat,
3-{7-[2-(Isopropylmethylamino)ethoxy]chinazolin-4-yl}-2-oxo-2,3-dihydro-1*H*-indol-5-carbonitrilfumarat und
3-[7-(2-Diisopropylamino)ethoxy)chinazolin-4-yl]-2-hydroxy-1H-indol-5-carbonitrilfumarat
als freie Base oder Salze davon handelt.

13. Verbindungen nach Anspruch 12 zur Verwendung in der Therapie.

14. Verwendung einer Verbindung nach Anspruch 12 bei der Herstellung eines Medikaments zur Prävention und/oder Behandlung von Alzheimer-Krankheit.

15. Verwendung einer Verbindung nach Anspruch 12 bei der Herstellung eines Medikaments zur Prävention und/oder Behandlung von demenziellen Krankheiten ausgewählt aus frontotemporaler Erkrankung des Parkinsontyps, Guam-Parkinsondemenz-Komplex, HIV-Demenz, Prädemenz-Zuständen, vaskulärer Demenz, Demenz mit Lewy-Körperchen, frontotemporaler Demenz und Dementia Pugilistica.

16. Verwendung einer Verbindung nach Anspruch 12 bei der Herstellung eines Medikaments zur Prävention und/oder Behandlung von amyotropher Lateralsklerose, kortikobasaler Degeneration, Down-Syndrom, Chorea Huntington, Parkinson-Krankheit, postenzephalitischem Parkinsonismus, progressiver supranukleärer Lähmung, Pick-Krankheit, Niemann-Pick-Krankheit, Schlaganfall, Kopftrauma und anderen chronischen neurodegenerativen Erkrankungen, Bipolarerkrankung, affektiven Störungen, Depression, Schizophrenie, kognitiven Störungen und Haarausfall.

17. Verwendung einer Verbindung nach Anspruch 12 bei der Herstellung eines Medikaments zur Prävention und/oder Behandlung von schwacher kognitiver Störung, altersbedingter Gedächtnisstörung, altersbedingter kognitiver Abnahme, kognitiver Störung ohne Demenz, schwacher kognitiver Abnahme, schwacher neurokognitiver Abnahme, Vergeßlichkeit im hohen Alter, Gedächtnisstörung und kognitiver Störung und androgenetischer Alopezie.

## Revendications

1. Utilisation d'un composé de formule I 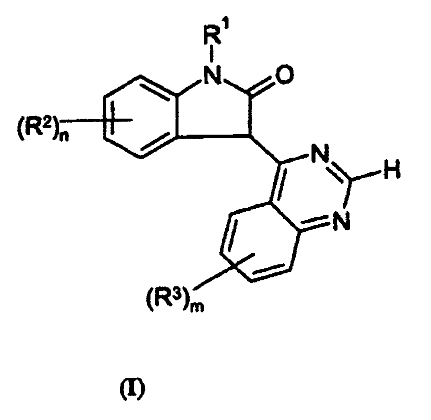 dans laquelle :
R¹ est hydrogène ou C₁₋₃alkyle ;
R² est hydroxy, halogéno, trifluorométhyle, cyano, amino, nitro, carboxy, C₁₋₃alkyle, C₁₋₃alcoxy, C₁₋₃alcanoyloxy, C₂₋₄alcanoyle, C₁₋₄alcanoylamino, C₁₋₄alcoxycarbonyle, C₁₋₄alkylthio, C₁₋₄alkylsulfinyle, C₁₋₄alkylsulfonyle, carbamoyle, *N*-C₁₋₄alkylcarbamoyle, *N,N-*di(C₁₋₄alkyl)carbamoyle, aminosulfonyle, *N*-C₁₋₄alkylaminosulfonyle, *N,N*-di(C₁₋₄alkyl)aminosulfonyle, C₁₋₄alkylsulfonylamino, ou un groupement R⁴X¹,
où X¹ est une liaison directe, C₂₋₄alcanoyle, CONR⁵R⁶, SO₂NR⁷R⁸ ou SO₂R⁹ (où R⁵ et R⁷ sont chacun indépendamment hydrogène ou C₁₋₂alkyle, et R⁶, R⁸ et
R⁹ sont chacun indépendamment C₁₋₄alkyle, et où R⁴ est lié à R⁶, R⁸ ou R⁹) ; et
R⁴ est phényle ou un groupement hétérocyclique à 5 ou 6 chaînons avec un ou deux hétéroatomes choisis indépendamment parmi O, S et N, lequel groupement hétérocyclique peut être saturé ou insaturé et lequel groupement phényle ou hétérocyclique peut être substitué par un ou deux substituants choisis indépendamment parmi hydroxy, halogéno, C₁₋₃alkyle, C₁₋₃alcoxy, C₁₋₃alcanoyloxy, trifluorométhyle, cyano, amino, nitro et C₁₋₄alcoxycarbonyle ;
R³ est hydroxy, halogéno, nitro, trifluorométhyle, C₁₋₃alkyle, cyano, amino ou R¹⁰X², où X² est O, CH₂, S, SO, SO₂, NR¹¹CO, CONR¹², SO₂NR¹³, NR¹⁴SO₂ ou NR¹⁵ (où R¹¹, R¹², R¹³, R¹⁴ et R¹⁵ sont chacun indépendamment hydrogène, C₁₋₃alkyle ou C₁₋₃alcoxyC₂₋₃alkyle), ou X² est une liaison directe ; et
R¹⁰ est choisi dans l'un des groupes suivants :
1) hydrogène ou C₁₋₅alkyle qui peut être substitué par un ou plusieurs groupements choisis indépendamment parmi hydroxy, fluoro et amino ;
2) C₁₋₅alkylX³COR¹⁶ (où X³ est O ou NR¹⁷ (où R¹⁷ est hydrogène, C₁₋₃alkyle ou C₁₋₃alcoxyC₂₋₃alkyle) et R¹⁶ est C₁₋₃alkyle, NR¹⁸R¹⁹ ou OR²⁰ (où R¹⁸, R¹⁹ et R²⁰ sont chacun indépendamment hydrogène, C₁₋₃alkyle ou C₁₋₃alcoxyC₂₋₃alkyle)) ;
3) C₁₋₅alkylX⁴R²¹ (où X⁴ est O, S, SO, SO₂, OCO, NR²²CO, CONR²³, SO₂NR²⁴, NR²⁵SO₂ ou NR²⁶ (où R²², R²³, R²⁴, R²⁵ et R²⁶ sont chacun indépendamment hydrogène, C₁₋₃alkyle ou C₁₋₃alcoxyC₂₋₃alkyle) et R²¹ est hydrogène, C₁₋₃alkyle, cyclopentyle, cyclohexyle ou un groupement hétérocyclique saturé à 5 ou 6 chaînons avec un ou deux hétéroatomes choisis indépendamment parmi O, S et N, lequel groupement C₁₋₃alkyle peut être substitué par un ou deux substituants choisis indépendamment parmi oxo, hydroxy, halogéno et C₁₋₄alcoxy et lequel groupement hétérocyclique peut être substitué par un ou deux substituants choisis indépendamment parmi oxo, hydroxy, halogéno, C₁₋₄alkyle, C₁₋₄hydroxyalkyle et C₁₋₄alcoxy) ;
4) C₁₋₅alkylX⁵C₁₋₅alkylX⁶R²⁷ (où X⁵ et X⁶ sont chacun indépendamment O, S, SO, SO₂, NR²⁸CO, CONR²⁹, SO₂NR³⁰, NR³¹SO₂ ou NR³² (où R²⁸, R²⁹, R³⁰, R³¹ et R³² sont chacun indépendamment hydrogène, C₁₋₃alkyle ou C₁₋₃alcoxyC₂₋₃alkyle) et R²⁷ est hydrogène ou C₁₋₃alkyle) ;
5) C₁₋₅alkylR³³ (où R³³ est un groupement hétérocyclique saturé à 5 ou 6 chaînons avec un ou deux hétéroatomes choisis indépendamment parmi O, S et N, lequel groupement hétérocyclique peut être substitué par un ou deux substituants choisis indépendamment parmi oxo, hydroxy, halogéno, C₁₋₄alkyle, C₁₋₆carbonyle, C₁₋₄hydroxyalkyle et C₁₋₄alcoxy) ;
6) C₂₋₅alcénylR³³ (où R³³ est tel que défini précédemment) ;
7) C₂₋₅alcynylR³³ (où R³³ est tel que défini précédemment) ;
8) R³⁴ (où R³⁴ est un groupement pyridone, un groupement phényle ou un groupement hétérocyclique aromatique à 5 ou 6 chaînons avec 1 à 3 hétéroatomes choisis indépendamment parmi O, N et S, lequel groupement pyridone, phényle ou hétérocyclique peut porter jusqu'à 5 substituants choisis indépendamment parmi hydroxy, halogéno, amino, C₁₋₄alkyle, C₁₋₄alcoxy, C₁₋₄hydroxyalkyle, C₁₋₄aminoalkyle, C₁₋₄alkylamino, C₁₋₄hydroxyalcoxy, carboxy, cyano, CONR³⁵R³⁶ et NR³⁷COR³⁸ (où R³⁵, R³⁶, R³⁷ et R³⁸ sont chacun indépendamment hydrogène, C₁₋₄alkyle ou C₁₋₃alcoxyC₂₋₃alkyle)) ;
9) C₁₋₅alkylR³⁴ (où R³⁴ est tel que défini précédemment) ;
10) C₂₋₅alcénylR³⁴ (où R³⁴ est tel que défini précédemment) ;
11) C₂₋₅alcynylR³⁴ (où R³⁴ est tel que défini précédemment) ;
12) C₁₋₅alkylX⁷R³⁴ (où X⁷ est O, S, SO, SO₂, NR³⁹CO, CONR⁴⁰, SO₂NR⁴¹, NR⁴²SO₂ ou NR⁴³ (où R³⁹, R⁴⁰, R⁴¹, R⁴² et R⁴³ sont chacun indépendamment hydrogène, C₁₋₃alkyle ou C₁₋₃alcoxyC₂₋₃alkyle) et R³⁴ est tel que défini précédemment) ;
13) C₂₋₅alcénylX⁸R³⁴ (où X⁸ est O, S, SO, SO₂, NR⁴⁴CO, CONR⁴⁵, SO₂NR⁴⁶, NR⁴⁷SO₂ ou NR⁴⁸ (où R⁴⁴, R⁴⁵, R⁴⁶, R⁴⁷ et R⁴⁸ sont chacun indépendamment hydrogène, C₁₋₃alkyle ou C₁₋₃alcoxyC₂₋₃alkyle) et R³⁴ est tel que défini précédemment) ;
14) C₂₋₅alcynylX⁹R³⁴ (où X⁹ est O, S, SO, SO₂, NR⁴⁹CO, CONR⁵⁰, SO₂NR⁵¹, NR⁵²SO₂ ou NR⁵³ (où R⁴⁹, R⁵⁰, R⁵¹, R⁵² et R⁵³ sont chacun indépendamment hydrogène, C₁₋₃alkyle ou C₁₋₃alcoxyC₂₋₃alkyle) et R³⁴ est tel que défini précédemment) ;
15) C₁₋₃alkylX¹⁰C₁₋₃alkylR³⁴ (où X¹⁰ est O, S, SO, SO₂, NR⁵⁴CO, ONR⁵⁵, SO₂NR⁵⁶, NR⁵⁷SO₂ ou NR⁵⁸ (où R⁵⁴, R⁵⁵, R⁵⁶, R⁵⁷ et R⁵⁸ sont chacun indépendamment hydrogène, C₁₋₃alkyle ou C₁₋₃alcoxyC₂₋₃alkyle) et R³⁴ est tel que défini précédemment) ;
16) R³³ (où R³³ est tel que défini précédemment) ; et
17) C₁₋₃alkylX¹⁰C₁₋₃alkylR³³ (où X¹⁰ et R³³ sont tels que définis précédemment) ;
18) C₁₋₅alkylCOR³³ (où R³³ est tel que défini précédemment) ;
n est 0, 1, 2, 3 ou 4 ;
m est 0, 1, 2, 3 ou 4 ;
sous forme de base libre ou de sel pharmaceutiquement acceptable de celui-ci, dans la fabrication d'un médicament destiné à la prévention et/ou au traitement de la maladie d'Alzheimer.

2. Utilisation d'un composé de formule I telle que définie dans la revendication 1, dans la fabrication d'un médicament destiné à la prévention et/ou au traitement de maladies liées à une démence choisies parmi la démence frontotemporale de type Parkinson, le complexe de la démence de Parkinson de Guam, la démence liée au VIH, les états de prédémence, la démence vasculaire, la démence à corps de Lewy, la démence frontotemporale et la démence pugilistique.

3. Utilisation d'un composé de formule I telle que définie dans la revendication 1, dans la fabrication d'un médicament destiné à la prévention et/ou au traitement de la sclérose latérale amyotrophique, de la dégénérescence corticobasale, du syndrome de Down, de la maladie de Huntington, de la maladie de Parkinson, du syndrome parkinsonien post-encéphalytique, de la paralysie supranucléaire progressive, de la maladie de Pick, de la maladie de Niemann-Pick, de l'accident cérébro-vasculaire, du traumatisme crânien et d'autres maladies neurodégénératives chroniques, d'une maladie bipolaire, de troubles affectifs, de la dépression, de la schizophrénie, de troubles cognitifs et de l'alopécie.

4. Utilisation d'un composé de formule I telle que définie dans la revendication 1, dans la fabrication d'un médicament destiné à la prévention et/ou au traitement de la déficience cognitive légère, de la déficience de la mémoire liée à l'âge, du déclin cognitif lié à l'âge, de la déficience cognitive sans démence, du déclin cognitif léger, du déclin neurocognitif léger, de l'oubli bénin lié à l'âge, de la déficience de la mémoire, de la déficience cognitive et de l'alopécie androgénétique.

5. Utilisation d'un composé selon l'une quelconque des revendications 1 à 4, **caractérisée en ce que** R³ est R¹⁰X²,
où X² est 0, CH₂, S, SO, SO₂, NR¹¹CO, CONR¹², SO₂NR¹³, NR¹⁴SO₂ ou NR¹⁵ (où R¹¹, R¹², R¹³, R¹⁴ et R¹⁵ sont chacun indépendamment hydrogène, C₁₋₃alkyle ou C₁₋₃alcoxyC₂₋₃alkyle), ou X² est une liaison directe ; et
R¹⁰ est choisi dans l'un des groupes suivants :
1) hydrogène ou C₁₋₅alkyle qui peut être substitué par un ou plusieurs groupements choisis indépendamment parmi hydroxy, fluoro et amino ;
2) C₁₋₅alkylX³COR¹⁶ (où X³ est O ou NR¹⁷ (où R¹⁷ est hydrogène, C₁₋₃alkyle ou C₁₋₃alcoxyC₂₋₃alkyle) et R¹⁶ est C₁₋₃alkyle, NR¹⁸R¹⁹ ou OR²⁰ (où R¹⁸, R¹⁹ et R²⁰ sont chacun indépendamment hydrogène, C₁₋₃alkyle ou C₁₋₃alcoxyC₂₋₃alkyle)) ;
3) C₁₋₅alkyl_{X}⁴R²¹ (où X⁴ est O, S, SO, SO₂, OCO, NR²²CO, CONR²³, SO₂NR²⁴, NR²⁵SO₂ ou NR²⁶ (où R²², R²³, R²⁴, R²⁵ et R²⁶ sont chacun indépendamment hydrogène, C₁₋₃alkyle ou C₁₋₃alcoxyC₂₋₃alkyle) et R²¹ est hydrogène, C₁₋₃alkyle, cyclopentyle, cyclohexyle ou un groupement hétérocyclique saturé à 5 ou 6 chaînons avec un ou deux hétéroatomes choisis indépendamment parmi O, S et N, lequel groupement C₁₋₃alkyle peut être substitué par un ou deux substituants choisis indépendamment parmi oxo, hydroxy, halogéno et C₁₋₄alcoxy et lequel groupement hétérocyclique peut être substitué par un ou deux substituants choisis indépendamment parmi oxo, hydroxy, halogéno, C₁₋₄alkyle, C₁₋₄hydroxyalkyle et C₁₋₄alcoxy) ;
4) C₁₋₅alkylX⁵C₁₋₅alkylX⁶R²⁷ (où X⁵ et X⁶ sont chacun indépendamment O, S, SO, SO₂, NR²⁸CO, CONR²⁹, SO₂NR³⁰, NR³¹SO₂ ou NR³² (où R²⁸, R²⁹, R³⁰, R³¹ et R³² sont chacun indépendamment hydrogène, C₁₋₃alkyle ou C₁₋₃alcoxyC₂₋₃alkyle) et R²⁷ est hydrogène ou C₁₋₃alkyle) ;
5) C₁₋₅alkylR³³ (où R³³ est un groupement hétérocyclique saturé à 5 ou 6 chaînons avec un ou deux hétéroatomes choisis indépendamment parmi O, S et N, lequel groupement hétérocyclique peut être substitué par un ou deux substituants choisis indépendamment parmi oxo, hydroxy, halogéno, C₁₋₄alkyle, C₁₋₄hydroxyalkyle et C₁₋₄alcoxy) ;
6) C₂₋₅alcénylR³³ (où R³³ est tel que défini précédemment) ;
7) C₂₋₅alcynylR³³ (où R³³ est tel que défini précédemment) ;
8) R³⁴ (où R³⁴ est un groupement pyridone, un groupement phényle ou un groupement hétérocyclique aromatique à 5 ou 6 chaînons avec 1 à 3 hétéroatomes choisis indépendamment parmi O, N et S, lequel groupement pyridone, phényle ou hétérocyclique peut porter jusqu'à 5 substituants choisis indépendamment parmi hydroxy, halogéno, amino, C₁₋₄alkyle, C₁₋₄alcoxy, C₁₋₄hydroxyalkyle, C₁₋₄aminoalkyle, C₁₋₄alkylamino, C₁₋₄hydroxyalcoxy, carboxy, cyano, CONR³⁵R³⁶ et NR³⁷COR³⁸ (où R³⁵, R³⁶, R³⁷ et R³⁸ sont chacun indépendamment hydrogène, C₁₋₄alkyle ou C₁₋₃alcoxyC₂₋₃alkyle)) ;
9) C₁₋₅alkylR³⁴ (où R³⁴ est tel que défini précédemment) ;
10) C₂₋₅alcénylR³⁴ (où R³⁴ est tel que défini précédemment) ;
11) C₂₋₅alcynylR³⁴ (où R³⁴ est tel que défini précédemment) ;
12) C₁-₅alkylX⁷R³⁴ (où X⁷ est O, S, SO, SO₂, NR³⁹CO, CONR⁴⁰, SO_{2N}R⁴¹, NR⁴²SO₂ ou NR⁴³ (où R³⁹, R⁴⁰, R⁴¹, R⁴² et R⁴³ sont chacun indépendamment hydrogène, C₁₋₃alkyle ou C₁₋₃alcoxyC₂₋₃alkyle) et R³⁴ est tel que défini précédemment) ;
13) C₂₋₅alcénylX⁸R³⁴ (où X⁸ est O, S, SO, SO₂, NR⁴⁴CO, CONR⁴⁵, SO₂NR⁴⁶, NR⁴⁷SO₂ ou NR⁴⁸ (où R⁴⁴, R⁴⁵, R⁴⁶, R⁴⁷ et R⁴⁸ sont chacun indépendamment hydrogène, C₁₋₃alkyle ou C₁₋₃alcoxyC₂₋₃alkyle) et R³⁴ est tel que défini précédemment) ;
14) C₂₋₅alcynylX⁹R³⁴ (où X⁹ est O, S, SO, SO₂, NR⁴⁹CO, CONR⁵⁰, SO₂NR⁵¹, NR⁵²SO₂ ou NR⁵³ (où R⁴⁹, R⁵⁰, R⁵¹, R⁵² et R⁵³ sont chacun indépendamment hydrogène, C₁₋₃alkyle ou C₁₋₃alcoxyC₂₋₃alkyle) et R³⁴ est tel que défini précédemment) ;
15) C₁₋₃alkylX¹⁰C₁₋₃alkylR³⁴ (où X¹⁰ est O, S, SO, SO₂, NR⁵⁴CO, ONR⁵⁵, SO₂NR⁵⁶, NR⁵⁷SO₂ ou NR⁵⁸ (où R⁵⁴, R⁵⁵, R⁵⁶, R⁵⁷ et R⁵⁸ sont chacun indépendamment hydrogène, C₁₋₃alkyle ou C₁₋₃alcoxyC₂₋₃alkyle) et R³⁴ est tel que défini précédemment) ;
16) R³³ (où R³³ est tel que défini précédemment) ; et
17) C₁₋₃alkylX¹⁰C₁₋₃alkylR³³ (où X¹⁰ et R³³ sont tels que définis précédemment).

6. Utilisation d'un composé selon l'une quelconque des revendications 1 à 5, **caractérisée en ce que** R¹ est hydrogène.

7. Utilisation d'un composé selon l'une quelconque des revendications 1 à 6, **caractérisée en ce que** R² est halogéno, cyano, nitro, carboxy, C₁₋₄alcoxycarbonyle, trifluorométhyle, C₁₋₃alkyle, C₁₋₃alcoxy, *N*-C₁₋₄alkylcarbamoyle, *N,N*-di(C₁₋₄alkyl)carbamoyle, aminosulfonyle, ou un groupement R⁴X¹,
où X¹ est CONR⁵R⁶ (où R⁵ est hydrogène ou C₁₋₂alkyle, et R⁶ est C₁₋₄alkyle, et où R⁴ est lié à R⁶) ; et
R⁴ est phényle ou un groupement hétérocyclique à 5 ou 6 chaînons avec un ou deux hétéroatomes choisis indépendamment parmi O et N, lequel groupement hétérocyclique peut être saturé ou insaturé et lequel groupement phényle ou hétérocyclique peut être substitué par un ou deux substituants choisis indépendamment parmi hydroxy, halogéno, C₁₋₃alkyle, C₁₋₃alcoxy, C₁₋₃alcanoyloxy, trifluorométhyle, cyano, amino, nitro et C₁₋₄alcoxycarbonyle ;
n est 0, 1 ou 2.

8. Utilisation d'un composé selon l'une quelconque des revendications 1 à 7, **caractérisée en ce que** R³ est R¹⁰X²,
où X² est O ; et
R¹⁰ est choisi dans l'un des groupes suivants :
3) C₁₋₅alkylX⁴R²¹ (où X⁴ est O ou NR²⁶ (où R²¹ et R²⁶ sont chacun indépendamment hydrogène, C₁₋₃alkyle, cyclopentyle ou cyclohexyle)) ;
4) C₁₋₅alkylX⁵C₁₋₅alkylX⁶R²⁷ (où X⁵ et X⁶ sont O et R²⁷ est hydrogène ou C₁₋₃alkyle) ;
5) C₁₋₅alkylR³³ (où R³³ est un groupement hétérocyclique saturé à 6 chaînons avec un ou deux hétéroatomes choisis indépendamment parmi 0 et N, lequel groupement hétérocyclique peut être substitué par un ou deux substituants choisis indépendamment parmi oxo, hydroxy, halogéno, C₁₋₄alkyle, C₁₋₄hydroxyalkyle et C₁₋₄alcoxy) ;
9) C₁₋₅alkylR³⁴ (où R³⁴ est un groupement hétérocyclique aromatique à 5 chaînons avec 1 à 3 hétéroatomes choisis indépendamment parmi O et N, lequel groupement hétérocyclique peut porter jusqu'à 5 substituants choisis indépendamment parmi halogéno, amino, C₁₋₄alkyle, C₁₋₄alcoxy, C₁₋₄hydroxyalkyle, C₁₋₄aminoalkyle, C₁₋₄alkylamino, C₁₋₄hydroxyalcoxy, carboxy, hydroxy, cyano, CONR³⁵R³⁶ et NR³⁷COR³⁸ (où R³⁵, R³⁶, R³⁷ et R³⁸ sont chacun indépendamment hydrogène, C₁₋₄alkyle ou C₁₋₃alcoxyC₂₋₃alkyle)) ; et
17) C₁₋₃alkylX¹⁰C₁₋₃alkylR³³ (où X¹⁰ est O et R³³ sont tels que définis précédemment) ;
m est 0, 1 ou 2.

9. Utilisation d'un composé selon l'une quelconque des revendications 1 à 4, 6 et 7, **caractérisée en ce que** R³ est R¹⁰X²,
où X² est O ; et R¹⁰ est choisi dans l'un des groupes suivants :
1) hydrogène ou C₁₋₅alkyle ;
5) C₁₋₅alkylR³³ (où R³³ est un groupement hétérocyclique saturé à 5 ou 6 chaînons avec un ou deux hétéroatomes choisis indépendamment parmi O, S et N, lequel groupement hétérocyclique peut être substitué par un ou deux substituants choisis indépendamment parmi oxo, hydroxy, halogéno, C₁₋₄alkyle, C₁₋₆carbonyle, C1₋₄hydroxyalkyle et C₁₋₄alcoxy) ;
18) C₁₋₅alkylCOR³³ (où R³³ est tel que défini précédemment).

10. Utilisation d'un composé selon l'une quelconque des revendications 1 à 8, **caractérisée en ce que** R³ est R¹⁰X²,
où X² est O ; et R¹⁰ est
4) C₁₋₅alkylX⁵C₁₋₅alkylX⁶R²⁷ (où X⁵ et X⁶ sont O et R²⁷ est hydrogène ou C₁₋₃alkyle).

11. Utilisation d'un composé selon l'une quelconque des revendications 1 à 10, **caractérisée en ce que** R² est substitué en position 5 et/ou 6 et R³ est substitué en position 6, 7 et/ou 8.

12. Composé, **caractérisé en ce qu'**il s'agit de chlorhydrate de 3-[7-(3-diméthylaminopropoxy)-quinazolin-4-yl]-2-hydroxy-1*H*-indol-5-carbonitrile, fumarate de 3-[7-(2-diméthylaminoéthoxy)quinazolin-4-yl]-2-hydroxy-1*H*-indol-5-carbonitrile, fumarate de 3-{7-[2-(isopropylméthylamino)éthoxy]-quinazolin-4-yl}-2-oxo-2,3-dihydro-1*H*-indol-5-carbonitrile et fumarate de 3-[7-(2-diisopropylamino)éthoxy)-quinazolin-4-yl]-2-hydroxy-1*H*-indol-5-carbonitrile, sous forme de base libre ou de sels de celui-ci.

13. Composés selon la revendication 12, destinés à être utilisés en thérapie.

14. Utilisation d'un composé selon la revendication 12, dans la fabrication d'un médicament destiné à la prévention et/ou au traitement de la maladie d'Alzheimer.

15. Utilisation d'un composé selon la revendication 12, dans la fabrication d'un médicament destiné à la prévention et/ou au traitement de maladies liées à une démence choisies dans le groupe constitué de la démence frontotemporale de type Parkinson, du complexe de la démence de Parkinson de Guam, de la démence liée au VIH, des états de prédémence, de la démence vasculaire, de la démence à corps de Lewy, de la démence frontotemporale et de la démence pugilistique.

16. Utilisation d'un composé selon la revendication 12, dans la fabrication d'un médicament destiné à la prévention et/ou au traitement de la sclérose latérale amyotrophique, de la dégénérescence corticobasale, du syndrome de Down, de la maladie de Huntington, de la maladie de Parkinson, du syndrome parkinsonien post-encéphalytique, de la paralysie supranucléaire progressive, de la maladie de Pick, de la maladie de Niemann-Pick, de l'accident cérébro-vasculaire, du traumatisme crânien et d'autres maladies neurodégénératives chroniques, d'une maladie bipolaire, de troubles affectifs, de la dépression, de la schizophrénie, de troubles cognitifs et de l'alopécie.

17. Utilisation d'un composé selon la revendication 12, dans la fabrication d'un médicament destiné à la prévention et/ou au traitement de la déficience cognitive légère, de la déficience de la mémoire liée à l'âge, du déclin cognitif lié à l'âge, de la déficience cognitive sans démence, du déclin cognitif léger, du déclin neurocognitif léger, de l'oubli bénin lié à l'âge, de la déficience de la mémoire, de la déficience cognitive et de l'alopécie androgénétique.
